(19) Europäisches Patentamt
European Patent Office
Office européen des brevets

(11) EP 3 415 635 B1

(12) EUROPEAN PATENT SPECIFICATION

(45) Date of publication and mention of the grant of the patent:
**04.08.2021 Bulletin 2021/31**

(51) Int Cl.:
***C12Q 1/6881*** *(2018.01)* ***C12Q 1/6883*** *(2018.01)*

(21) Application number: **17175573.9**

(22) Date of filing: **12.06.2017**

(54) **AGE DETERMINATION OF A HUMAN INDIVIDUAL**

ALTERSBESTIMMUNG EINES MENSCHEN

DÉTERMINATION DE L'ÂGE D'UN INDIVIDU HUMAIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**19.12.2018 Bulletin 2018/51**

(73) Proprietor: **Beiersdorf Aktiengesellschaft**
**20245 Hamburg (DE)**

(72) Inventors:
- **WINNEFELD, Marc**
**22880 Wedel (DE)**
- **WURZER, Elisabeth**
**20255 Hamburg (DE)**
- **KRISTOF, Boris**
**25469 Halsetenbek (DE)**
- **KADERALI, Lars**
**17493 Greifswald (DE)**

(74) Representative: **Haseltine Lake Kempner LLP**
**Bürkleinstrasse 10**
**80538 München (DE)**

(56) References cited:
**WO-A2-2013/033627 CN-B- 104 357 561**
**US-A1- 2015 259 742**

- **FELIX BORMANN ET AL: "Reduced DNA methylation patterning and transcriptional connectivity define human skin aging", AGING CELL, vol. 15, no. 3, 23 March 2016 (2016-03-23) , pages 563-571, XP055400994, GB ISSN: 1474-9718, DOI: 10.1111/acel.12470**
- **Anonymous: "Infinium HumanMethylation450 BeadChip", Illumina , 9 March 2012 (2012-03-09), XP055401052, Retrieved from the Internet: URL:https://support.illumina.com/content/dam/illumina-marketing/documents/products/datasheets/datasheet_humanmethylation450.pd f [retrieved on 2017-08-24]**
- **Carmen M Koch ET AL: "Epigenetic-aging-signature to determine age in different tissues", Aging, 1 October 2011 (2011-10-01), pages 1018-1027, XP055160615, United States DOI: 10.18632/aging.100395 Retrieved from the Internet: URL:http://www.ncbi.nlm.nih.gov/pubmed/22067257 [retrieved on 2015-01-08]**
- **LINDSAY M. REYNOLDS ET AL: "Age-related variations in the methylome associated with gene expression in human monocytes and T cells", NATURE COMMUNICATIONS, vol. 5, 18 November 2014 (2014-11-18), page 5366, XP055428472, DOI: 10.1038/ncomms6366 -& L Reynolds ET AL: "Age-related variations in the methylome associated with gene expression in human monocytes and T cells - Supplementary Table 1", Nature communications, 18 November 2014 (2014-11-18), XP055428477, Retrieved from the Internet: URL:http://www.nature.com/articles/ncomms6366 [retrieved on 2017-11-24]**
- **Anonymous: "Direct Cycle Sequencing Kit Limited Use Label License: Research Use Only", , 1 February 2011 (2011-02-01), XP55726710, Retrieved from the Internet: URL:https://tools.thermofisher.com/content/sfs/manuals/cms_091370.pdf [retrieved on 2020-09-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The file contains technical information submitted after the application was filed and not included in this specification

## Description

### FIELD OF THE INVENTION

The present invention relates to a method for determining the biological age of human skin by analysing the epigenetic pattern of particular sites of the DNA. The methylation levels at selected CpG sites of a skin sample are evaluated to determine the biological age of human skin accurately. The measured biological age is then correlated to the chronological age with a mean absolute error of less than 5.05 years. The present invention also relates to the use of a nucleic acid molecule for determining the biological ages of human skin according to the method of the invention, and a computer-readable medium for determining the biological age of human skin.

### BACKGROUND OF THE INVENTION

Epigenetic changes of DNA are known to occur over time and have been recognised as indicators of the extent of the aging process in human individuals.

In particular, the extent of methylation of CpG dinucleotides has been identified as being related to age.

Methods of biological age determination by the analysis of various CpG-dinucleotides are known. In Weidner et al, Genome Biology, 2014 15R24, for example, 102 age-related CpG sites in blood have been identifies as suitable for estimating the biological age of an individual. The described method uses data from 3 CpG sites to achieve a mean absolute deviation from the chronological age of less than 5 years. EP 2 711 431 also analyses blood cells and predicts the chronological age of the subject based on 6 of the 102 identified age-related CpG sites. The prediction based on 6 CpG sites to determine the biological age was found to differ from the chronological age by about 4.53 years to as much as 10.30 years. In another study, a difference of ± 3.6 years between the chronological age and biological age was found by Horvath, Genome Biology 2013, 14:R115 by characterising 353 CpG sites from blood cells.

The use of blood samples in the analysis of epigenetic change is suboptimal due to the various cell types found in the blood that have differing DNA methylation patterns. Furthermore, bacterial and viral infections can significantly alter the cell composition in blood, which can shift the methylation pattern.

Human skin has also been used as a model for the analysis of age-related epigenetic changes and correlated to the chronological age of an individual. The known predictor according to Horvath, Genome Biology 2013, 14:R115 was used on human epidermis in Bormann et al, Aging Cell, 2016, 15, page 563 to 571 and the average absolute prediction error was found to be 14.5 years. This shows that the published predictor underestimates the chronological age of epidermis samples. In Bormann et al, Aging Cell, 2016, 15, page 563 to 571, a further predictor was developed using the methylation of 450,000 CpG dinucleotides, which were simultaneously analysed by an array-based approach. The biological age determined using the method therein was within an error of less than 5.25 years of the chronological age. However the drawback of this method is the large number of sites that need to be analysed, which is time consuming and cost intensive.

It is therefore desirable to provide improved and/or alternative methods to determine the biological age of an individual. Preferably, it is desired to provide a method that uses a reduced number of CpG sites.

### SUMMARY OF THE INVENTION

The present invention is defined in the appended claims.

In accordance with a first aspect, there is provided a method for determining the biological age of human skin comprising:

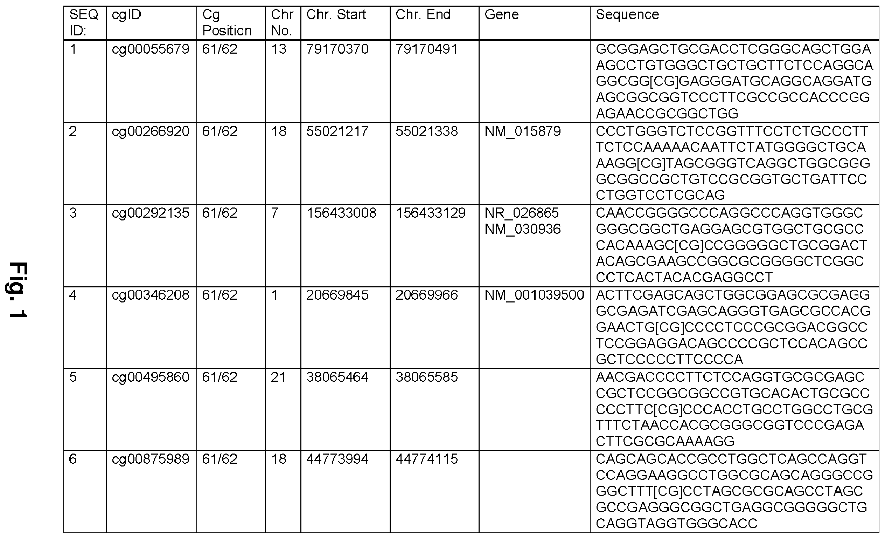

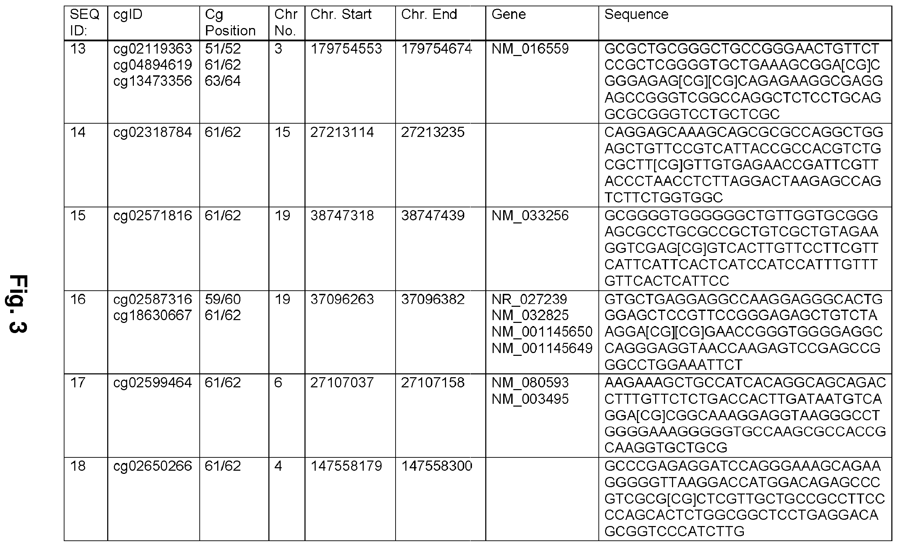

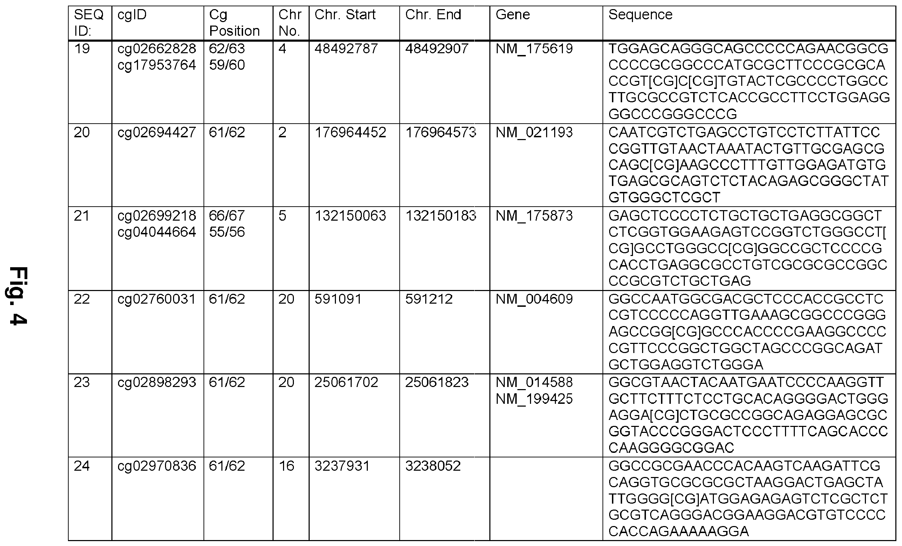

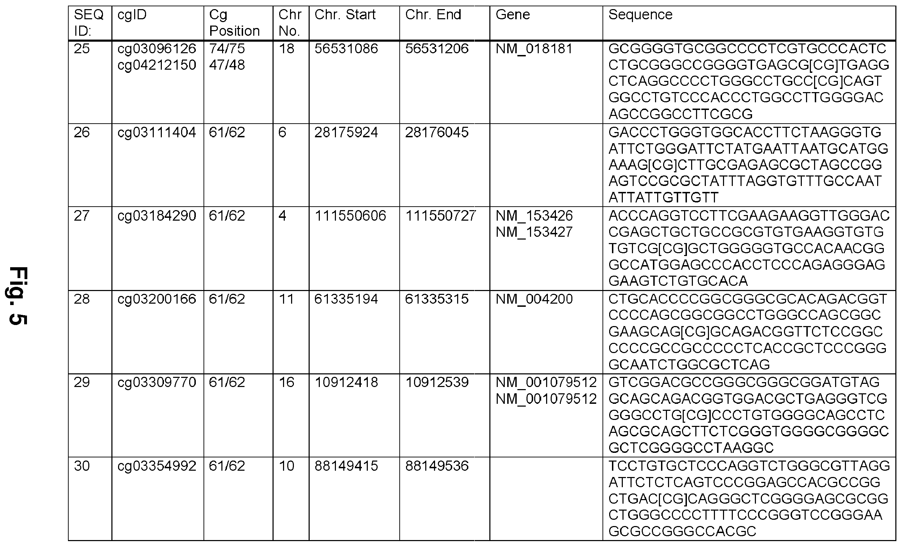

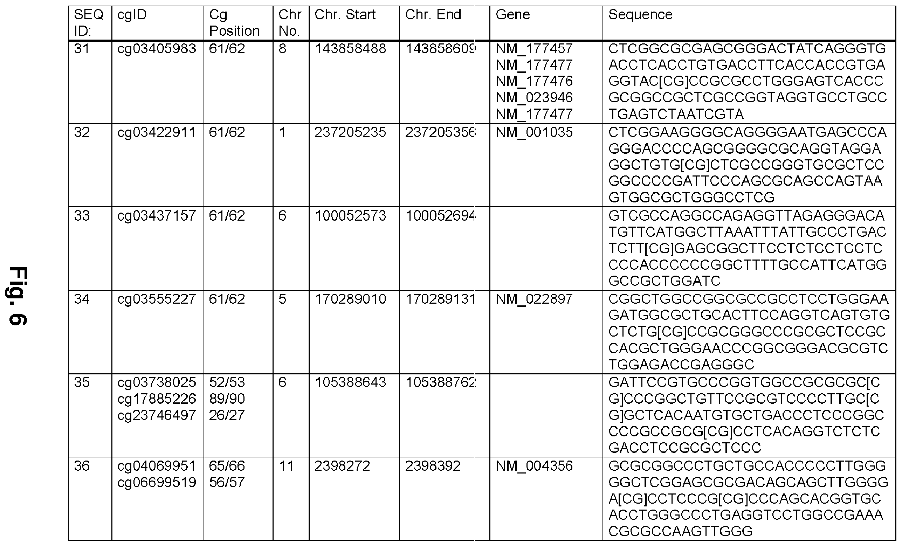

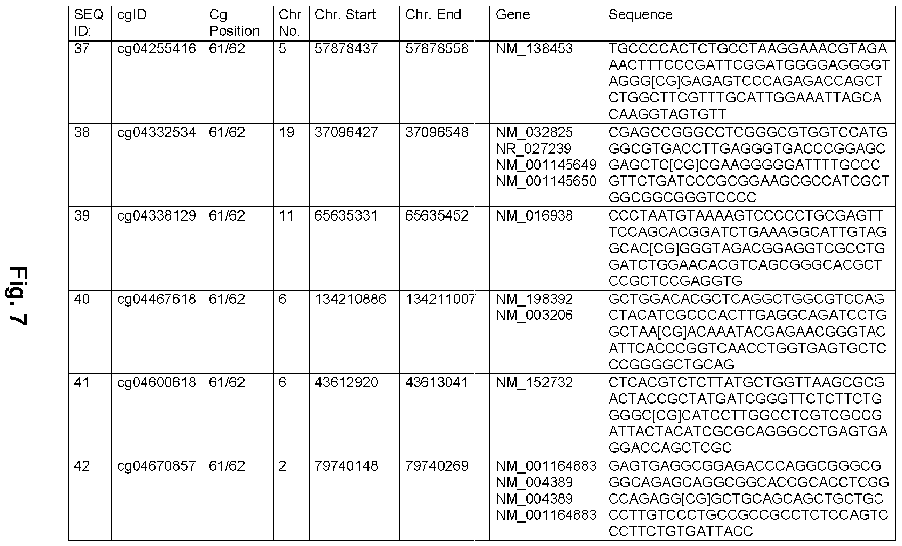

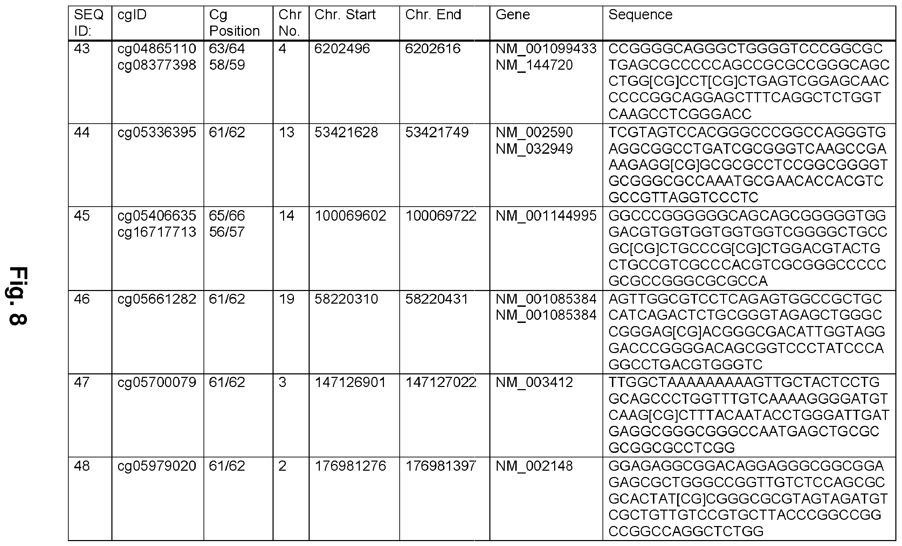

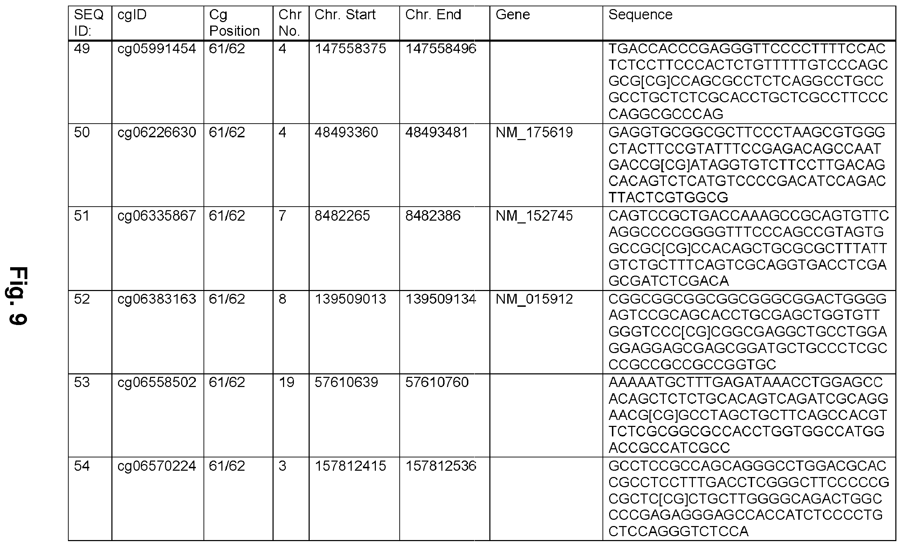

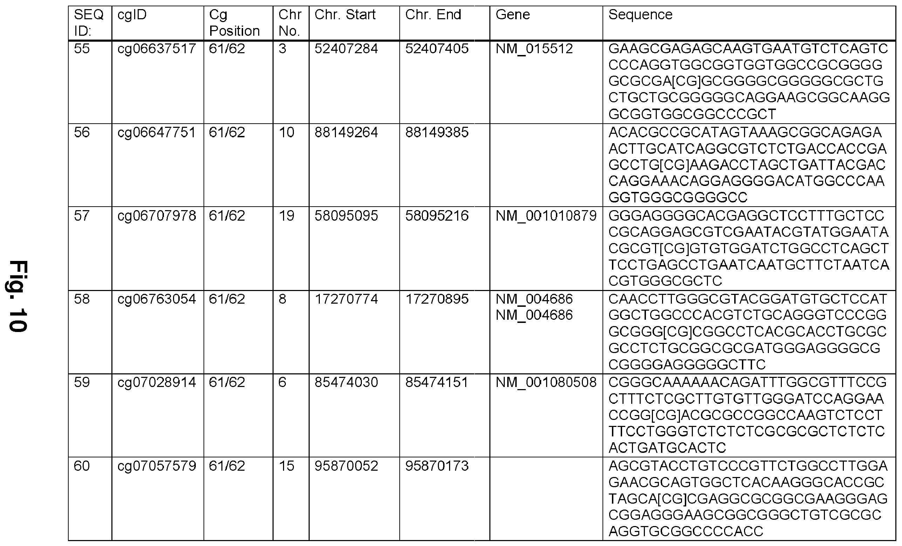

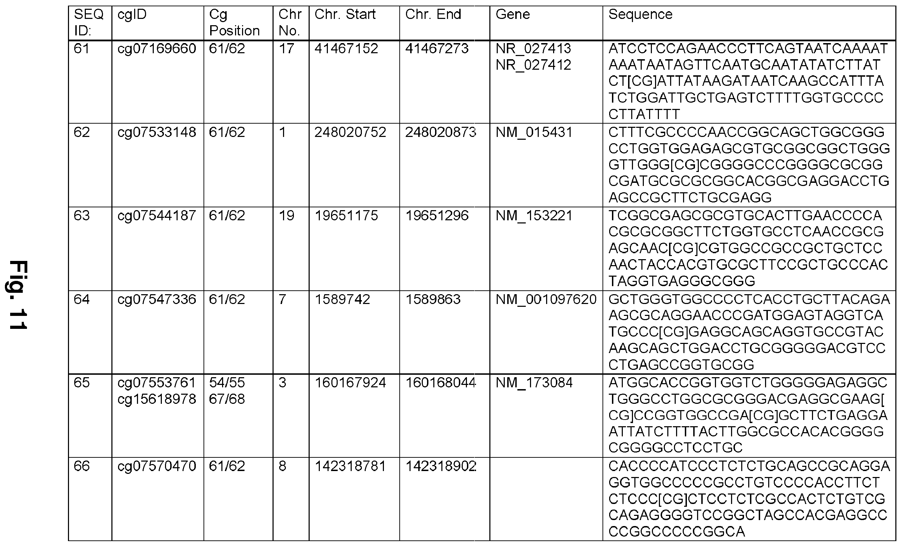

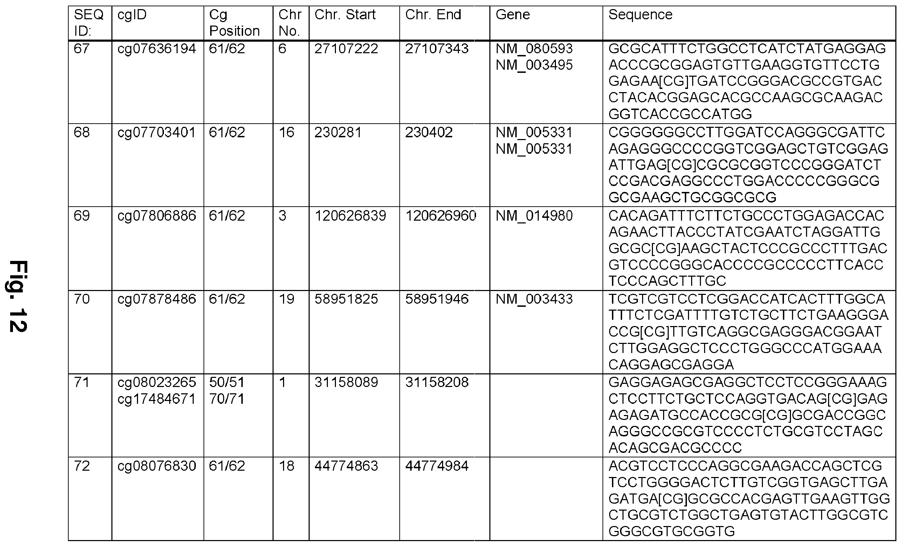

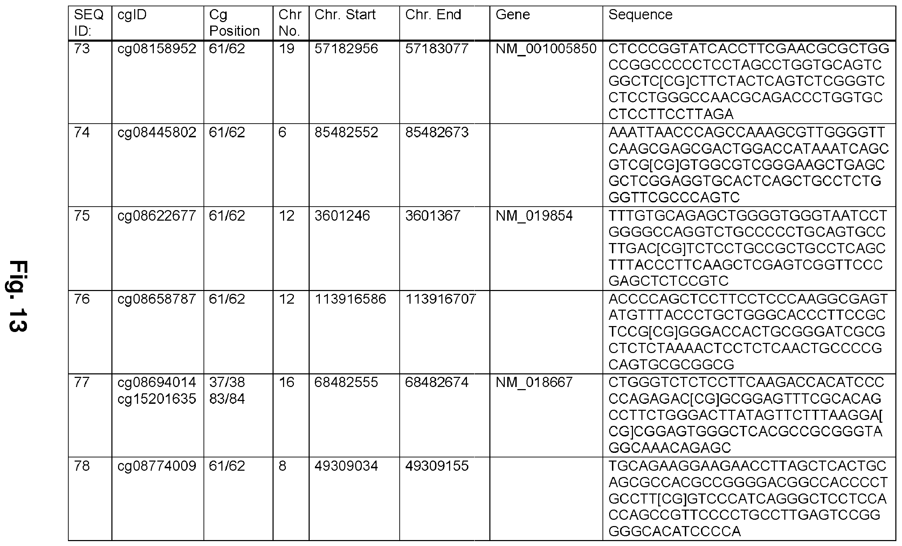

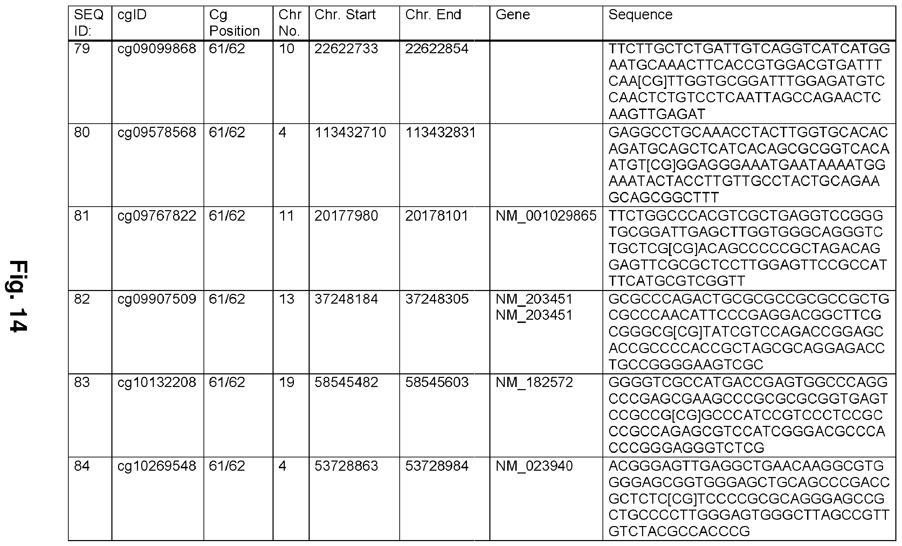

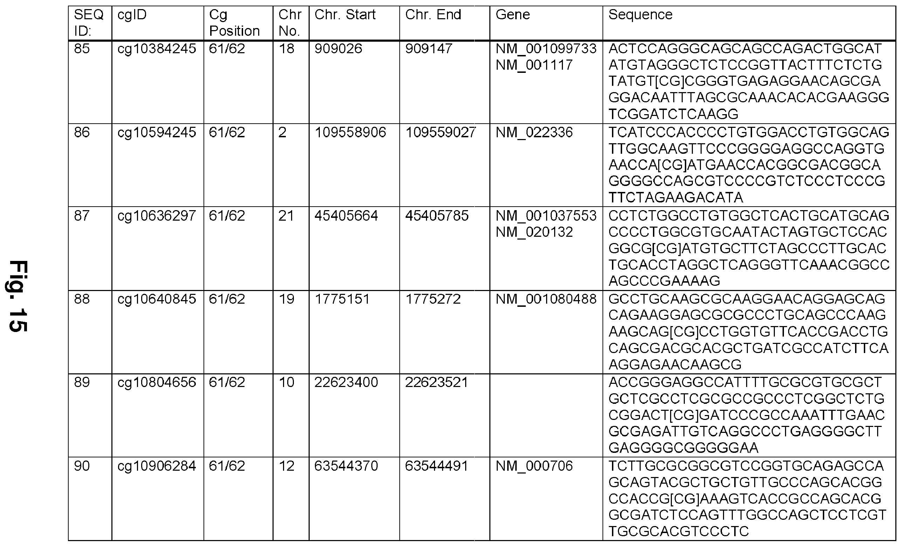

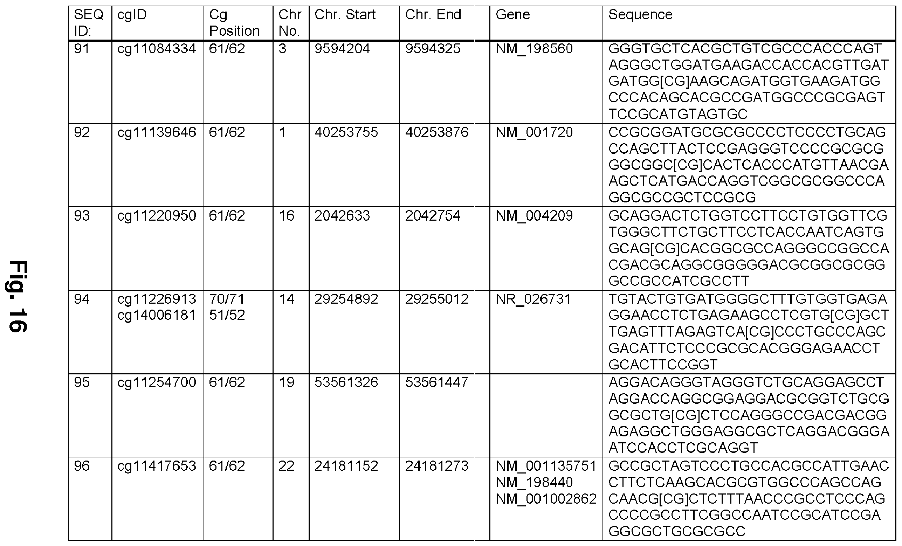

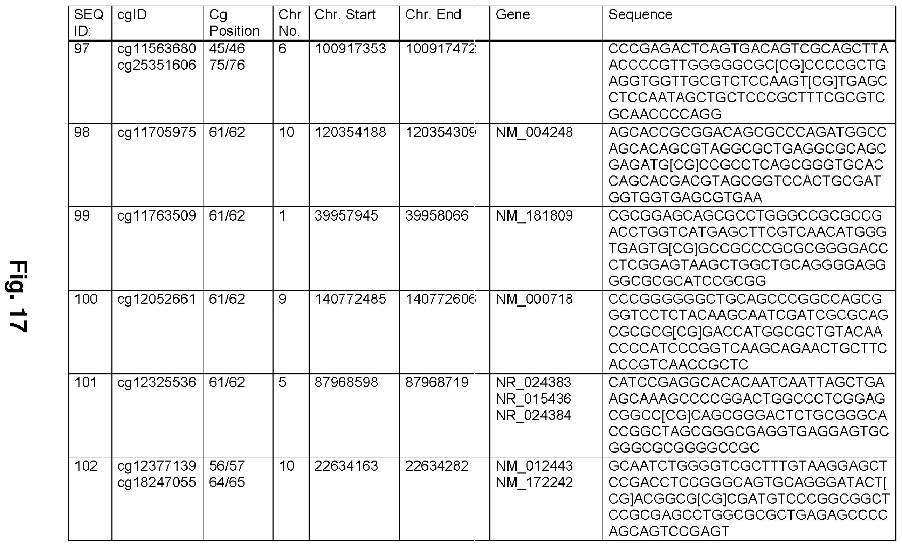

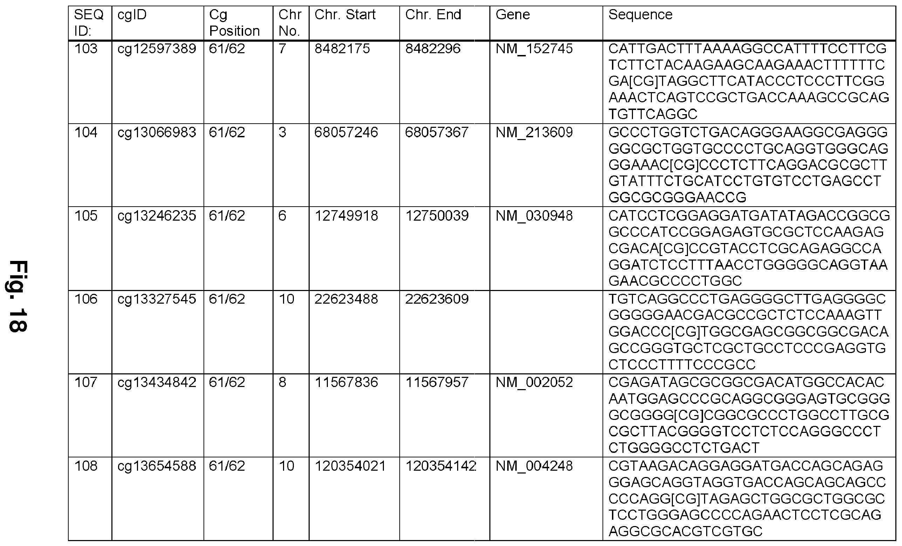

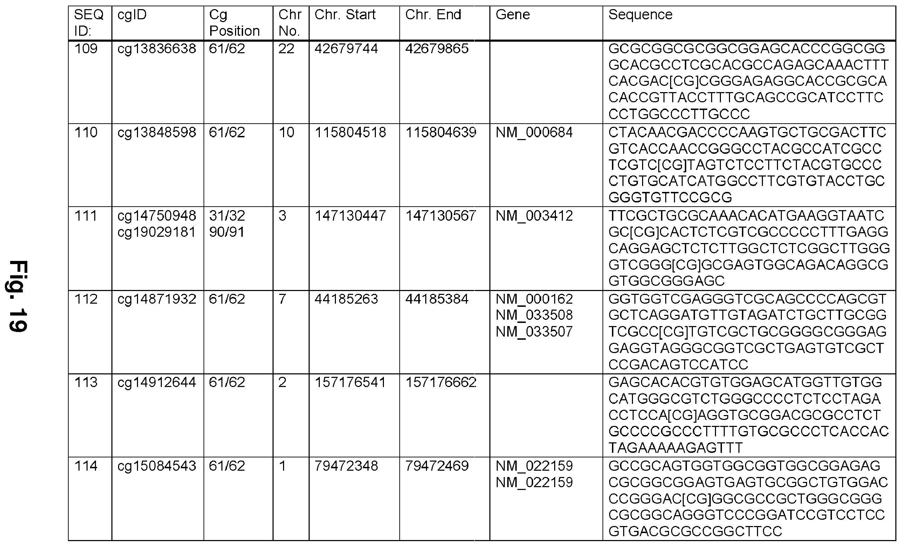

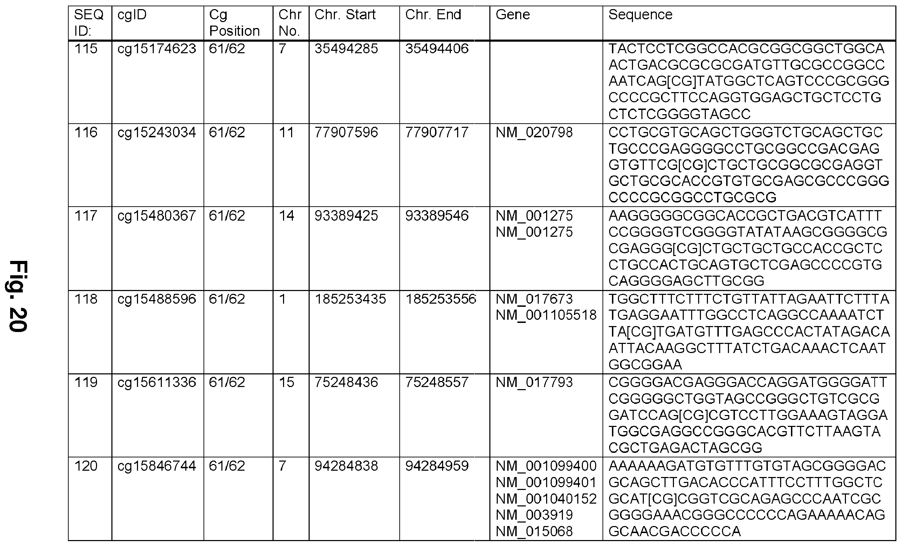

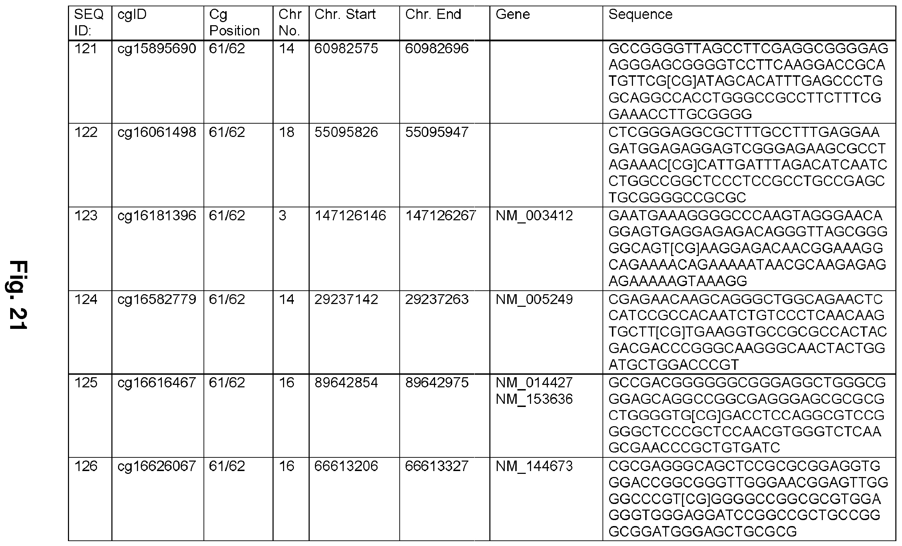

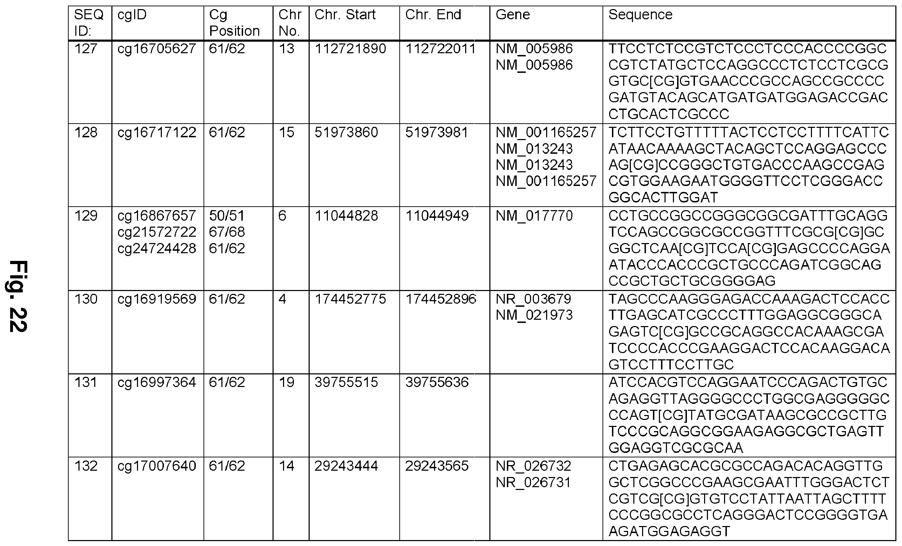

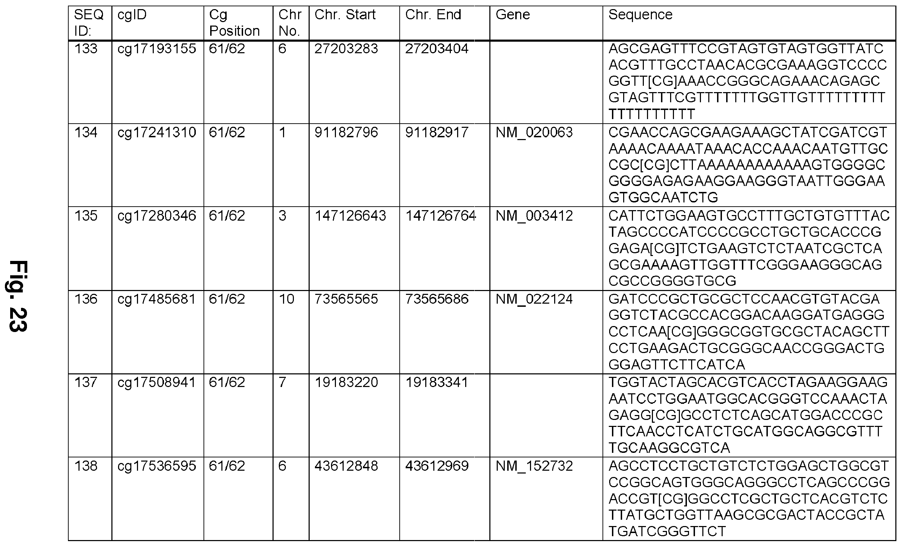

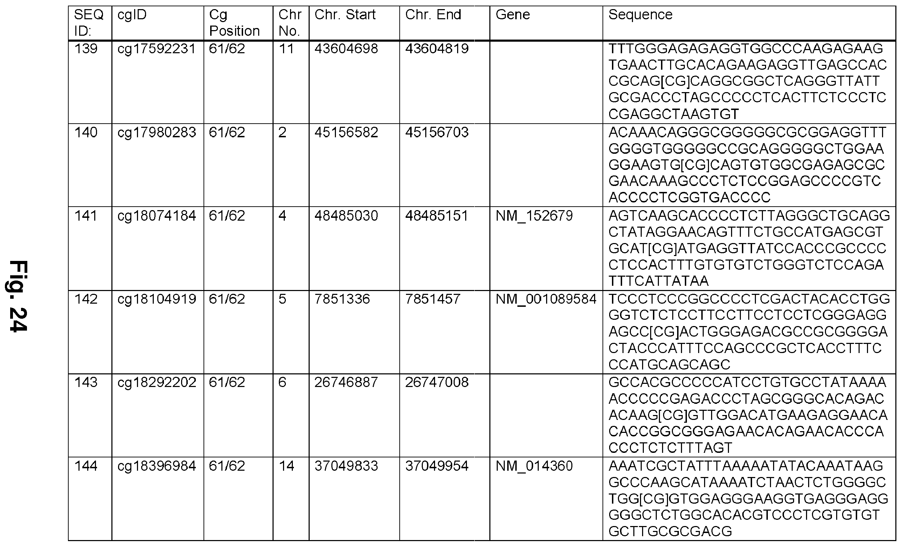

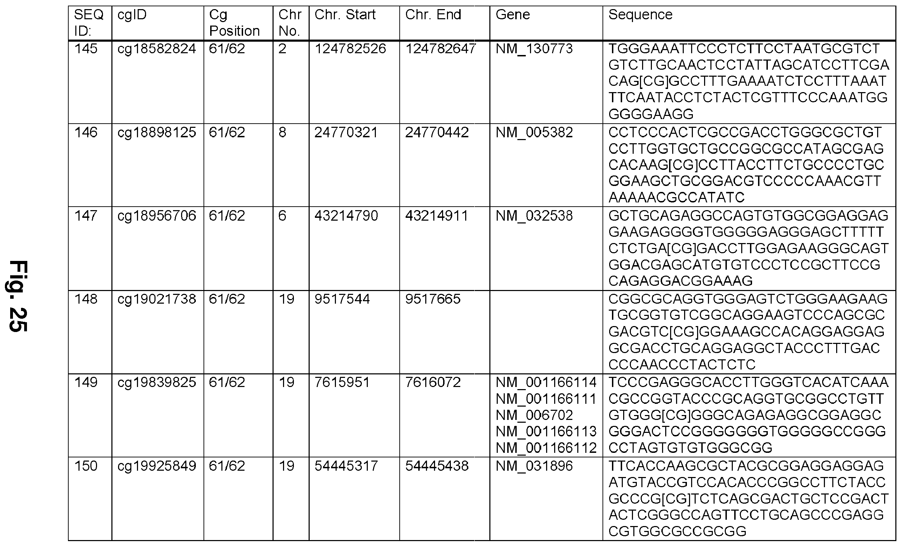

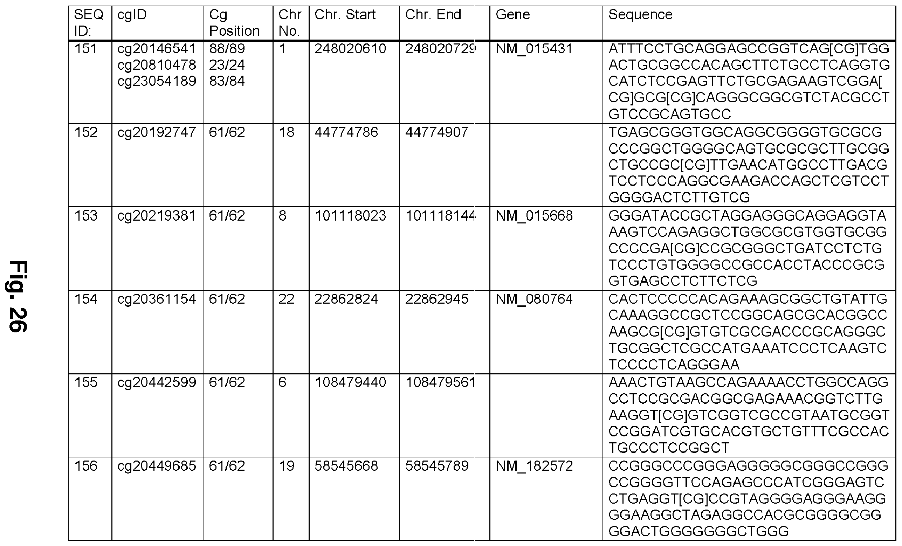

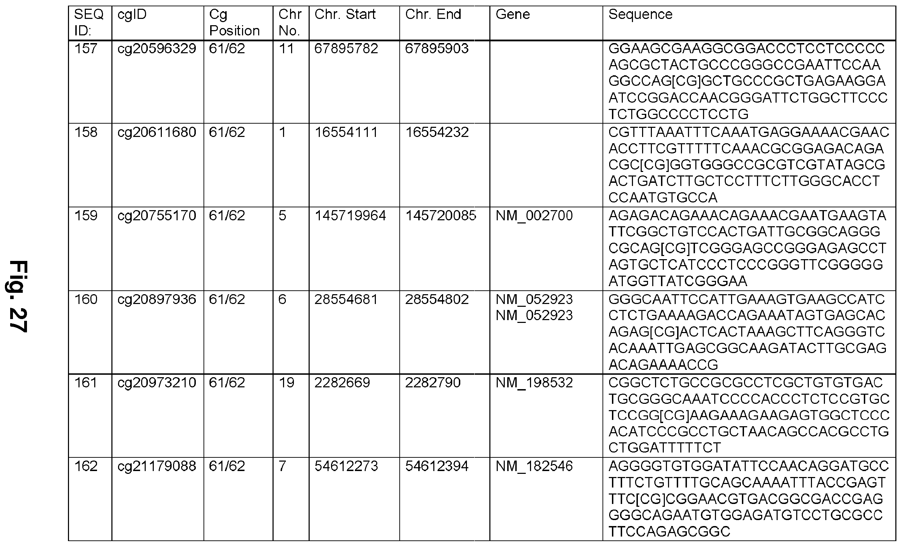

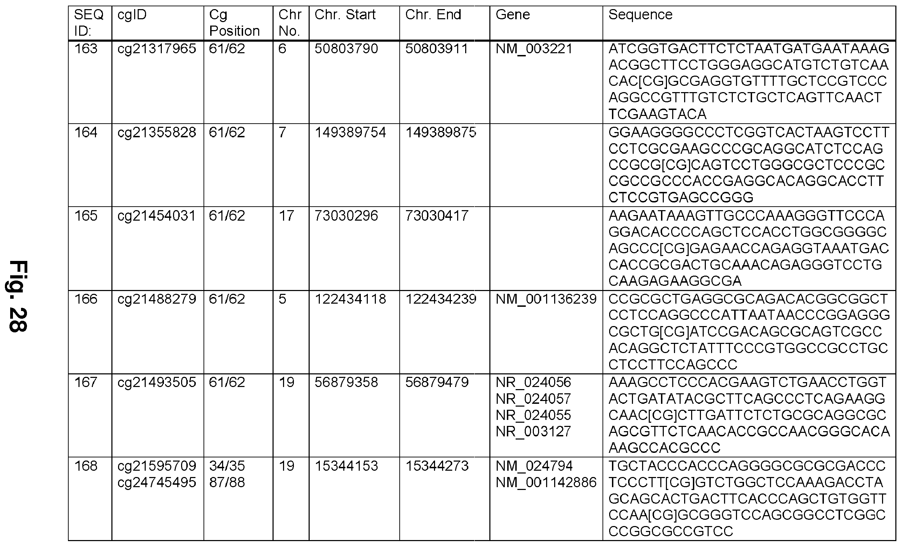

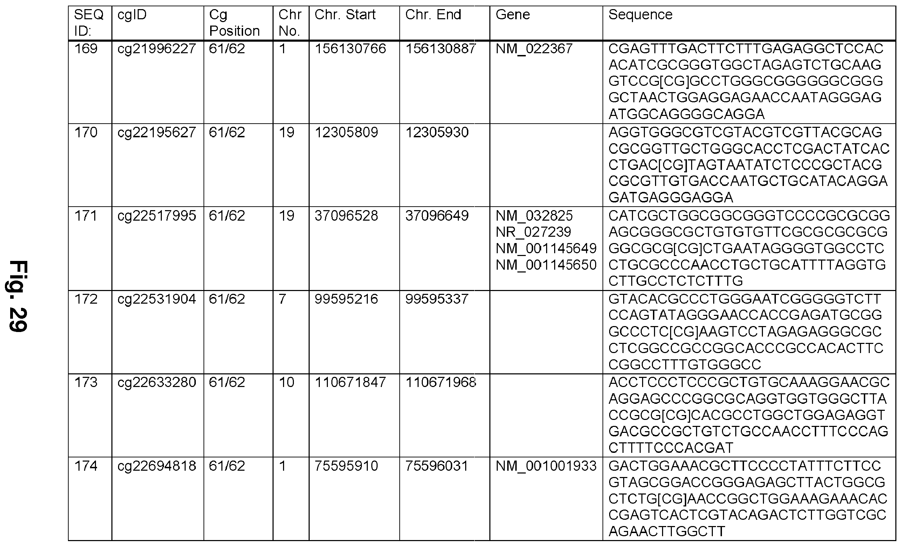

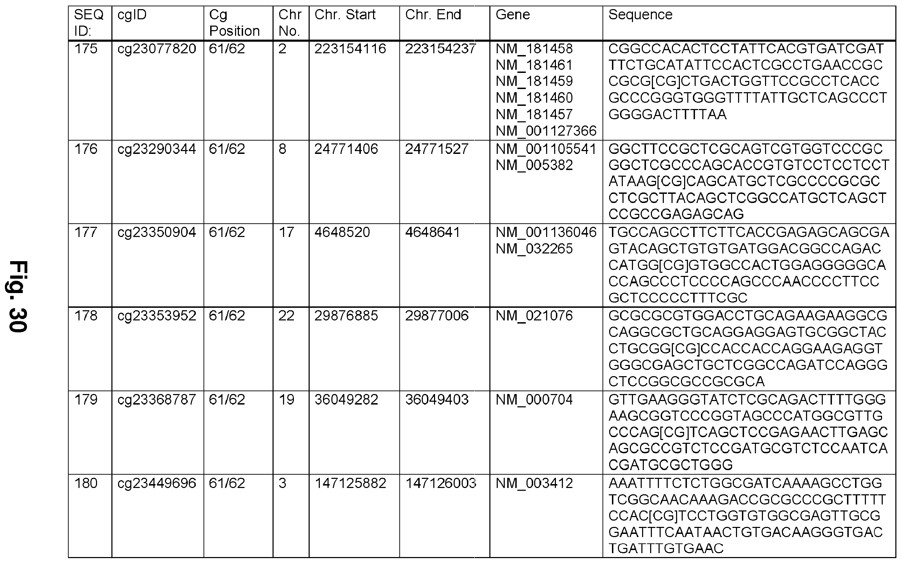

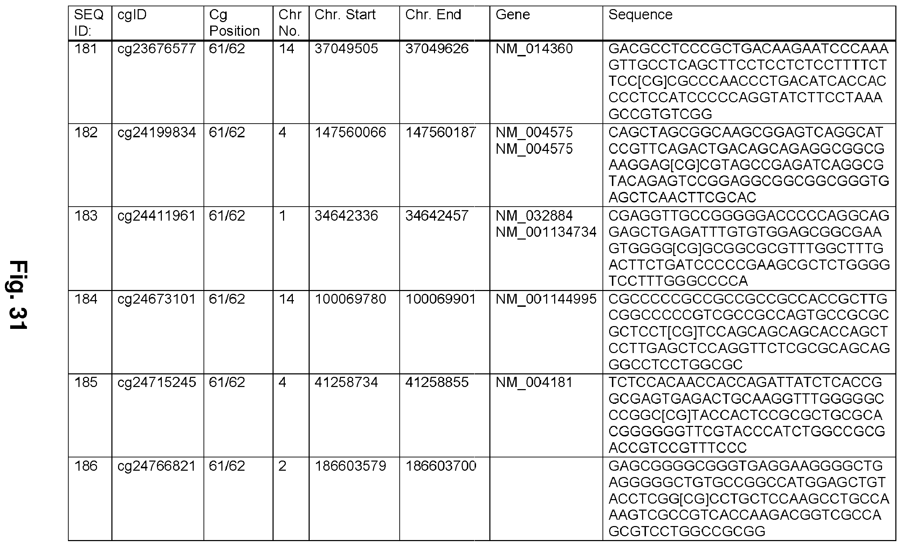

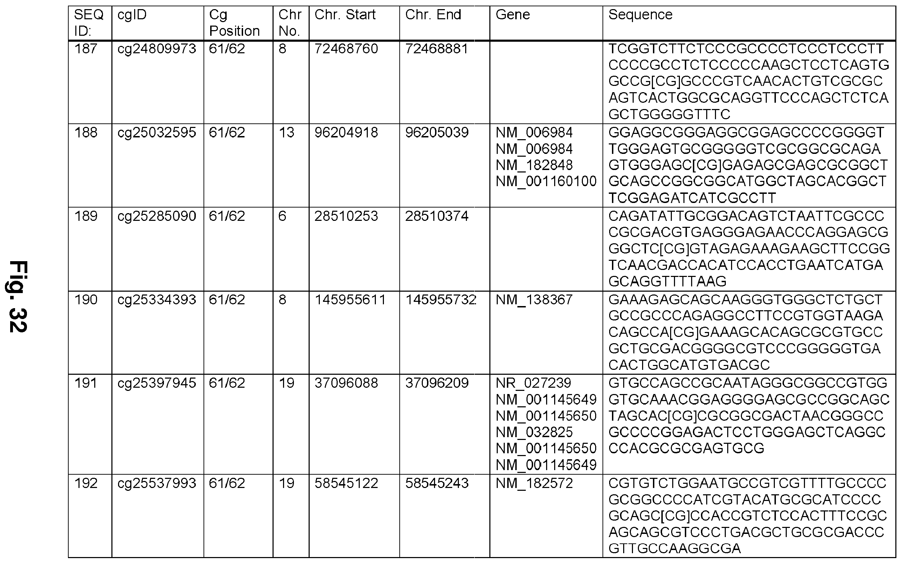

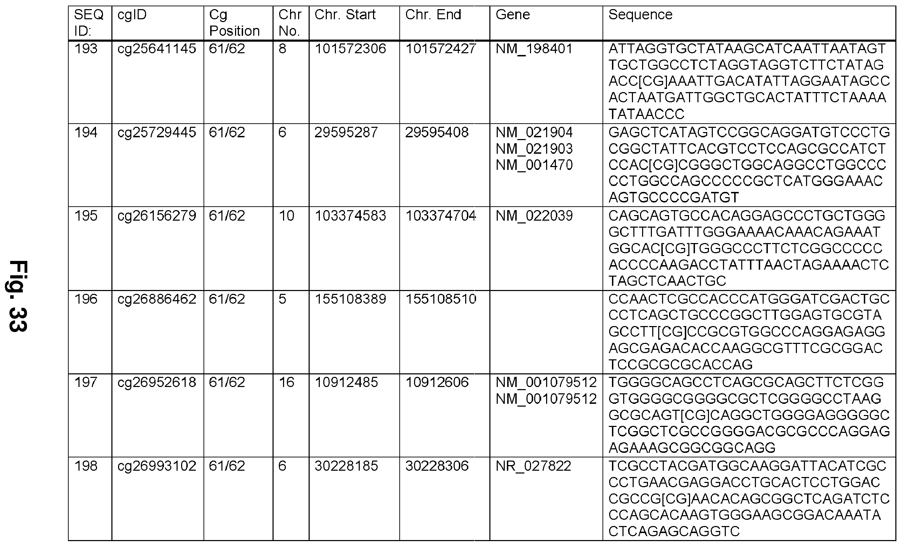

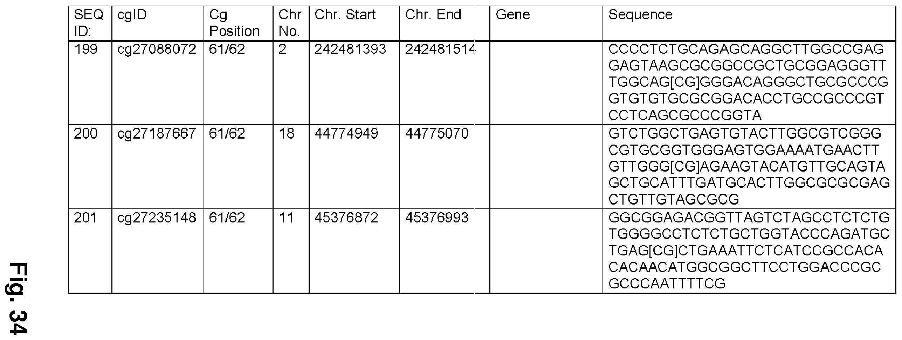

a) determining a methylation level of at least two CpG-dinucleotides of at least one nucleotide sequence selected from the group consisting of SEQ ID NO: 1 to SEQ ID NO: 201 as shown in Figures 1 to 34 of human skin cells; and
b) determining the biological age of said skin cells by comparing each determined methylation level with empirically determined data representing a correlation between the methylation level of said CpG-nucleotide and the chronological age of at least one human individual.

Disclosed herein is a method of testing an active agent comprising the method according to the first aspect, further comprising the steps of:

c) contacting the skin cells of step a) with an active agent in vitro;
d) determining the biological age of the skin cells of step c) according to the method of the first aspect; and
e) comparing the biological age determined in steps a) to b) with the biological age determined in step d).

In accordance with a second aspect, there is provided the use of a nucleic acid molecule for determining the biological age of human skin according to the method of the first aspect, wherein said nucleic acid molecule comprises at least one nucleotide sequence selected from the group consisting of:

a) a nucleotide sequence comprising at least one sequence selected from the group consisting of SEQ ID NO: 1 to SEQ ID NO: 201 as shown in Figures 1 to 34;
b) a nucleotide sequence which differs from the nucleotide sequence of a) by replacement of at most 10% of the nucleotides, preferably at most 5 of the nucleotides, but for said CpG-dinucleotide;
c) a nucleotide sequence which corresponds to the complementary strand of the nucleotide sequence a) or b).

In accordance with a third aspect, there is provided a computer-readable medium having stored computer-executable instructions for causing a computer to perform a method for determining the biological age of human skin comprising:

a) inputting at least two values of a determined methylation level of at least two CpG-dinucleotides of at least one nucleotide sequence selected from the group consisting of SEQ ID NO:1 to SEQ ID NO: 201 as shown in Figures 1 to 34 of human skin cells;
b) comparing said value of the determined methylation level with stored data representing a correlation between the methylation level of said CpG-dinucleotide and the chronological age of at least one human individual; and
c) displaying the biological age.

Disclosed herein, but not part of the invention, is a kit for determining the biological age of human skin according to the method of the first aspect, comprising at least one oligonucleotide primer for amplifying and/or sequencing at least two CpG-nucleotides of at least one nucleotide sequence selected from the group consisting of SEQ ID NO: 1 to SEQ ID NO: 201.

Certain embodiments of the present invention may provide one or more of the following advantages:

- desired ease of sample collection from the individual;
- desired speed of obtaining bioinformatic data;
- desired ease of obtaining bioinformatic data;
- desired cost-effective data collection;
- desired accuracy of age prediction.

BRIEF DESCRIPTION OF THE DRAWINGS

The invention will further be illustrated by reference to the following figures:

Fig. 1 table of CpG sites and nucleotide sequences for SEQ ID No: 1 to 6;
Fig. 2 table of CpG sites and nucleotide sequences for SEQ ID No: 7 to 12;
Fig. 3 table of CpG sites and nucleotide sequences for SEQ ID No: 13 to 18;
Fig. 4 table of CpG sites and nucleotide sequences for SEQ ID No: 19 to 24;
Fig. 5 table of CpG sites and nucleotide sequences for SEQ ID No: 25 to 30;
Fig. 6 table of CpG sites and nucleotide sequences for SEQ ID No: 31 to 36;
Fig. 7 table of CpG sites and nucleotide sequences for SEQ ID No: 37 to 42;
Fig. 8 table of CpG sites and nucleotide sequences for SEQ ID No: 43 to 48;
Fig. 9 table of CpG sites and nucleotide sequences for SEQ ID No: 49 to 54;
Fig. 10 table of CpG sites and nucleotide sequences for SEQ ID No: 55 to 60;
Fig. 11 table of CpG sites and nucleotide sequences for SEQ ID No: 61 to 66;
Fig. 12 table of CpG sites and nucleotide sequences for SEQ ID No: 67 to 72;
Fig. 13 table of CpG sites and nucleotide sequences for SEQ ID No: 73 to 78;
Fig. 14 table of CpG sites and nucleotide sequences for SEQ ID No: 79 to 84;
Fig. 15 table of CpG sites and nucleotide sequences for SEQ ID No: 85 to 90;
Fig. 16 table of CpG sites and nucleotide sequences for SEQ ID No: 91 to 96;
Fig. 17 table of CpG sites and nucleotide sequences for SEQ ID No: 97 to 102;
Fig. 18 table of CpG sites and nucleotide sequences for SEQ ID No: 103 to 108;
Fig. 19 table of CpG sites and nucleotide sequences for SEQ ID No: 109 to 114;
Fig. 20 table of CpG sites and nucleotide sequences for SEQ ID No: 115 to 120;
Fig. 21 table of CpG sites and nucleotide sequences for SEQ ID No: 121 to 126;

Fig. 22 table of CpG sites and nucleotide sequences for SEQ ID No: 127 to 132;
Fig. 23 table of CpG sites and nucleotide sequences for SEQ ID No: 133 to 138;
Fig. 24 table of CpG sites and nucleotide sequences for SEQ ID No: 139 to 144;
Fig. 25 table of CpG sites and nucleotide sequences for SEQ ID No: 145 to 150;
Fig. 26 table of CpG sites and nucleotide sequences for SEQ ID No: 151 to 156;
Fig. 27 table of CpG sites and nucleotide sequences for SEQ ID No: 157 to 162;
Fig. 28 table of CpG sites and nucleotide sequences for SEQ ID No: 163 to 168;
Fig. 29 table of CpG sites and nucleotide sequences for SEQ ID No: 169 to 174;
Fig. 30 table of CpG sites and nucleotide sequences for SEQ ID No: 175 to 180;
Fig. 31 table of CpG sites and nucleotide sequences for SEQ ID No: 181 to 186;
Fig. 32 table of CpG sites and nucleotide sequences for SEQ ID No: 187 to 192;
Fig. 33 table of CpG sites and nucleotide sequences for SEQ ID No: 193 to 198;
Fig. 34 table of CpG sites and nucleotide sequences for SEQ ID No: 199 to 201;

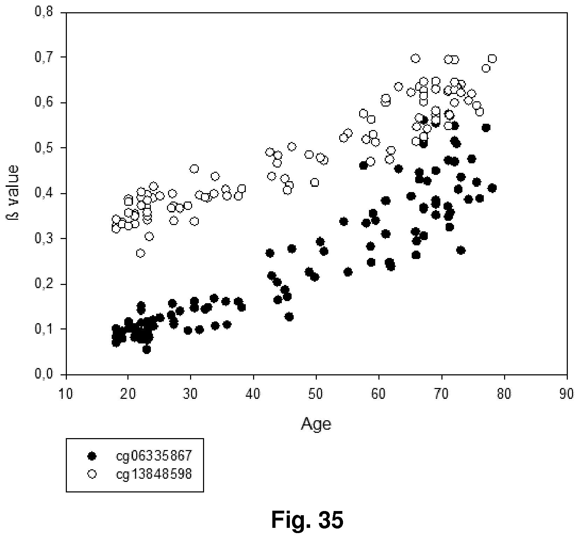

Fig. 35 graph represents the change in methylation with age for two exemplary CpGs markers: cg06335867 and cg13848598;
Fig. 36 graph represents a correlation of chronological age with biological age using the markers cg06335867 and cg13848598, using the formula: 70.002 + 8.992*cg06335867 + 12.998*cg13848598

It is understood that the following description and references to the figures concern exemplary embodiments of the present invention and shall not be limiting the scope of the claims.

DETAILED DESCRIPTION

The present invention is based on the finding that the methylation level of selected CpG nucleotides can determine the biological age of human skin.

An increase in age (aging) is the process of becoming older. Age may be viewed in various ways, such as chronological age and biological age.

As used herein, the term "chronological age" refers to the amount of time that has elapsed since the birth of an individual. The term "individual" as used herein refers to a human individual.

As used herein, the "biological age" refers to physical changes in an individual, such as the extent of epigenetic changes of DNA. The extent of epigenetic changes of DNA is determined in skin samples. The biological age is determined from skin samples and correlates to the biological age of the individual. The biological age as disclosed herein is determined using the method of the first aspect of this invention. In particular, the methylation level of specific CpG-dinucleotides are assessed as part of this invention. The methylation level can be determined, for example by methylation specific PCR, sequence analysis of bisulfite treated DNA, CHIP-sequencing (Illumina Methylation BeadChip Technology), molecular inversion probe assay, Methyl-CAP-sequencing, Next-Generation-sequencing, COBRA-Assay, methylation specific restriction patterns or MassARRAY assay.

In certain embodiments the biological age of the human skin is equal to the biological age of the individual.

Biological age may be influenced by many parameters such as genetic background, disease and lifestyle.

The biological age may differ from the chronological age. The biological age may provide an indication of the health of the individual when compared with the chronological age. If, for example, the biological age is lower than the chronological age, it may be concluded that on a biological level signs of aging are less predominant than expected. This may indicate that the individual in good health. Alternatively, if the biological age is higher than the chronological age, it may be taken as sign that the individual is in poor health.

In certain embodiments, the human skin cells are obtained by harvesting the entire skin sample required from the individual. In certain embodiments, the human skin are obtained by culturing the skin cells using an *in vitro* method.

Harvesting a sample from the individual may be carried out using suction blistering, punch biopsy, shave biopsy or during any surgical procedure such as plastic surgery, lifting, grafting, or the like.

The skin samples may be taken from the epidermis or dermis.

Human skin cells may be cultured from a small sample of skin cells harvested from an individual. The harvested human skin cells are grown in vitro in a vessel such as a petri dish in a medium or substrate that supplies essential nutrient.

The human skin cells used may be a mixture of harvested cells and cultured cells.

In certain embodiments, the specific region of the chromosome comprising CpG-dinucleotides may be coding regions or non-coding regions. In certain embodiments, the CpG dinucleotides may be present in a coding region and/or non-coding region. The CpG dinucleotides may be found in a single specific region or in different specific regions.

In the present invention, the specific region of the chromosome comprises at least one nucleotide sequence from SEQ ID NO. 1 to SEQ ID NO. 201 as shown in Figures 1 to 34. The correlation of each SEQ ID to the CpG dinucleotide according to the invention is shown in Figures 1 to 34.

In certain embodiments, the method of the first aspect further comprises the step of estimating the chronological age of human individuals. The correlation between the biological age and chronological age is high using the method according to the invention, wherein the difference between the biological age and chronological age, the mean absolute error (MAE) determined by the method described herein, is no more than about 7 years, no more than about 6 years, no more than about 5.5 years, no more than about 5 years, no more than about 4.5 years, no more than about 4 years, no more than about 3.5 years, no more than about 3 years, no more than about 2 years, no more than about 1.5 years, no more than about 1 year, no more than about 0.5 years, no more than 0 years.

The difference in biological age and chronological age may be determined for a single individual. The deviation of a single data point from the best fit line superimposed onto the data points from all individuals can vary from about 0 years to about 15 years. For example, the deviation of a single data point from the best fit line would be about 0 years, about 1 year, about 2 years, about 3 years, about 4 years about 5 years, about 6 years, about 7 years, about 8 years, about 9 years, about 10 years, about 11 years, about 12 years, about 13 years, about 14 years, about 15 years. This is an indication of the health status of the human individual and the human skin.

In certain embodiments, the biological age of an unknown individual is determined as described above. From this biological age, the chronological age of the individual is estimated by applying the expected MAE for the CpG data points used.

In certain embodiments, the biological age and chronological age are used to calculate a value h, which is indicative of the health of a human individual, and the human skin. In certain embodiments, the value h is calculated according to formula (I)

$$h = \text{biological age} - \text{chronological age} \quad (I)$$

Without wishing to be bound by theory it is considered that if the value for h obtained from formula (I) is positive, the human individual is in poor health. In this case, a larger h number is indicative of the health of the human individual being worse than a smaller h number. If the value of h obtained from formula (I) is negative, it may be assumed that the individual is in good health.

If the value for h is positive, a reduction of this value may be desired, thereby improving the health of the individual. The h value may be reduced by a number of means, such as exercising, eating healthily, sleeping the right amount, drinking enough water and/or avoiding stress.

The h value may also be reduced by applying active agents onto the skin cells in the form of pharmaceutical and/or cosmetic agents. The active agent may contact the skin cells *in vitro* or *in vivo.* Using the *in vitro* method, the active agent is added to the culture medium of skin cells. Using the *in vivo* method, the skin cells of a human individual may be contacted with the active agent using topical, subcutaneous or intradermal administration.

An "active agent" as used herein is any agent that has a therapeutic effect and/or cosmetic effect on the individual. A therapeutic effect is the treatment and/or prevention of a disease. A cosmetic effect is the improvement of appearance, such as treating and/or preventing the signs of molecular aging. Signs of molecular aging are, for example, stem cell exhaustion, altered intercellular communication, genomic instability, telomere attrition, epigenetic alterations, loss of proteostasis, deregulated nutrient sensing, mitochondrial dysfunction, cellular senescence and changed proliferative capacity. In certain embodiments the active agent is a cosmetic agent. A cosmetic agent when applied to an individual may promote attractiveness, alter appearance, beautify and/or cleanse. The cosmetic agent may also prevent and/or treat the signs of phenotypical aging of human skin are for example wrinkle formation, pale complexion, reduced wound healing capacity, loss of elasticity and tightness.

In certain embodiments, the biological age is determined before and after contacting the skin cells with an active agent. For example, the biological age is determined, the skin cell are contacted with an active agent, followed by determining the biological age. The biological age before and after contacting the skin cells with an active agent are compared. A reduction in the biological age after treatment indicates a therapeutic effect and/or cosmetic effect of the active agent. The time between contacting the skin cells with an active agent and determining the biological age may be varied. The steps of contacting the skin cells with an active agent and determining the biological age may be repeated to provide information about the effect of the active agent on the skin cells over time. This method may have the advantage of being a fast and cost effective way to identify active cosmetic and/or pharmaceutical compounds and/or extracts.

In certain embodiments, the method according to the invention may have one or more of the following effects:

- increased accuracy of estimation of chronological age from biological age;
- increased reliability of correlation between chronological age and biological age;
- reduced cost of determining the biological age;
- reduced time required to determine the biological age;
- efficient method to determining the effect of pharmaceutical or cosmetic agents on biological age.

The foregoing description is directed to particular embodiments of the present invention for the purpose of illustrating it. It will be apparent, however, to one skilled in the art, that many modifications and variations to the embodiments described herein are possible and the scope of the present invention is as defined in the appended claims.

## EXAMPLES

### Skin samples and patient group

Data of 108 epidermal samples (Bormann, Aging Cell 2016) from DNA-methylation arrays (Illumina 450k BeadChip) was collected. To obtain the age-associated CpG sites, the correlation coefficient (r) between the chronological age of the samples and corresponding beta-values for each CpG site were calculated. After pre-filtering, 225 age-associated CpG sites ($r > 0.8$ or $r < -0.8$) were selected. A multivariable linear model was trained using the complete set of 225 CpG sites.

In order to find suitable CpG combinations, the complete sample set was divided into training set and test set. Single CpGs or CpG combinations of at least two CpGs were chosen due to their prediction accuracy.

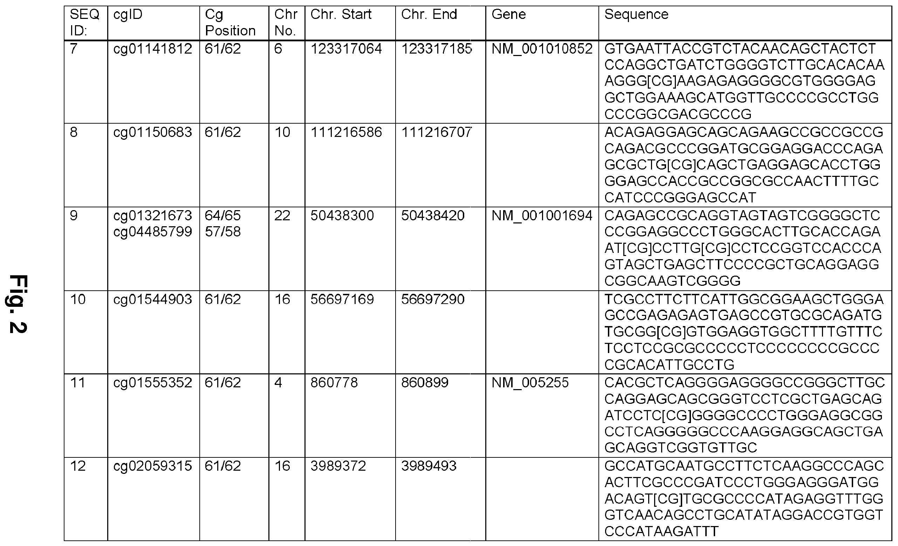

A correlation of the age dependent change in methylation is shown in Figure 2 for two CpGs markers: cg06335867 and cg13848598.

Skin samples were collected from healthy volunteers aged between 23 and 54 years using suction blistering. For whole epidermis samples, the epidermal parts of the skin were separated from the dermal parts by heat split. To isolate keratinocytes, epidermis was separated from dermis by dispase II (Roche 04942078001) digestion, and keratinocyte were isolated after trypsin (Life Technologies #25200056) treatment.

### DNA isolation and bisulfite conversion

The DNA was isolated using the QIAmp DNA Investigator Kit (Qiagen #56504). The bisulfite conversion of 500 ng of DNA was carried out using *in vitro* cultivated human keratinocytes and *ex vivo* human epidermis samples with an EpiTect Bisulfite Kit (Qiagen #59104). The DNA was converted by following manufacturer's protocol, but wherein an incubation time of 4h 55 min was used instead of 2h 55 min.

### Amplicon PCR and Sequencing

To analyse the CpGs, amplicons were generated using PCR. The PCR was carried out using the bisulfite converted DNA and the PyroMark PCR Kit (Qiagen #978703). The PCR composition is shown in Table 1. The PCR was performed with an annealing temperature of 55°C.

Table 1: PCR composition

| Component[1] | Amount |
|---|---|
| Bisulfite converted DNA | 50 ng in 2 μl of water |
| Mastermix (2×) | 12.5 μl |
| Coral loading dye | 2.5 μl |
| $MgCl_2$ (25 mM) | 1 μl |
| Forward primer (10 μM) | 0.5 μl |
| Reverse primer (10 μM) | 0.5 μl |
| water | 6 μl |
| [1] $MgCl_2$, forward primer and reverse primer were used as aqueous solutions. | |

The PCR fragments were unravelled in a 1% agrosegel in TAE buffer solution and cut out of the gel. The amplicons were purified using a Qiaquick gel extraction kit (Qiagen #28706).

The DNA sequencing was carried out using a 454-sequencing approach (Roche) according to the manufacturer's protocol.

The extent of methylation was determined from the proportion of methylated and unmethylated Reads.

**Calculating the biological age**

To calculate the biological age, the proportion of methylation at a CpG-Locus was determined, to provide the beta-value. The average beta-value ($\beta$) was calculated for each CpG locus and each sample. It was then converted to the m-value (m) using formula (1):

$$m = \log2(\beta /1- \beta) \qquad (1)$$

The m-value may then be used in, for example, in one of the stated equations (2) to (4) for cg06335867 and cg13848598:

$$\text{Biological age} = 70.002 + 8.992*\text{cg06335867} + 12.998*\text{cg13848598} \qquad (2)$$

$$\text{Biological age} = 68.785 + 7.346*\text{cg06335867} + 15.865*\text{cg13848598} \qquad (3)$$

$$\text{Biological age} = 60.372 + 6.923*\text{cg06335867} + 12.904*\text{cg13848598} \qquad (4)$$

Example 1

Skin samples were collected from 6 individuals an analysed according to the methods provided above. The CpG-nucleotides analysed were cg06335867 and cg13848598. The following calculation of biological age were carried out using equation (2) and compared to the chronological age.

Subject 1

Chronological age: 30 years old 12

Marker cg06335867:

Measured $\beta$ - value (cg06335867): 0.1702
Calculated m - value (cg06335867) = log2 (0.1702/1-0.1702) = -2.2855326

Marker cg13848598:

Measured $\beta$ - value (cg13848598): 0.25
Calculated m-value (cg13848598) = log2 (0.25/1-0.25) = -1.5849625

Biological age:

$$70.002 + 8.992 \times (-2.2855326) + 12.998 \times (-1.5849625) = 28.85 \text{ years old}$$

Difference between chronological and biological age: 1.15 years.

Subject 2

Chronological age: 35 years old

Marker cg06335867:

Measured $\beta$ - value (cg06335867): 0.2286
Calculated m - value (cg06335867) = log2 (0.2286/1-0.2286) = -1.7546537

Marker cg13848598:

Measured $\beta$ - value (cg13848598): 0.3775

Calculated m-value (cg13848598) = log2 (0.3775/1-0.3775) = -0.7215972

Biological age:

70.002 + 8.992 × (-1.7546537) + 12.998 × (-0.7215972) = 44.84 years old

Difference between chronological and biological age: 9.84 years.

Subject 3

Chronological age: 57 years old

Marker cg06335867:

Measured β - value (cg06335867): 0.1681

Calculated m - value (cg06335867) = log2 (0.1681/1-0.1681) = -2.3070904

Marker cg13848598:

Measured β - value (cg13848598): 0.5514
Calculated m-value (cg13848598) = log2 (0.5514/1-0.5514) = 0.2976696

Biological age:

70.002 + 8.992 × (-2.3070904) + 12.998 × (0.2976696) = 53.13 years hold

Difference between chronological and biological age: 3.87 years.

Subject 4

Chronological age: 72 years old

Marker cg06335867:

Measured β - value (cg06335867): 0.3262
Calculated m - value (cg06335867) = log2 (0.3262/1-0.3262) = -1.0465636

Marker cg13848598:

Measured β - value (cg13848598): 0.6071
Calculated m-value (cg13848598) = log2 (0.6071/1-0.6071) = 0.62777201

Biological age:

70.002 + 8.992 × (-1.0465636) + 12.998 × (0.62777201) = 68.75 years hold

Difference between chronological and biological age: 3.25 years.

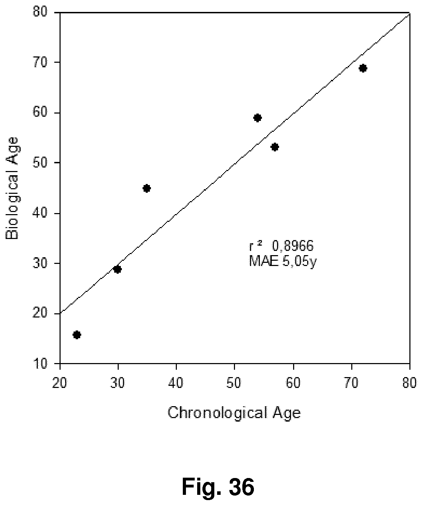

The calculated biological age was plotted against the chronological age of each one of six individuals, as seen in Figure 36. The mean absolute error across these data points was 5.05 years, which demonstrates a high degree of correlation between the calculated biological age according to the invention and chronological age. Furthermore, a good correlation was seen at all ages. Since the method predicts biological age with high accuracy over all samples, deviating biological age is indicative of the health status of a single individual.

## Claims

1. A method for determining the biological age of human skin comprising:

   a) determining a methylation level of at least two CpG-dinucleotides of at least one nucleotide sequence selected from the group consisting of SEQ ID NO: 1 to SEQ ID NO: 201 as shown in Figures 1 to 34 of human skin cells; and
   b) determining the biological age of said skin cells by comparing each determined methylation level with empirically determined data representing a correlation between the methylation level of said CpG-nucleotide and the chronological age of at least one human individual.

2. The method according to claim 1, wherein the at least one nucleotide sequence is selected from the group consisting of SEQ ID NO: 51 and SEQ ID NO: 110.

3. The method of any one of the preceding claims, wherein said data comprises at least one linear regression equation, preferably one equation which comprises at least two linear regressions.

4. The method according to claims 1 to 3, further comprising the step of calculating a value h indicative of the health of a human individual, where h is calculated based on the biological age and chronological age.

5. The method according to claim 4, wherein h is calculated based on the difference between biological age and chronological age.

6. A method of testing an active agent comprising the method of any one of claims 1 to 3, further comprising the steps of:

   c) contacting the human skin cells with an active agent *in vitro;*
   d) determining the biological age of the skin cells of step c) according to the method of any one of claims 1 to 3; and
   e) comparing the biological age determined in steps a) to b) with the biological age determined in step d).

7. The method of claim 6, wherein the active agent is a cosmetic agent and/or a therapeutic agent.

8. A method of claim 6 or claim 7 to identify an active agent that prevents and/or treats the signs of phenotypical aging of human skin.

9. Use of a nucleic acid molecule for determining the biological age of human skin according to the method of any one of claims 1 to 3, wherein said nucleic acid molecule comprises at least one nucleotide sequence selected from the group consisting of:

a) a nucleotide sequence comprising at least one sequence selected from the group consisting of SEQ ID NO: 1 to SEQ ID NO: 201 as shown in Figures 1 to 34;
b) a nucleotide sequence which differs from the nucleotide sequence of a) by replacement of at most 10% of the nucleotides, preferably at most 5 of the nucleotides, but for said CpG-dinucleotide;
c) a nucleotide sequence which corresponds to the complementary strand of the nucleotide sequence a) or b).

**10.** Computer-readable medium having stored computer-executable instructions for causing a computer to perform a method for determining the biological age of human skin comprising:

a) inputting at least two values of a determined methylation level of at least two CpG-dinucleotides of at least one nucleotide sequence selected from the group consisting of SEQ ID NO: 1 to SEQ ID NO: 201 as shown in Figures 1 to 34 of human skin cells;
b) comparing each value of the determined methylation level with stored data representing a correlation between the methylation level of said CpG-dinucleotide and the chronological age of at least one human individual; and
c) displaying the biological age.

**11.** Computer-readable medium according to claim 10, wherein said stored data comprise at least one linear regression equation, preferable one equation which comprises at least two linear regressions.

**Patentansprüche**

**1.** Verfahren zum Bestimmen des biologischen Alters menschlicher Haut, umfassend:

a) Bestimmen eines Methylierungsgrads von mindestens zwei CpG-Dinukleotiden aus mindestens einer Nukleotidsequenz, ausgewählt aus der Gruppe SEQ ID NR.: 1 bis SEQ ID NR.: 201, wie in Figuren 1 bis 34 gezeigt, menschlicher Hautzellen; und
b) Bestimmen des biologischen Alters der Hautzellen durch Vergleichen jedes bestimmten Methylierungsgrads mit empirisch bestimmten Daten, die eine Korrelation zwischen dem Methylierungsgrad des CpG-Nukleotids und dem chronologischen Alter von mindestens einem menschlichen Individuum darstellt.

**2.** Verfahren gemäß Anspruch 1, worin die mindestens eine Nukleotidsequenz ausgewählt ist aus der Gruppe SEQ ID NR.: 51 und SEQ ID NR.: 110.

**3.** Verfahren gemäß irgendeinem der vorhergehenden Ansprüche, wobei die Daten mindestens eine lineare Regressionsgleichung umfassen, vorzugsweise eine Gleichung, die mindestens zwei lineare Regressionen umfasst.

**4.** Verfahren gemäß irgendeinem der Ansprüche 1 bis 3, ferner umfassend den Schritt des Berechnens eines Werts h, welcher die Gesundheit eines menschlichen Individuums angibt, wobei h auf der Grundlage des biologischen Alters und des chronologischen Alters berechnet wird.

**5.** Verfahren gemäß Anspruch 4, wobei h auf der Grundlage des Unterschieds zwischen dem biologischen Alter und dem chronologischen Alter berechnet wird.

**6.** Verfahren zum Testen eines Wirkstoffs, umfassend das Verfahren aus irgendeinem der Ansprüche 1 bis 3, ferner umfassend die Schritte:

c) Zusammenbringen der Hautzellen mit einem Wirkstoff *in vitro;*
d) Bestimmen des biologischen Alters der Hautzellen aus Schritt c) nach dem Verfahren aus irgendeinem der Ansprüche 1 bis 3; und
e) Vergleichen des in Schritten a) bis b) bestimmten biologischen Alters mit dem in Schritt d) bestimmten biologischen Alter.

**7.** Verfahren gemäß Anspruch 6, wobei der Wirkstoff ein kosmetisches Mittel und/oder ein therapeutisches Mittel ist.

8. Verfahren gemäß Anspruch 6 oder Anspruch 7 zur Identifizierung eines Wirkstoffs, der die Anzeichen phänotypischer Alterung menschlicher Haut verhindert und/oder behandelt.

9. Verwendung eines Nukleinsäuremoleküls zum Bestimmen des biologischen Alters menschlicher Haut nach dem Verfahren aus irgendeinem der Ansprüche 1 bis 3, wobei das Nukleinsäuremolekül mindestens eine Nukleotidsequenz umfasst, ausgewählt der Gruppe

   a) eine Nukleotidsequenz, umfassend mindestens eine Sequenz, ausgewählt aus der Gruppe SEQ ID NR.: 1 bis SEQ ID NR.: 201, wie in Figuren 1 bis 34 gezeigt;
   b) eine Nukleotidsequenz, die sich von der Nukleotidsequenz aus a) unterscheidet durch Austausch höchstens 10% der Nukleotide, bevoryugt höchstens 5 der Nukleotide, mit Ausnahme der CpG-Dinukleotide;
   c) eine Nukleotidsequenz, die dem komplementären Strang der Nukleotidsequenz a) oder b) entspricht.

10. Rechnerlesbares Medium mit darauf gespeicherten, von einem Rechner ausführbaren, Anweisungen, die den Rechner dazu veranlassen, ein Verfahren zum Bestimmen des biologischen Alters menschlicher Haut durchzuführen, umfassend:

   a) Eingeben von mindestens zwei Werten eines bestimmten Methylierungsgrads von mindestens zwei CpG-Dinukleotiden einer Nukleotidsequenz, ausgewählt aus der Gruppe SEQ ID NR.: 1 bis SEQ ID NR.: 201, wie in Figuren 1 bis 34 gezeigt;
   b) Vergleichen des Werts des bestimmten Methylierungsgrads mit gespeicherten Daten, die eine Korrelation zwischen dem Methylierungsgrad des CpG-Dinukleotids und dem chronologischen Alter von mindestens einem menschlichen Individuum darstellt; und
   c) Anzeigen des biologischen Alters.

11. Rechnerlesbares Medium gemäß Anspruch 10, wobei die gespeicherten Daten mindestens eine lineare Regressionsgleichung umfassen, vorzugsweise eine Gleichung, die mindestens zwei lineare Regressionen umfasst.

## Revendications

1. Un procédé pour déterminer l'âge biologique de peau humaine comprenant :

   a) déterminer un niveau de méthylation d'au moins deux dinucléotides CpG d'au moins une séquence de nucléotides sélectionnée parmi le groupe constitué en SEQ ID NO.: 1 à SEQ ID NO.: 201 comme montré dans les figures 1 à 34 de cellules de peau humaine ; et
   b) déterminer l'âge biologique desdites cellules de peau en comparant chaque niveau de méthylation déterminé avec des données déterminées empiriquement qui représentent une corrélation entre le niveau de méthylation dudit nucléotide CpG et l'âge chronologique d'au moins un individu humain.

2. Le procédé selon la revendication 1, dans lequel l'au moins une séquence de nucléotides est sélectionnée parmi le groupe constitué en SEQ ID NO.: 51 et SEQ ID NO.: 110.

3. Le procédé selon une quelconque des revendications précédentes, dans lequel lesdites données comprennent au moins une équation de régression linéaire, de préférence une équation qui comprend au moins deux régressions linéaires.

4. Le procédé selon une quelconque des revendications 1 à 3, comprenant en outre l'étape de calculer une valeur h indicative de la santé d'un individu humain, où h est calculé à la base de l'âge biologique et de l'âge chronologique.

5. Le procédé selon la revendication 4, dans lequel h est calculé à la base de la différence entre l'âge biologique et l'âge chronologique.

6. Un procédé pour tester un agent actif comprenant le procédé d'une quelconque des revendication 1 à 3, comprenant en outre les étapes de :

   c) contacter les cellules de peau humaine avec un agent actif *in vitro ;*
   d) déterminer l'âge biologique des cellules de peau de l'étape c) selon le procédé d'une quelconque des revendications 1 à 3 ; et
e) comparer l’âge biologique déterminé dans les étapes a) à b) à l’âge biologique déterminé dans l’étape d).

**7.** Le procédé selon la revendication 6, dans lequel l’agent actif est un agent cosmétique et/ou un agent thérapeutique.

**8.** Le procédé selon la revendication 6 ou la revendication 7 pour identifier un agent actif qui prévient et/ou traite les signaux de vieillissement phénotypique de la peau humaine.

**9.** Utilisation d’une molécule d’acide nucléaire pour déterminer l’âge de peau humaine selon le procédé d’une quelconque des revendications 1 à 3, dans laquelle ladite molécule d’acide nucléaire comprend au moins une séquence de nucléotides sélectionnée parmi le groupe constitué en :

a) une séquence de nucléotides comprenant au moins une séquence sélectionnée parmi le groupe constitué en SEQ ID NO.: 1 à SEQ ID NO.: 201 comme montré dans les figures 1 à 34 ;
b) une séquence de nucléotides qui diffère de la séquence de nucléotides de a) par le remplacement d’au plus 10% de nucléotides, de préférence d’au plus 5 des nucléotides, avec l’exception dudit dinucléotide CpG ;
c) une séquence de nucléotides qui correspond au brin complémentaire à la séquence de nucléotides de a) ou de b).

**10.** Médium pouvant être lu par un ordinateur ayant stocké dessus des instructions pouvant être exécutées par un ordinateur pour causer un ordinateur à exécuter un procédé pour déterminer l’âge biologique de peau humaine comprenant :

a) entrer au moins deux valeurs d’un niveau de méthylation déterminé d’au moins deux dinucléotides CpG d’au moins une séquence de nucléotides sélectionnée parmi le groupe constitué en SEQ ID NO.: 1 à SEQ ID NO.: 201 comme montré dans les figures 1 à 34 de cellules de peau humaine ;
b) comparer chaque valeur de niveau de méthylation déterminé avec des données stockées qui représentent une corrélation entre le niveau de méthylation dudit dinucléotide CpG et de l’âge chronologique d’au moins un individu humain ; et
c) afficher l’âge biologique.

**11.** Médium pouvant être lu par un ordinateur selon la revendication 10, dans lequel lesdites données stockées comprennent au moins une équation de régression linéaire, de préférence une équation qui comprend au moins deux régressions linéaires.

| SEQ ID: | cgID | Cg Position | Chr No. | Chr. Start | Chr. End | Gene | Sequence |
|---|---|---|---|---|---|---|---|
| 1 | cg00055679 | 61/62 | 13 | 79170370 | 79170491 | | GCGGAGCTGCGACCTCGGGCAGCTGGAAGCCTGTGGGCTGCTGCTTCTCCAGGCAGGCGG[CG]GAGGGATGCAGGCAGGATGAGCGGCGGTCCCTTCGCCGCCACCCGGAGAACCGCGGCTGG |
| 2 | cg00266920 | 61/62 | 18 | 55021217 | 55021338 | NM_015879 | CCCTGGGTCTCCGGTTTCCTCTGCCCTTTCTCCAAAAACAATTCTATGGGGCTGCAAAGG[CG]TAGCGGGTCAGGCTGGCGGGGCGGCCGCTGTCCGCGGTGCTGATTCCCTGGTCCTCGCAG |
| 3 | cg00292135 | 61/62 | 7 | 156433008 | 156433129 | NR_026865<br>NM_030936 | CAACCGGGGCCCAGGCCCAGGTGGGCGGGCGGCTGAGGAGCGTGGCTGCGCCCACAAAGC[CG]CCGGGGGCTGCGGACTACAGCGAAGCCGGCGCGGGGCTCGGCCCTCACTACACGAGGCCT |
| 4 | cg00346208 | 61/62 | 1 | 20669845 | 20669966 | NM_001039500 | ACTTCGAGCAGCTGGCGGAGCGCGAGGGCGAGATCGAGCAGGGTGAGCGCCACGGAACTG[CG]CCCCTCCCGCGGACGGCCTCCGGAGGACAGCCCCGCTCCACAGCCGCTCCCCCTTCCCCA |
| 5 | cg00495860 | 61/62 | 21 | 38065464 | 38065585 | | AACGACCCCTTCTCCAGGTGCGCGAGCCGCTCCGGCGGCCGTGCACACTGCGCCCCCTTC[CG]CCCACCTGCCTGGCCTGCGTTTCTAACCACGCGGGCGGTCCCGAGACTTCGCGCAAAAGG |
| 6 | cg00875989 | 61/62 | 18 | 44773994 | 44774115 | | CAGCAGCACCGCCTGGCTCAGCCAGGTCCAGGAAGGCCTGGCGCAGCAGGGCCGGGCTTT[CG]CCTAGCGCGCAGCCTAGCGCCGAGGGCGGCTGAGGCGGGGGCTGCAGGTAGGTGGGCACC |

Fig. 1

| SEQ ID: | cgID | Cg Position | Chr No. | Chr. Start | Chr. End | Gene | Sequence |
|---|---|---|---|---|---|---|---|
| 7 | cg01141812 | 61/62 | 6 | 123317064 | 123317185 | NM_001010852 | GTGAATTACCGTCTACAACAGCTACTCT<br>CCAGGCTGATCTGGGGTCTTGCACACAA<br>AGGG[CG]AAGAGAGGGGCGTGGGGAG<br>GCTGGAAAGCATGGTTGCCCCGCCTGG<br>CCCGGCGACGCCCG |
| 8 | cg01150683 | 61/62 | 10 | 111216586 | 111216707 | | ACAGAGGAGCAGCAGAAGCCGCCGCCG<br>CAGACGCCCGGATGCGGAGGACCCAGA<br>GCGCTG[CG]CAGCTGAGGAGCACCTGG<br>GGAGCCACCGCCGGCGCCAACTTTTGC<br>CATCCCGGGAGCCAT |
| 9 | cg01321673<br>cg04485799 | 64/65<br>57/58 | 22 | 50438300 | 50438420 | NM_001001694 | CAGAGCCGCAGGTAGTAGTCGGGGCTC<br>CCGGAGGCCCTGGGCACTTGCACCAGA<br>AT[CG]CCTTG[CG]CCTCCGGTCCACCCA<br>GTAGCTGAGCTTCCCCGCTGCAGGAGG<br>CGGCAAGTCGGGG |
| 10 | cg01544903 | 61/62 | 16 | 56697169 | 56697290 | | TCGCCTTCTTCATTGGCGGAAGCTGGGA<br>GCCGAGAGAGTGAGCCGTGCGCAGATG<br>TGCGG[CG]GTGGAGGTGGCTTTTGTTTC<br>TCCTCCGCGCCCCCTCCCCCCCCGCCC<br>CGCACATTGCCTG |
| 11 | cg01555352 | 61/62 | 4 | 860778 | 860899 | NM_005255 | CACGCTCAGGGGAGGGGCCGGGCTTGC<br>CAGGAGCAGCGGGTCCTCGCTGAGCAG<br>ATCCTC[CG]GGGGCCCCTGGAGGCGG<br>CCTCAGGGGGCCCAAGGAGGCAGCTGA<br>GCAGGTCGGTGTTGC |
| 12 | cg02059315 | 61/62 | 16 | 3989372 | 3989493 | | GCCATGCAATGCCTTCTCAAGGCCCAGC<br>ACTTCGCCCGATCCCTGGGAGGGATGG<br>ACAGT[CG]TGCGCCCCATAGAGGTTTGG<br>GTCAACAGCCTGCATATAGGACCGTGGT<br>CCCATAAGATTT |

**Fig. 2**

| SEQ ID: | cgID | Cg Position | Chr No. | Chr. Start | Chr. End | Gene | Sequence |
|---|---|---|---|---|---|---|---|
| 13 | cg02119363<br>cg04894619<br>cg13473356 | 51/52<br>61/62<br>63/64 | 3 | 179754553 | 179754674 | NM_016559 | GCGCTGCGGGCTGCCGGGAACTGTTCT CCGCTCGGGGTGCTGAAAGCGGA[CG]C GGGAGAG[CG][CG]CAGAGAAGGCGAGG AGCCGGGTCGGCCAGGCTCTCCTGCAG GCGCGGGTCCTGCTCGC |
| 14 | cg02318784 | 61/62 | 15 | 27213114 | 27213235 | | CAGGAGCAAAGCAGCGCGCCAGGCTGG AGCTGTTCCGTCATTACCGCCACGTCTG CGCTT[CG]GTTGTGAGAACCGATTCGTT ACCCTAACCTCTTAGGACTAAGAGCCAG TCTTCTGGTGGC |
| 15 | cg02571816 | 61/62 | 19 | 38747318 | 38747439 | NM_033256 | GCGGGGTGGGGGCTGTTGGTGCGGG AGCGCCTGCGCCGCTGTCGCTGTAGAA GGTCGAG[CG]GTCACTTGTTCCTTCGTT CATTCATTCACTCATCCATCCATTTGTTT GTTCACTCATTCC |
| 16 | cg02587316<br>cg18630667 | 59/60<br>61/62 | 19 | 37096263 | 37096382 | NR_027239<br>NM_032825<br>NM_001145650<br>NM_001145649 | GTGCTGAGGAGGCCAAGGAGGGCACTG GGAGCTCCGTTCCGGGAGAGCTGTCTA AGGA[CG][CG]GAACCGGGTGGGGAGGC CAGGGAGGTAACCAAGAGTCCGAGCCG GGCCTGGAAATTCT |
| 17 | cg02599464 | 61/62 | 6 | 27107037 | 27107158 | NM_080593<br>NM_003495 | AAGAAAGCTGCCATCACAGGCAGCAGAC CTTTGTTCTCTGACCACTTGATAATGTCA GGA[CG]CGGCAAAGGAGGTAAGGGCCT GGGGAAAGGGGGTGCCAAGCGCCACCG CAAGGTGCTGCG |
| 18 | cg02650266 | 61/62 | 4 | 147558179 | 147558300 | | GCCCGAGAGGATCCAGGGAAAGCAGAA GGGGGTTAAGGACCATGGACAGAGCCC GTCGCG[CG]CTCGTTGCTGCCGCCTTCC CCAGCACTCTGGCGGCTCCTGAGGACA GCGGTCCCATCTTG |

Fig. 3

| SEQ ID: | cgID | Cg Position | Chr No. | Chr. Start | Chr. End | Gene | Sequence |
|---|---|---|---|---|---|---|---|
| 19 | cg02662828<br>cg17953764 | 62/63<br>59/60 | 4 | 48492787 | 48492907 | NM_175619 | TGGAGCAGGGCAGCCCCCAGAACGGCGCCCCGCGGCCCATGCGCTTCCCGCGCACCGT[CG]C[CG]TGTACTCGCCCCTGGCCTTGCGCCGTCTCACCGCCTTCCTGGAGGGGCCCGGGCCCG |
| 20 | cg02694427 | 61/62 | 2 | 176964452 | 176964573 | NM_021193 | CAATCGTCTGAGCCTGTCCTCTTATTCCCGGTTGTAACTAAATACTGTTGCGAGCGCAGC[CG]AAGCCCTTTGTTGGAGATGTGTGAGCGCAGTCTCTACAGAGCGGGCTATGTGGGCTCGCT |
| 21 | cg02699218<br>cg04044664 | 66/67<br>55/56 | 5 | 132150063 | 132150183 | NM_175873 | GAGCTCCCCTCTGCTGCTGAGGCGGCTCTCGGTGGAAGAGTCCGGTCTGGGCCT[CG]GCCTGGGCC[CG]GGCCGCTCCCCGCACCTGAGGCGCCTGTCGCGCGCCGGCCCGCGTCTGCTGAG |
| 22 | cg02760031 | 61/62 | 20 | 591091 | 591212 | NM_004609 | GGCCAATGGCGACGCTCCCACCGCCTCCGTCCCCCAGGTTGAAAGCGGCCCGGGAGCCGG[CG]GCCCACCCCGAAGGCCCCCGTTCCCGGCTGGCTAGCCCGGCAGATGCTGGAGGTCTGGGA |
| 23 | cg02898293 | 61/62 | 20 | 25061702 | 25061823 | NM_014588<br>NM_199425 | GGCGTAACTACAATGAATCCCCAAGGTTGCTTCTTTCTCCTGCACAGGGGACTGGGAGGA[CG]CTGCGCCGGCAGAGGAGCGCGGTACCCGGGACTCCCTTTTCAGCACCCCAAGGGGCGGAC |
| 24 | cg02970836 | 61/62 | 16 | 3237931 | 3238052 | | GGCCGCGAACCCACAAGTCAAGATTCGCAGGTGCGCGCGCTAAGGACTGAGCTATTGGGG[CG]ATGGAGAGAGTCTCGCTCTGCGTCAGGGACGGAAGGACGTGTCCCCCACCAGAAAAAGGA |

Fig. 4

| SEQ ID: | cgID | Cg Position | Chr No. | Chr. Start | Chr. End | Gene | Sequence |
|---|---|---|---|---|---|---|---|
| 25 | cg03096126<br>cg04212150 | 74/75<br>47/48 | 18 | 56531086 | 56531206 | NM_018181 | GCGGGGTGCGGCCCCTCGTGCCCACTCCTGCGGGCCGGGGTGAGCG[CG]TGAGGCTCAGGCCCCTGGGCCTGCC[CG]CAGTGGCCTGTCCCACCCTGGCCTTGGGGACAGCCGGCCTTCGCG |
| 26 | cg03111404 | 61/62 | 6 | 28175924 | 28176045 | | GACCCTGGGTGGCACCTTCTAAGGGTGATTCTGGGATTCTATGAATTAATGCATGGAAAG[CG]CTTGCGAGAGCGCTAGCCGGAGTCCGCGCTATTTAGGTGTTTGCCAATATTATTGTTGTT |
| 27 | cg03184290 | 61/62 | 4 | 111550606 | 111550727 | NM_153426<br>NM_153427 | ACCCAGGTCCTTCGAAGAAGGTTGGGACCGAGCTGCTGCCGCGTGTGAAGGTGTGTGTCG[CG]GCTGGGGGTGCCACAACGGGCCATGGAGCCCACCTCCCAGAGGGAGGAAGTCTGTGCACA |
| 28 | cg03200166 | 61/62 | 11 | 61335194 | 61335315 | NM_004200 | CTGCACCCCGGCGGGCGCACAGACGGTCCCCAGCGGCGGCCTGGGCCAGCGGCGAAGCAG[CG]GCAGACGGTTCTCCGGCCCCCGCCGCCCCCTCACCGCTCCCGGGGCAATCTGGCGCTCAG |
| 29 | cg03309770 | 61/62 | 16 | 10912418 | 10912539 | NM_001079512<br>NM_001079512 | GTCGGACGCCGGGCGGGCGGATGTAGGCAGCAGACGGTGGACGCTGAGGGTCGGGGCCTG[CG]CCCTGTGGGGCAGCCTCAGCGCAGCTTCTCGGGTGGGGCGGGGCGCTCGGGGCCTAAGGC |
| 30 | cg03354992 | 61/62 | 10 | 88149415 | 88149536 | | TCCTGTGCTCCCAGGTCTGGGCGTTAGGATTCTCTCAGTCCCGGAGCCACGCCGGCTGAC[CG]CAGGGCTCGGGGAGCGCGGCTGGGCCCCTTTTCCCGGGTCCGGGAAGCGCCGGGCCACGC |

**Fig. 5**

| SEQ ID: | cgID | Cg Position | Chr No. | Chr. Start | Chr. End | Gene | Sequence |
|---|---|---|---|---|---|---|---|
| 31 | cg03405983 | 61/62 | 8 | 143858488 | 143858609 | NM_177457<br>NM_177477<br>NM_177476<br>NM_023946<br>NM_177477 | CTCGGCGCGAGCGGGACTATCAGGGTGACCTCACCTGTGACCTTCACCACCGTGAGGTAC[CG]CCGCGCCTGGGAGTCACCCGCGGCCGCTCGCCGGTAGGTGCCTGCCTGAGTCTAATCGTA |
| 32 | cg03422911 | 61/62 | 1 | 237205235 | 237205356 | NM_001035 | CTCGGAAGGGGCAGGGGAATGAGCCCAGGGACCCCAGCGGGGCGCAGGTAGGAGGCTGTG[CG]CTCGCCGGGTGCGCTCCGGCCCCGATTCCCAGCGCAGCCAGTAAGTGGCGCTGGGCCTCG |
| 33 | cg03437157 | 61/62 | 6 | 100052573 | 100052694 | | GTCGCCAGGCCAGAGGTTAGAGGGACATGTTCATGGCTTAAATTTATTGCCCTGACTCTT[CG]GAGCGGCTTCCTCTCCTCCTCCCCACCCCCCGGCTTTTGCCATTCATGGGCCGCTGGATC |
| 34 | cg03555227 | 61/62 | 5 | 170289010 | 170289131 | NM_022897 | CGGCTGGCCGGCGCCGCCTCCTGGGAAGATGGCGCTGCACTTCCAGGTCAGTGTGCTCTG[CG]CCGCGGGCCCGCGCTCCGCCACGCTGGGAACCCGGCGGGACGCGTCTGGAGACCGAGGGC |
| 35 | cg03738025<br>cg17885226<br>cg23746497 | 52/53<br>89/90<br>26/27 | 6 | 105388643 | 105388762 | | GATTCCGTGCCCGGTGGCCGCGCGC[CG]CCCGGCTGTTCCGCGTCCCCTTGC[CG]GCTCACAATGTGCTGACCCTCCCGGCCCCGCCGCG[CG]CCTCACAGGTCTCTCGACCTCCGCGCTCCC |
| 36 | cg04069951<br>cg06699519 | 65/66<br>56/57 | 11 | 2398272 | 2398392 | NM_004356 | GCGCGGCCCTGCTGCCACCCCCTTGGGGGCTCGGAGCGCGACAGCAGCTTGGGGA[CG]CCTCCCG[CG]CCCAGCACGGTGCACCTGGGCCCTGAGGTCCTGGCCGAAACGCGCCAAGTTGGG |

Fig. 6

| SEQ ID: | cgID | Cg Position | Chr No. | Chr. Start | Chr. End | Gene | Sequence |
|---|---|---|---|---|---|---|---|
| 37 | cg04255416 | 61/62 | 5 | 57878437 | 57878558 | NM_138453 | TGCCCCACTCTGCCTAAGGAAACGTAGA AACTTTCCCGATTCGGATGGGGAGGGGT AGGG[CG]GAGAGTCCCAGAGACCAGCT CTGGCTTCGTTTGCATTGGAAATTAGCA CAAGGTAGTGTT |
| 38 | cg04332534 | 61/62 | 19 | 37096427 | 37096548 | NM_032825 NR_027239 NM_001145649 NM_001145650 | CGAGCCGGGCCTCGGGCGTGGTCCATG GGCGTGACCTTGAGGGTGACCCGGAGC GAGCTC[CG]CGAAGGGGGATTTTGCCC GTTCTGATCCCGCGGAAGCGCCATCGCT GGCGGCGGGTCCCC |
| 39 | cg04338129 | 61/62 | 11 | 65635331 | 65635452 | NM_016938 | CCCTAATGTAAAAGTCCCCCTGCGAGTT TCCAGCACGGATCTGAAAGGCATTGTAG GCAC[CG]GGGTAGACGGAGGTCGCCTG GATCTGGAACACGTCAGCGGGCACGCT CCGCTCCGAGGTG |
| 40 | cg04467618 | 61/62 | 6 | 134210886 | 134211007 | NM_198392 NM_003206 | GCTGGACACGCTCAGGCTGGCGTCCAG CTACATCGCCCACTTGAGGCAGATCCTG GCTAA[CG]ACAAATACGAGAACGGGTAC ATTCACCCGGTCAACCTGGTGAGTGCTC CCGGGGCTGCAG |
| 41 | cg04600618 | 61/62 | 6 | 43612920 | 43613041 | NM_152732 | CTCACGTCTCTTATGCTGGTTAAGCGCG ACTACCGCTATGATCGGGTTCTCTTCTG GGGC[CG]CATCCTTGGCCTCGTCGCCG ATTACTACATCGCGCAGGGCCTGAGTGA GGACCAGCTCGC |
| 42 | cg04670857 | 61/62 | 2 | 79740148 | 79740269 | NM_001164883 NM_004389 NM_004389 NM_001164883 | GAGTGAGGCGGAGACCCAGGCGGGCG GGCAGAGCAGGCGGCACCGCACCTCGG CCAGAGG[CG]GCTGCAGCAGCTGCTGC CCTTGTCCCTGCCGCCGCCTCTCCAGTC CCTTCTGTGATTACC |

**Fig. 7**

| SEQ ID: | cgID | Cg Position | Chr No. | Chr. Start | Chr. End | Gene | Sequence |
|---|---|---|---|---|---|---|---|
| 43 | cg04865110<br>cg08377398 | 63/64<br>58/59 | 4 | 6202496 | 6202616 | NM_001099433<br>NM_144720 | CCGGGGCAGGGCTGGGGTCCCGGCGC<br>TGAGCGCCCCCAGCCGCGCCGGGCAGC<br>CTGG[CG]CCT[CG]CTGAGTCGGAGCAAC<br>CCCCGGCAGGAGCTTTCAGGCTCTGGT<br>CAAGCCTCGGGACC |
| 44 | cg05336395 | 61/62 | 13 | 53421628 | 53421749 | NM_002590<br>NM_032949 | TCGTAGTCCACGGGCCCGGCCAGGGTG<br>AGGCGGCCTGATCGCGGGTCAAGCCGA<br>AAGAGG[CG]GCGCGCCTCCGGCGGGGT<br>GCGGGCGCCAAATGCGAACACCACGTC<br>GCCGTTAGGTCCCTC |
| 45 | cg05406635<br>cg16717713 | 65/66<br>56/57 | 14 | 100069602 | 100069722 | NM_001144995 | GGCCCGGGGGGCAGCAGCGGGGGTGG<br>GACGTGGTGGTGGTGGTCGGGGCTGCC<br>GC[CG]CTGCCCG[CG]CTGGACGTACTG<br>CTGCCGTCGCCCACGTCGCGGGCCCCC<br>GCGCCGGGCGCGCCA |
| 46 | cg05661282 | 61/62 | 19 | 58220310 | 58220431 | NM_001085384<br>NM_001085384 | AGTTGGCGTCCTCAGAGTGGCCGCTGC<br>CATCAGACTCTGCGGGTAGAGCTGGGC<br>CGGGAG[CG]ACGGGCGACATTGGTAGG<br>GACCCGGGGACAGCGGTCCCTATCCCA<br>GGCCTGACGTGGGTC |
| 47 | cg05700079 | 61/62 | 3 | 147126901 | 147127022 | NM_003412 | TTGGCTAAAAAAAAAGTTGCTACTCCTG<br>GCAGCCCTGGTTTGTCAAAAGGGGATGT<br>CAAG[CG]CTTTACAATACCTGGGATTGAT<br>GAGGCGGGCGGGCCAATGAGCTGCGC<br>GCGGCGCCTCGG |
| 48 | cg05979020 | 61/62 | 2 | 176981276 | 176981397 | NM_002148 | GGAGAGGCGGACAGGAGGGCGGCGGA<br>GAGCGCTGGGCCGGTTGTCTCCAGCGC<br>GCACTAT[CG]CGGGCGCGTAGTAGATGT<br>CGCTGTTGTCCGTGCTTACCCGGCCGG<br>CCGGCCAGGCTCTGG |

Fig. 8

| SEQ ID: | cgID | Cg Position | Chr No. | Chr. Start | Chr. End | Gene | Sequence |
|---|---|---|---|---|---|---|---|
| 49 | cg05991454 | 61/62 | 4 | 147558375 | 147558496 | | TGACCACCCGAGGGTTCCCCTTTTCCACTCTCCTTCCCACTCTGTTTTTGTCCCAGCGCG[CG]CCAGCGCCTCTCAGGCCTGCCGCCTGCTCTCGCACCTGCTCGCCTTCCCCAGGCGCCCAG |
| 50 | cg06226630 | 61/62 | 4 | 48493360 | 48493481 | NM_175619 | GAGGTGCGGCGCTTCCCTAAGCGTGGGCTACTTCCGTATTTCCGAGACAGCCAATGACCG[CG]ATAGGTGTCTTCCTTGACAGCACAGTCTCATGTCCCCGACATCCAGACTTACTCGTGGCG |
| 51 | cg06335867 | 61/62 | 7 | 8482265 | 8482386 | NM_152745 | CAGTCCGCTGACCAAAGCCGCAGTGTTCAGGCCCCGGGGTTTCCCAGCCGTAGTGGCCGC[CG]CCACAGCTGCGCGCTTTATTGTCTGCTTTCAGTCGCAGGTGACCTCGAGCGATCTCGACA |
| 52 | cg06383163 | 61/62 | 8 | 139509013 | 139509134 | NM_015912 | CGGCGGCGGCGGCGGGCGGACTGGGGAGTCCGCAGCACCTGCGAGCTGGTGTTGGGTCCC[CG]CGGCGAGGCTGCCTGGAGGAGGAGCGAGCGGATGCTGCCCTCGCCCGCCGCCGCCGGTGC |
| 53 | cg06558502 | 61/62 | 19 | 57610639 | 57610760 | | AAAAATGCTTTGAGATAAACCTGGAGCCACAGCTCTCTGCACAGTCAGATCGCAGGAACG[CG]GCCTAGCTGCTTCAGCCACGTTCTCGCGGCGCCACCTGGTGGCCATGGACCGCCATCGCC |
| 54 | cg06570224 | 61/62 | 3 | 157812415 | 157812536 | | GCCTCCGCCAGCAGGGCCTGGACGCACCGCCTCCTTTGACCTCGGGCTTCCCCCGCGCTC[CG]CTGCTTGGGGCAGACTGGCCCCGAGAGGGAGCCACCATCTCCCCTGCTCCAGGGTCTCCA |

**Fig. 9**

| SEQ ID: | cgID | Cg Position | Chr No. | Chr. Start | Chr. End | Gene | Sequence |
|---|---|---|---|---|---|---|---|
| 55 | cg06637517 | 61/62 | 3 | 52407284 | 52407405 | NM_015512 | GAAGCGAGAGCAAGTGAATGTCTCAGTC<br>CCCAGGTGGCGGTGGTGGCCGCGGGG<br>GCGCGA[CG]GCGGGGCGGGGGCGCTG<br>CTGCTGCGGGGGCAGGAAGCGGCAAGG<br>GCGGTGGCGGCCCGCT |
| 56 | cg06647751 | 61/62 | 10 | 88149264 | 88149385 | | ACACGCCGCATAGTAAAGCGGCAGAGA<br>ACTTGCATCAGGCGTCTCTGACCACCGA<br>GCCTG[CG]AAGACCTAGCTGATTACGAC<br>CAGGAAACAGGAGGGGACATGGCCCAA<br>GGTGGGCGGGGCC |
| 57 | cg06707978 | 61/62 | 19 | 58095095 | 58095216 | NM_001010879 | GGGAGGGGCACGAGGCTCCTTTGCTCC<br>CGCAGGAGCGTCGAATACGTATGGAATA<br>CGCGT[CG]GTGTGGATCTGGCCTCAGCT<br>TCCTGAGCCTGAATCAATGCTTCTAATCA<br>CGTGGGCGCTC |
| 58 | cg06763054 | 61/62 | 8 | 17270774 | 17270895 | NM_004686<br>NM_004686 | CAACCTTGGGCGTACGGATGTGCTCCAT<br>GGCTGGCCCACGTCTGCAGGGTCCCGG<br>GCGGG[CG]CGGCCTCACGCACCTGCGC<br>GCCTCTGCGGCGCGATGGGAGGGGCG<br>CGGGGAGGGGGCTTC |
| 59 | cg07028914 | 61/62 | 6 | 85474030 | 85474151 | NM_001080508 | CGGGCAAAAAACAGATTTGGCGTTTCCG<br>CTTTCTCGCTTGTGTTGGGATCCAGGAA<br>CCGG[CG]ACGCGCCGGCCAAGTCTCCT<br>TTCCTGGGTCTCTCTCGCGCGCTCTCTC<br>ACTGATGCACTC |
| 60 | cg07057579 | 61/62 | 15 | 95870052 | 95870173 | | AGCGTACCTGTCCCGTTCTGGCCTTGGA<br>GAACGCAGTGGCTCACAAGGGCACCGC<br>TAGCA[CG]CGAGGCGCGGCGAAGGGAG<br>CGGAGGGAAGCGGCGGGCTGTCGCGC<br>AGGTGCGGCCCCACC |

Fig. 10

| SEQ ID: | cgID | Cg Position | Chr No. | Chr. Start | Chr. End | Gene | Sequence |
|---|---|---|---|---|---|---|---|
| 61 | cg07169660 | 61/62 | 17 | 41467152 | 41467273 | NR_027413<br>NR_027412 | ATCCTCCAGAACCCTTCAGTAATCAAAAT<br>AAATAATAGTTCAATGCAATATATCTTAT<br>CT[CG]ATTATAAGATAATCAAGCCATTTA<br>TCTGGATTGCTGAGTCTTTTGGTGCCCC<br>CTTATTTT |
| 62 | cg07533148 | 61/62 | 1 | 248020752 | 248020873 | NM_015431 | CTTTCGCCCCAACCGGCAGCTGGCGGG<br>CCTGGTGGAGAGCGTGCGGCGGCTGGG<br>GTTGGG[CG]CGGGGCCCGGGGCGCGG<br>CGATGCGCGCGGCACGGCGAGGACCTG<br>AGCCGCTTCTGCGAGG |
| 63 | cg07544187 | 61/62 | 19 | 19651175 | 19651296 | NM_153221 | TCGGCGAGCGCGTGCACTTGAACCCCA<br>CGCGCGGCTTCTGGTGCCTCAACCGCG<br>AGCAAC[CG]CGTGGCCGCCGCTGCTCC<br>AACTACCACGTGCGCTTCCGCTGCCCAC<br>TAGGTGAGGGCGGG |
| 64 | cg07547336 | 61/62 | 7 | 1589742 | 1589863 | NM_001097620 | GCTGGGTGGCCCCTCACCTGCTTACAGA<br>AGCGCAGGAACCCGATGGAGTAGGTCA<br>TGCCC[CG]GAGGCAGCAGGTGCCGTAC<br>AAGCAGCTGGACCTGCGGGGGACGTCC<br>CTGAGCCGGTGCGG |
| 65 | cg07553761<br>cg15618978 | 54/55<br>67/68 | 3 | 160167924 | 160168044 | NM_173084 | ATGGCACCGGTGGTCTGGGGGAGAGGC<br>TGGGCCTGGCGCGGGACGAGGCGAAG[<br>CG]CCGGTGGCCGA[CG]GCTTCTGAGGA<br>ATTATCTTTTACTTGGCGCCACACGGGG<br>CGGGGCCTCCTGC |
| 66 | cg07570470 | 61/62 | 8 | 142318781 | 142318902 | | CACCCCATCCCTCTCTGCAGCCGCAGGA<br>GGTGGCCCCCGCCTGTCCCCACCTTCT<br>CTCCC[CG]CTCCTCTCGCCACTCTGTCG<br>CAGAGGGGTCCGGCTAGCCACGAGGCC<br>CCGGCCCCCGGCA |

**Fig. 11**

| SEQ ID: | cgID | Cg Position | Chr No. | Chr. Start | Chr. End | Gene | Sequence |
|---|---|---|---|---|---|---|---|
| 67 | cg07636194 | 61/62 | 6 | 27107222 | 27107343 | NM_080593<br>NM_003495 | GCGCATTTCTGGCCTCATCTATGAGGAG<br>ACCCGCGGAGTGTTGAAGGTGTTCCTG<br>GAGAA[CG]TGATCCGGGACGCCGTGAC<br>CTACACGGAGCACGCCAAGCGCAAGAC<br>GGTCACCGCCATGG |
| 68 | cg07703401 | 61/62 | 16 | 230281 | 230402 | NM_005331<br>NM_005331 | CGGGGGGCCTTGGATCCAGGGCGATTC<br>AGAGGGCCCCGGTCGGAGCTGTCGGAG<br>ATTGAG[CG]CGCGCGGTCCCGGGATCT<br>CCGACGAGGCCCTGGACCCCCGGGCG<br>GCGAAGCTGCGGCGCG |
| 69 | cg07806886 | 61/62 | 3 | 120626839 | 120626960 | NM_014980 | CACAGATTTCTTCTGCCCTGGAGACCAC<br>AGAACTTACCCTATCGAATCTAGGATTG<br>GCGC[CG]AAGCTACTCCCGCCCTTTGAC<br>GTCCCCGGGCACCCCGCCCCCTTCACC<br>TCCCAGCTTTGC |
| 70 | cg07878486 | 61/62 | 19 | 58951825 | 58951946 | NM_003433 | TCGTCGTCCTCGGACCATCACTTTGGCA<br>TTTCTCGATTTTGTCTGCTTCTGAAGGGA<br>CCG[CG]TTGTCAGGCGAGGGACGGAAT<br>CTTGGAGGCTCCCTGGGCCCATGGAAA<br>CAGGAGCGAGGA |
| 71 | cg08023265<br>cg17484671 | 50/51<br>70/71 | 1 | 31158089 | 31158208 | | GAGGAGAGCGAGGCTCCTCCGGGAAAG<br>CTCCTTCTGCTCCAGGTGACAG[CG]GAG<br>AGAGATGCCACCGCG[CG]GCGACCGGC<br>AGGGCCGCGTCCCCTCTGCGTCCTAGC<br>ACAGCGACGCCCC |
| 72 | cg08076830 | 61/62 | 18 | 44774863 | 44774984 | | ACGTCCTCCCAGGCGAAGACCAGCTCG<br>TCCTGGGGACTCTTGTCGGTGAGCTTGA<br>GATGA[CG]GCGCCACGAGTTGAAGTTGG<br>CTGCGTCTGGCTGAGTGTACTTGGCGTC<br>GGGCGTGCGGTG |

Fig. 12

| SEQ ID: | cgID | Cg Position | Chr No. | Chr. Start | Chr. End | Gene | Sequence |
|---|---|---|---|---|---|---|---|
| 73 | cg08158952 | 61/62 | 19 | 57182956 | 57183077 | NM_001005850 | CTCCCGGTATCACCTTCGAACGCGCTGG CCGGCCCCCTCCTAGCCTGGTGCAGTC GGCTC[CG]CTTCTACTCAGTCTCGGGTC CTCCTGGGCCAACGCAGACCCTGGTGC CTCCTTCCTTAGA |
| 74 | cg08445802 | 61/62 | 6 | 85482552 | 85482673 | | AAATTAACCCAGCCAAAGCGTTGGGGTT CAAGCGAGCGACTGGACCATAAATCAGC GTCG[CG]GTGGCGTCGGGAAGCTGAGC GCTCGGAGGTGCACTCAGCTGCCTCTG GGTTCGCCCAGTC |
| 75 | cg08622677 | 61/62 | 12 | 3601246 | 3601367 | NM_019854 | TTTGTGCAGAGCTGGGGTGGGTAATCCT GGGGCCAGGTCTGCCCCCTGCAGTGCC TTGAC[CG]TCTCCTGCCGCTGCCTCAGC TTTACCCTTCAAGCTCGAGTCGGTTCCC GAGCTCTCCGTC |
| 76 | cg08658787 | 61/62 | 12 | 113916586 | 113916707 | | ACCCCAGCTCCTTCCTCCCAAGGCGAGT ATGTTTACCCTGCTGGGCACCCTTCCGC TCCG[CG]GGGACCACTGCGGGATCGCG CTCTCTAAAACTCCTCTCAACTGCCCCG CAGTGCGCGGCG |
| 77 | cg08694014 cg15201635 | 37/38 83/84 | 16 | 68482555 | 68482674 | NM_018667 | CTGGGTCTCTCCTTCAAGACCACATCCC CCAGAGAC[CG]GCGGAGTTTCGCACAG CCTTCTGGGACTTATAGTTCTTTAAGGA[ CG]CGGAGTGGGCTCACGCCGCGGGTA GGCAAACAGAGC |
| 78 | cg08774009 | 61/62 | 8 | 49309034 | 49309155 | | TGCAGAAGGAAGAACCTTAGCTCACTGC AGCGCCACGCCGGGGACGGCCACCCCT GCCTT[CG]GTCCCATCAGGGCTCCTCCA CCAGCCGTTCCCCTGCCTTGAGTCCGG GGGCACATCCCCA |

Fig. 13

| SEQ ID: | cgID | Cg Position | Chr No. | Chr. Start | Chr. End | Gene | Sequence |
|---|---|---|---|---|---|---|---|
| 79 | cg09099868 | 61/62 | 10 | 22622733 | 22622854 | | TTCTTGCTCTGATTGTCAGGTCATCATGG AATGCAAACTTCACCGTGGACGTGATTT CAA[CG]TTGGTGCGGATTTGGAGATGTC CAACTCTGTCCTCAATTAGCCAGAACTC AAGTTGAGAT |
| 80 | cg09578568 | 61/62 | 4 | 113432710 | 113432831 | | GAGGCCTGCAAACCTACTTGGTGCACAC AGATGCAGCTCATCACAGCGCGGTCACA ATGT[CG]GGAGGGAAATGAATAAAATGG AAATACTACCTTGTTGCCTACTGCAGAA GCAGCGGCTTT |
| 81 | cg09767822 | 61/62 | 11 | 20177980 | 20178101 | NM_001029865 | TTCTGGCCCACGTCGCTGAGGTCCGGG TGCGGATTGAGCTTGGTGGGCAGGGTC TGCTCG[CG]ACAGCCCCCGCTAGACAG GAGTTCGCGCTCCTTGGAGTTCCGCCAT TTCATGCGTCGGTT |
| 82 | cg09907509 | 61/62 | 13 | 37248184 | 37248305 | NM_203451 NM_203451 | GCGCCCAGACTGCGCGCCGCGCCGCTG CGCCCAACATTCCCGAGGACGGCTTCG CGGGCG[CG]TATCGTCCAGACCGGAGC ACCGCCCCACCGCTAGCGCAGGAGACC TGCCGGGGAAGTCGC |
| 83 | cg10132208 | 61/62 | 19 | 58545482 | 58545603 | NM_182572 | GGGGTCGCCATGACCGAGTGGCCCAGG CCCGAGCGAAGCCCGCGCGCGGTGAGT CCGCCG[CG]GCCCATCCGTCCCTCCGC CCGCCAGAGCGTCCATCGGGACGCCCA CCCGGGAGGGTCTCG |
| 84 | cg10269548 | 61/62 | 4 | 53728863 | 53728984 | NM_023940 | ACGGGAGTTGAGGCTGAACAAGGCGTG GGGAGCGGTGGGAGCTGCAGCCCGACC GCTCTC[CG]TCCCCGCGCAGGGAGCCG CTGCCCCTTGGGAGTGGGCTTAGCCGTT GTCTACGCCACCCG |

Fig. 14

| SEQ ID: | cgID | Cg Position | Chr No. | Chr. Start | Chr. End | Gene | Sequence |
|---|---|---|---|---|---|---|---|
| 85 | cg10384245 | 61/62 | 18 | 909026 | 909147 | NM_001099733<br>NM_001117 | ACTCCAGGGCAGCAGCCAGACTGGCAT<br>ATGTAGGGCTCTCCGGTTACTTTCTCTG<br>TATGT[CG]CGGGTGAGAGGAACAGCGA<br>GGACAATTTAGCGCAAACACACGAAGGG<br>TCGGATCTCAAGG |
| 86 | cg10594245 | 61/62 | 2 | 109558906 | 109559027 | NM_022336 | TCATCCCACCCCTGTGGACCTGTGGCAG<br>TTGGCAAGTTCCCGGGGAGGCCAGGTG<br>AACCA[CG]ATGAACCACGGCGACGGCA<br>GGGGCCAGCGTCCCCGTCTCCCTCCCG<br>TTCTAGAAGACATA |
| 87 | cg10636297 | 61/62 | 21 | 45405664 | 45405785 | NM_001037553<br>NM_020132 | CCTCTGGCCTGTGGCTCACTGCATGCAG<br>CCCCTGGCGTGCAATACTAGTGCTCCAC<br>GGCG[CG]ATGTGCTTCTAGCCCTTGCAC<br>TGCACCTAGGCTCAGGGTTCAAACGGCC<br>AGCCCGAAAAG |
| 88 | cg10640845 | 61/62 | 19 | 1775151 | 1775272 | NM_001080488 | GCCTGCAAGCGCAAGGAACAGGAGCAG<br>CAGAAGGAGCGCGCCCTGCAGCCCAAG<br>AAGCAG[CG]CCTGGTGTTCACCGACCTG<br>CAGCGACGCACGCTGATCGCCATCTTCA<br>AGGAGAACAAGCG |
| 89 | cg10804656 | 61/62 | 10 | 22623400 | 22623521 | | ACCGGGAGGCCATTTTGCGCGTGCGCT<br>GCTCGCCTCGCGCCGCCCTCGGCTCTG<br>CGGACT[CG]GATCCCGCCAAATTTGAAC<br>GCGAGATTGTCAGGCCCTGAGGGGCTT<br>GAGGGGCGGGGGAA |
| 90 | cg10906284 | 61/62 | 12 | 63544370 | 63544491 | NM_000706 | TCTTGCGCGGCGTCCGGTGCAGAGCCA<br>GCAGTACGCTGCTGTTGCCCAGCACGG<br>CCACCG[CG]AAAGTCACCGCCAGCACG<br>GCGATCTCCAGTTTGGCCAGCTCCTCGT<br>TGCGCACGTCCCTC |

**Fig. 15**

| SEQ ID: | cgID | Cg Position | Chr No. | Chr. Start | Chr. End | Gene | Sequence |
|---|---|---|---|---|---|---|---|
| 91 | cg11084334 | 61/62 | 3 | 9594204 | 9594325 | NM_198560 | GGGTGCTCACGCTGTCGCCCACCCAGTAGGGCTGGATGAAGACCACCACGTTGATGATGG[CG]AAGCAGATGGTGAAGATGGCCCACAGCACGCCGATGGCCCGCGAGTTCCGCATGTAGTGC |
| 92 | cg11139646 | 61/62 | 1 | 40253755 | 40253876 | NM_001720 | CCGCGGATGCGCGCCCCTCCCCTGCAGCCAGCTTACTCCGAGGGTCCCCGCGCGGGCGGC[CG]CACTCACCCATGTTAACGAAGCTCATGACCAGGTCGGCGCGGCCCAGGCGCCGCTCCGCG |
| 93 | cg11220950 | 61/62 | 16 | 2042633 | 2042754 | NM_004209 | GCAGGACTCTGGTCCTTCCTGTGGTTCGTGGGCTTCTGCTTCCTCACCAATCAGTGGCAG[CG]CACGGCGCCAGGGCCGGCCACGACGCAGGCGGGGGACGCGGCGCGGGCCGCCATCGCCTT |
| 94 | cg11226913<br>cg14006181 | 70/71<br>51/52 | 14 | 29254892 | 29255012 | NR_026731 | TGTACTGTGATGGGGCTTTGTGGTGAGAGGAACCTCTGAGAAGCCTCGTG[CG]GCTTGAGTTTAGAGTCA[CG]CCCTGCCCAGCGACATTCTCCCGCGCACGGGAGAACCTGCACTTCCGGT |
| 95 | cg11254700 | 61/62 | 19 | 53561326 | 53561447 |  | AGGACAGGGTAGGGTCTGCAGGAGCCTAGGACCAGGCGGAGGACGCGGTCTGCGGCGCTG[CG]CTCCAGGGCCGACGACGGAGAGGCTGGGAGGCGCTCAGGACGGGAATCCACCTCGCAGGT |
| 96 | cg11417653 | 61/62 | 22 | 24181152 | 24181273 | NM_001135751<br>NM_198440<br>NM_001002862 | GCCGCTAGTCCCTGCCACGCCATTGAACCTTCTCAAGCACGCGTGGCCCAGCCAGCAACG[CG]CTCTTTAACCCGCCTCCCAGCCCCGCCTTCGGCCAATCCGCATCCGAGGCGCTGCGCGCC |

Fig. 16

| SEQ ID: | cgID | Cg Position | Chr No. | Chr. Start | Chr. End | Gene | Sequence |
|---|---|---|---|---|---|---|---|
| 97 | cg11563680<br>cg25351606 | 45/46<br>75/76 | 6 | 100917353 | 100917472 | | CCCGAGACTCAGTGACAGTCGCAGCTTAACCCCGTTGGGGGCGC[CG]CCCCGCTGAGGTGGTTGCGTCTCCAAGT[CG]TGAGCCTCCAATAGCTGCTCCCGCTTTCGCGTCGCAACCCCAGG |
| 98 | cg11705975 | 61/62 | 10 | 120354188 | 120354309 | NM_004248 | AGCACCGCGGACAGCGCCCAGATGGCCAGCACAGCGTAGGCGCTGAGGCGCAGCGAGATG[CG]CCGCCTCAGCGGGTGCACCAGCACGACGTAGCGGTCCACTGCGATGGTGGTGAGCGTGAA |
| 99 | cg11763509 | 61/62 | 1 | 39957945 | 39958066 | NM_181809 | CGCGGAGCAGCGCCTGGGCCGCGCCGACCTGGTCATGAGCTTCGTCAACATGGGTGAGTG[CG]GCCGCCCGCGCGGGGACCCTCGGAGTAAGCTGGCTGCAGGGGAGGGGCGCGCATCCGCGG |
| 100 | cg12052661 | 61/62 | 9 | 140772485 | 140772606 | NM_000718 | CCCGGGGGGCTGCAGCCCGGCCAGCGGGTCCTCTACAAGCAATCGATCGCGCAGCGCGCG[CG]GACCATGGCGCTGTACAACCCCATCCCGGTCAAGCAGAACTGCTTCACCGTCAACCGCTC |
| 101 | cg12325536 | 61/62 | 5 | 87968598 | 87968719 | NR_024383<br>NR_015436<br>NR_024384 | CATCCGAGGCACACAATCAATTAGCTGAAGCAAAGCCCCGGACTGGCCCTCGGAGCGGCC[CG]CAGCGGGACTCTGCGGGCACCGGCTAGCGGGCGAGGTGAGGAGTGCGGGCGCGGGGCCGC |
| 102 | cg12377139<br>cg18247055 | 56/57<br>64/65 | 10 | 22634163 | 22634282 | NM_012443<br>NM_172242 | GCAATCTGGGGTCGCTTTGTAAGGAGCTCCGACCTCCGGGCAGTGCAGGGATACT[CG]ACGGCG[CG]CGATGTCCCGGCGGCTCCGCGAGCCTGGCGCGCTGAGAGCCCCAGCAGTCCGAGT |

**Fig. 17**

| SEQ ID: | cgID | Cg Position | Chr No. | Chr. Start | Chr. End | Gene | Sequence |
|---|---|---|---|---|---|---|---|
| 103 | cg12597389 | 61/62 | 7 | 8482175 | 8482296 | NM_152745 | CATTGACTTTAAAAGGCCATTTTCCTTCGTCTTCTACAAGAAGCAAGAAACTTTTTTCGA[CG]TAGGCTTCATACCCTCCCTTCGGAAACTCAGTCCGCTGACCAAAGCCGCAGTGTTCAGGC |
| 104 | cg13066983 | 61/62 | 3 | 68057246 | 68057367 | NM_213609 | GCCCTGGTCTGACAGGGAAGGCGAGGGGGCGCTGGTGCCCCTGCAGGTGGGCAGGGAAAC[CG]CCCTCTTCAGGACGCGCTTGTATTTCTGCATCCTGTGTCCTGAGCCTGGCGCGGGAACCG |
| 105 | cg13246235 | 61/62 | 6 | 12749918 | 12750039 | NM_030948 | CATCCTCGGAGGATGATATAGACCGGCGGCCCATCCGGAGAGTGCGCTCCAAGAGCGACA[CG]CCGTACCTCGCAGAGGCCAGGATCTCCTTTAACCTGGGGGCAGGTAAGAACGCCCCTGGC |
| 106 | cg13327545 | 61/62 | 10 | 22623488 | 22623609 | | TGTCAGGCCCTGAGGGGCTTGAGGGGCGGGGGAACGACGCCGCTCTCCAAAGTTGGACCC[CG]TGGCGAGCGGCGGCGACAGCCGGGTGCTCGCTGCCTCCCGAGGTGCTCCCTTTTCCCGCC |
| 107 | cg13434842 | 61/62 | 8 | 11567836 | 11567957 | NM_002052 | CGAGATAGCGCGGCGACATGGCCACACAATGGAGCCCGCAGGCGGGAGTGCGGGGCGGGG[CG]CGGCGCCCTGGCCTTGCGCGCTTACGGGGTCCTCTCCAGGGCCCTCTGGGGCCTCTGACT |
| 108 | cg13654588 | 61/62 | 10 | 120354021 | 120354142 | NM_004248 | CGTAAGACAGGAGGATGACCAGCAGAGGGAGCAGGTAGGTGACCAGCAGCAGCCCCCAGG[CG]TAGAGCTGGCGCTGGCGCTCCTGGGAGCCCCAGAACTCCTCGCAGAGGCGCACGTCGTGC |

Fig. 18

| SEQ ID: | cgID | Cg Position | Chr No. | Chr. Start | Chr. End | Gene | Sequence |
|---|---|---|---|---|---|---|---|
| 109 | cg13836638 | 61/62 | 22 | 42679744 | 42679865 | | GCGCGGCGCGGCGGAGCACCCGGCGG<br>GCACGCCTCGCACGCCAGAGCAAACTTT<br>CACGAC[CG]CGGGAGAGGCACCGCGCA<br>CACCGTTACCTTTGCAGCCGCATCCTTC<br>CCTGGCCCTTGCCC |
| 110 | cg13848598 | 61/62 | 10 | 115804518 | 115804639 | NM_000684 | CTACAACGACCCCAAGTGCTGCGACTTC<br>GTCACCAACCGGGCCTACGCCATCGCC<br>TCGTC[CG]TAGTCTCCTTCTACGTGCCC<br>CTGTGCATCATGGCCTTCGTGTACCTGC<br>GGGTGTTCCGCG |
| 111 | cg14750948<br>cg19029181 | 31/32<br>90/91 | 3 | 147130447 | 147130567 | NM_003412 | TTCGCTGCGCAAACACATGAAGGTAATC<br>GC[CG]CACTCTCGTCGCCCCCTTTGAGG<br>CAGGAGCTCTCTTGGCTCTCGGCTTGGG<br>GTCGGG[CG]GCGAGTGGCAGACAGGCG<br>GTGGCGGGAGC |
| 112 | cg14871932 | 61/62 | 7 | 44185263 | 44185384 | NM_000162<br>NM_033508<br>NM_033507 | GGTGGTCGAGGGTCGCAGCCCCAGCGT<br>GCTCAGGATGTTGTAGATCTGCTTGCGG<br>TCGCC[CG]TGTCGCTGCGGGGCGGGAG<br>GAGGTAGGGCGGTCGCTGAGTGTCGCT<br>CCGACAGTCCATCC |
| 113 | cg14912644 | 61/62 | 2 | 157176541 | 157176662 | | GAGCACACGTGTGGAGCATGGTTGTGG<br>CATGGGCGTCTGGGCCCCTCTCCTAGA<br>CCTCCA[CG]AGGTGCGGACGCGCCTCT<br>GCCCCGCCCTTTTGTGCGCCCTCACCAC<br>TAGAAAAAGAGTTT |
| 114 | cg15084543 | 61/62 | 1 | 79472348 | 79472469 | NM_022159<br>NM_022159 | GCCGCAGTGGTGGCGGTGGCGGAGAG<br>CGCGGCGGAGTGAGTGCGGCTGTGGAC<br>CCGGGAC[CG]GGCGCCGCTGGGCGGG<br>CGCGGCAGGGTCCCGGATCCGTCCTCC<br>GTGACGCGCCGGCTTCC |

**Fig. 19**

| SEQ ID: | cgID | Cg Position | Chr No. | Chr. Start | Chr. End | Gene | Sequence |
|---|---|---|---|---|---|---|---|
| 115 | cg15174623 | 61/62 | 7 | 35494285 | 35494406 | | TACTCCTCGGCCACGCGGCGGCTGGCA ACTGACGCGCGCGATGTTGCGCCGGCC AATCAG[CG]TATGGCTCAGTCCCGCGGG CCCCGCTTCCAGGTGGAGCTGCTCCTG CTCTCGGGGTAGCC |
| 116 | cg15243034 | 61/62 | 11 | 77907596 | 77907717 | NM_020798 | CCTGCGTGCAGCTGGGTCTGCAGCTGC TGCCCGAGGGGCCTGCGGCCGACGAG GTGTTCG[CG]CTGCTGCGGCGCGAGGT GCTGCGCACCGTGTGCGAGCGCCCGGG CCCCGCGGCCTGCGCG |
| 117 | cg15480367 | 61/62 | 14 | 93389425 | 93389546 | NM_001275 NM_001275 | AAGGGGGCGGCACCGCTGACGTCATTT CCGGGGTCGGGGTATATAAGCGGGGCG CGAGGG[CG]CTGCTGCTGCCACCGCTC CTGCCACTGCAGTGCTCGAGCCCCGTG CAGGGGAGCTTGCGG |
| 118 | cg15488596 | 61/62 | 1 | 185253435 | 185253556 | NM_017673 NM_001105518 | TGGCTTTCTTTCTGTTATTAGAATTCTTTA TGAGGAATTTGGCCTCAGGCCAAAATCT TA[CG]TGATGTTTGAGCCCACTATAGACA ATTACAAGGCTTTATCTGACAAACTCAAT GGCGGAA |
| 119 | cg15611336 | 61/62 | 15 | 75248436 | 75248557 | NM_017793 | CGGGGACGAGGGACCAGGATGGGGATT CGGGGGCTGGTAGCCGGGCTGTCGCG GATCCAG[CG]CGTCCTTGGAAAGTAGGA TGGCGAGGCCGGGCACGTTCTTAAGTA CGCTGAGACTAGCGG |
| 120 | cg15846744 | 61/62 | 7 | 94284838 | 94284959 | NM_001099400 NM_001099401 NM_001040152 NM_003919 NM_015068 | AAAAAAGATGTGTTTGTGTAGCGGGGAC GCAGCTTGACACCCATTTCCTTTGGCTC GCAT[CG]CGGTCGCAGAGCCCAATCGC GGGGAAACGGGCCCCCCAGAAAAACAG GCAACGACCCCCA |

Fig. 20

| SEQ ID: | cgID | Cg Position | Chr No. | Chr. Start | Chr. End | Gene | Sequence |
|---|---|---|---|---|---|---|---|
| 121 | cg15895690 | 61/62 | 14 | 60982575 | 60982696 | | GCCGGGGTTAGCCTTCGAGGCGGGGAGAGGGAGCGGGGTCCTTCAAGGACCGCATGTTCG[CG]ATAGCACATTTGAGCCCTGGCAGGCCACCTGGGCCGCCTTCTTTCGGAAACCTTGCGGGG |
| 122 | cg16061498 | 61/62 | 18 | 55095826 | 55095947 | | CTCGGGAGGCGCTTTGCCTTTGAGGAAGATGGAGAGGAGTCGGGAGAAGCGCCTAGAAAC[CG]CATTGATTTAGACATCAATCCTGGCCGGCTCCCTCCGCCTGCCGAGCTGCGGGGCCGCGC |
| 123 | cg16181396 | 61/62 | 3 | 147126146 | 147126267 | NM_003412 | GAATGAAAGGGGCCCAAGTAGGGAACAGGAGTGAGGAGAGACAGGGTTAGCGGGGGCAGT[CG]AAGGAGACAACGGAAAGGCAGAAAACAGAAAAATAACGCAAGAGAGAGAAAAAGTAAAGG |
| 124 | cg16582779 | 61/62 | 14 | 29237142 | 29237263 | NM_005249 | CGAGAACAAGCAGGGCTGGCAGAACTCCATCCGCCACAATCTGTCCCTCAACAAGTGCTT[CG]TGAAGGTGCCGCGCCACTACGACGACCCGGGCAAGGGCAACTACTGGATGCTGGACCCGT |
| 125 | cg16616467 | 61/62 | 16 | 89642854 | 89642975 | NM_014427<br>NM_153636 | GCCGACGGGGGGCGGGAGGCTGGGCGGGAGCAGGCCGGCGAGGGAGCGCGCGCTGGGGTG[CG]GACCTCCAGGCGTCCGGGGCTCCCGCTCCAACGTGGGTCTCAAGCGAACCCGCTGTGATC |
| 126 | cg16626067 | 61/62 | 16 | 66613206 | 66613327 | NM_144673 | CGCGAGGGCAGCTCCGCGCGGAGGTGGGACCGGCGGGTTGGGAACGGAGTTGGGGCCCGT[CG]GGGGCCGGCGCGTGGAGGGTGGGAGGATCCGGCCGCTGCCGGGCGGATGGGAGCTGCGCG |

Fig. 21

| SEQ ID: | cgID | Cg Position | Chr No. | Chr. Start | Chr. End | Gene | Sequence |
|---|---|---|---|---|---|---|---|
| 127 | cg16705627 | 61/62 | 13 | 112721890 | 112722011 | NM_005986<br>NM_005986 | TTCCTCTCCGTCTCCCTCCCACCCCGGC<br>CGTCTATGCTCCAGGCCCTCTCCTCGCG<br>GTGC[CG]GTGAACCCGCCAGCCGCCCC<br>GATGTACAGCATGATGATGGAGACCGAC<br>CTGCACTCGCCC |
| 128 | cg16717122 | 61/62 | 15 | 51973860 | 51973981 | NM_001165257<br>NM_013243<br>NM_013243<br>NM_001165257 | TCTTCCTGTTTTTACTCCTCCTTTTCATTC<br>ATAACAAAAGCTACAGCTCCAGGAGCCC<br>AG[CG]CCGGGCTGTGACCCAAGCCGAG<br>CGTGGAAGAATGGGGTTCCTCGGGACC<br>GGCACTTGGAT |
| 129 | cg16867657<br>cg21572722<br>cg24724428 | 50/51<br>67/68<br>61/62 | 6 | 11044828 | 11044949 | NM_017770 | CCTGCCGGCCGGGCGGCGATTTGCAGG<br>TCCAGCCGGCGCCGGTTTCGCG[CG]GC<br>GGCTCAA[CG]TCCA[CG]GAGCCCCAGGA<br>ATACCCACCCGCTGCCCAGATCGGCAG<br>CCGCTGCTGCGGGGAG |
| 130 | cg16919569 | 61/62 | 4 | 174452775 | 174452896 | NR_003679<br>NM_021973 | TAGCCCAAGGGAGACCAAAGACTCCACC<br>TTGAGCATCGCCCTTTGGAGGCGGGCA<br>GAGTC[CG]GCCGCAGGCCACAAAGCGA<br>TCCCCACCCGAAGGACTCCACAAGGACA<br>GTCCTTTCCTTGC |
| 131 | cg16997364 | 61/62 | 19 | 39755515 | 39755636 | | ATCCACGTCCAGGAATCCCAGACTGTGC<br>AGAGGTTAGGGGCCCTGGCGAGGGGGC<br>CCAGT[CG]TATGCGATAAGCGCCGCTTG<br>TCCCGCAGGCGGAAGAGGCGCTGAGTT<br>GGAGGTCGCGCAA |
| 132 | cg17007640 | 61/62 | 14 | 29243444 | 29243565 | NR_026732<br>NR_026731 | CTGAGAGCACGCGCCAGACACAGGTTG<br>GCTCGGCCCGAAGCGAATTTGGGACTCT<br>CGTCG[CG]GTGTCCTATTAATTAGCTTTT<br>CCCGGCGCCTCAGGGACTCCGGGGTGA<br>AGATGGAGAGGT |

Fig. 22

| SEQ ID: | cgID | Cg Position | Chr No. | Chr. Start | Chr. End | Gene | Sequence |
|---|---|---|---|---|---|---|---|
| 133 | cg17193155 | 61/62 | 6 | 27203283 | 27203404 | | AGCGAGTTTCCGTAGTGTAGTGGTTATC ACGTTTGCCTAACACGCGAAAGGTCCCC GGTT[CG]AAACCGGGCAGAAACAGAGC GTAGTTTCGTTTTTTTGGTTGTTTTTTTTT TTTTTTTTTT |
| 134 | cg17241310 | 61/62 | 1 | 91182796 | 91182917 | NM_020063 | CGAACCAGCGAAGAAAGCTATCGATCGT AAAACAAAATAAACACCAAACAATGTTGC CGC[CG]CTTAAAAAAAAAAAAGTGGGGC GGGGAGAGAAGGAAGGGTAATTGGGAA GTGGCAATCTG |
| 135 | cg17280346 | 61/62 | 3 | 147126643 | 147126764 | NM_003412 | CATTCTGGAAGTGCCTTTGCTGTGTTTAC TAGCCCCATCCCCGCCTGCTGCACCCG GAGA[CG]TCTGAAGTCTCTAATCGCTCA GCGAAAAGTTGGTTTCGGGAAGGGCAG CGCCGGGGTGCG |
| 136 | cg17485681 | 61/62 | 10 | 73565565 | 73565686 | NM_022124 | GATCCCGCTGCGCTCCAACGTGTACGA GGTCTACGCCACGGACAAGGATGAGGG CCTCAA[CG]GGGCGGTGCGCTACAGCTT CCTGAAGACTGCGGGCAACCGGGACTG GGAGTTCTTCATCA |
| 137 | cg17508941 | 61/62 | 7 | 19183220 | 19183341 | | TGGTACTAGCACGTCACCTAGAAGGAAG AATCCTGGAATGGCACGGGTCCAAACTA GAGG[CG]GCCTCTCAGCATGGACCCGC TTCAACCTCATCTGCATGGCAGGCGTTT TGCAAGGCGTCA |
| 138 | cg17536595 | 61/62 | 6 | 43612848 | 43612969 | NM_152732 | AGCCTCCTGCTGTCTCTGGAGCTGGCGT CCGGCAGTGGGCAGGGCCTCAGCCCGG ACCGT[CG]GGCCTCGCTGCTCACGTCTC TTATGCTGGTTAAGCGCGACTACCGCTA TGATCGGGTTCT |

Fig. 23

| SEQ ID: | cgID | Cg Position | Chr No. | Chr. Start | Chr. End | Gene | Sequence |
|---|---|---|---|---|---|---|---|
| 139 | cg17592231 | 61/62 | 11 | 43604698 | 43604819 | | TTTGGGAGAGAGGTGGCCCAAGAGAAG TGAACTTGCACAGAAGAGGTTGAGCCAC CGCAG[CG]CAGGCGGCTCAGGGTTATT GCGACCCTAGCCCCCTCACTTCTCCCTC CGAGGCTAAGTGT |
| 140 | cg17980283 | 61/62 | 2 | 45156582 | 45156703 | | ACAAACAGGGCGGGGGCGCGGAGGTTT GGGGTGGGGGCCGCAGGGGGCTGGAA GGAAGTG[CG]CAGTGTGGCGAGAGCGC GAACAAAGCCCTCTCCGGAGCCCCGTC ACCCCTCGGTGACCCC |
| 141 | cg18074184 | 61/62 | 4 | 48485030 | 48485151 | NM_152679 | AGTCAAGCACCCCTCTTAGGGCTGCAGG CTATAGGAACAGTTTCTGCCATGAGCGT GCAT[CG]ATGAGGTTATCCACCCGCCCC CTCCACTTTGTGTGTCTGGGTCTCCAGA TTTCATTATAA |
| 142 | cg18104919 | 61/62 | 5 | 7851336 | 7851457 | NM_001089584 | TCCCTCCCGGCCCCTCGACTACACCTGG GGTCTCTCCTTCCTTCCTCCTCGGGAGG AGCC[CG]ACTGGGAGACGCCGCGGGGA CTACCCATTTCCAGCCCGCTCACCTTTC CCATGCAGCAGC |
| 143 | cg18292202 | 61/62 | 6 | 26746887 | 26747008 | | GCCACGCCCCCATCCTGTGCCTATAAAA ACCCCCGAGACCCTAGCGGGCACAGAC ACAAG[CG]GTTGGACATGAAGAGGAACA CACCGGCGGGAGAACACAGAACACCCA CCCTCTCTTTAGT |
| 144 | cg18396984 | 61/62 | 14 | 37049833 | 37049954 | NM_014360 | AAATCGCTATTTAAAAATATACAAATAAG GCCCAAGCATAAAATCTAACTCTGGGGC TGG[CG]GTGGAGGGAAGGTGAGGGAGG GGGCTCTGGCACACGTCCCTCGTGTGT GCTTGCGCGACG |

Fig. 24

| SEQ ID: | cgID | Cg Position | Chr No. | Chr. Start | Chr. End | Gene | Sequence |
|---|---|---|---|---|---|---|---|
| 145 | cg18582824 | 61/62 | 2 | 124782526 | 124782647 | NM_130773 | TGGGAAATTCCCTCTTCCTAATGCGTCTGTCTTGCAACTCCTATTAGCATCCTTCGACAG[CG]GCCTTTGAAAATCTCCTTTAAATTTCAATACCTCTACTCGTTTCCCAAATGGGGGGAAGG |
| 146 | cg18898125 | 61/62 | 8 | 24770321 | 24770442 | NM_005382 | CCTCCCACTCGCCGACCTGGGCGCTGTCCTTGGTGCTGCCGGCGCCATAGCGAGCACAAG[CG]CCTTACCTTCTGCCCCTGCGGAAGCTGCGGACGTCCCCCAAACGTTAAAAACGCCATATC |
| 147 | cg18956706 | 61/62 | 6 | 43214790 | 43214911 | NM_032538 | GCTGCAGAGGCCAGTGTGGCGGAGGAGGAAGAGGGGTGGGGGAGGGAGCTTTTTCTCTGA[CG]GACCTTGGAGAAGGGCAGTGGACGAGCATGTGTCCCTCCGCTTCCGCAGAGGACGGAAAG |
| 148 | cg19021738 | 61/62 | 19 | 9517544 | 9517665 | | CGGCGCAGGTGGGAGTCTGGGAAGAAGTGCGGTGTCGGCAGGAAGTCCCAGCGCGACGTC[CG]GGAAAGCCACAGGAGGAGGCGACCTGCAGGAGGCTACCCTTTGACCCCAACCCTACTCTC |
| 149 | cg19839825 | 61/62 | 19 | 7615951 | 7616072 | NM_001166114<br>NM_001166111<br>NM_006702<br>NM_001166113<br>NM_001166112 | TCCCGAGGGCACCTTGGGTCACATCAAACGCCGGTACCCGCAGGTGCGGCCTGTTGTGGG[CG]GGGCAGAGAGGCGGAGGCGGGACTCCGGGGGGGTGGGGGCCGGGCCTAGTGTGTGGGCGG |
| 150 | cg19925849 | 61/62 | 19 | 54445317 | 54445438 | NM_031896 | TTCACCAAGCGCTACGCGGAGGAGGAGATGTACCGTCCACACCCGGCCTTCTACCGCCCG[CG]TCTCAGCGACTGCTCCGACTACTCGGGCCAGTTCCTGCAGCCCGAGGCGTGGCGCCGCGG |

**Fig. 25**

| SEQ ID: | cgID | Cg Position | Chr No. | Chr. Start | Chr. End | Gene | Sequence |
|---|---|---|---|---|---|---|---|
| 151 | cg20146541<br>cg20810478<br>cg23054189 | 88/89<br>23/24<br>83/84 | 1 | 248020610 | 248020729 | NM_015431 | ATTTCCTGCAGGAGCCGGTCAG[CG]TGGACTGCGGCCACAGCTTCTGCCTCAGGTGCATCTCCGAGTTCTGCGAGAAGTCGGA[CG]GCG[CG]CAGGGCGGCGTCTACGCCTGTCCGCAGTGCC |
| 152 | cg20192747 | 61/62 | 18 | 44774786 | 44774907 | | TGAGCGGGTGGCAGGCGGGGTGCGCGCCCGGCTGGGGCAGTGCGCGCTTGCGGCTGCCGC[CG]TTGAACATGGCCTTGACGTCCTCCCAGGCGAAGACCAGCTCGTCCTGGGGACTCTTGTCG |
| 153 | cg20219381 | 61/62 | 8 | 101118023 | 101118144 | NM_015668 | GGGATACCGCTAGGAGGGCAGGAGGTAAAGTCCAGAGGCTGGCGCGTGGTGCGGCCCCGA[CG]CCGCGGGCTGATCCTCTGTCCCTGTGGGGCCGCCACCTACCCGCGGTGAGCCTCTTCTCG |
| 154 | cg20361154 | 61/62 | 22 | 22862824 | 22862945 | NM_080764 | CACTCCCCCACAGAAAGCGGCTGTATTGCAAAGGCCGCTCCGGCAGCGCACGGCCAAGCG[CG]GTGTCGCGACCCGCAGGGCTGCGGCTCGCCATGAAATCCCTCAAGTCTCCCCTCAGGGAA |
| 155 | cg20442599 | 61/62 | 6 | 108479440 | 108479561 | | AAACTGTAAGCCAGAAAACCTGGCCAGGCCTCCGCGACGGCGAGAAACGGTCTTGAAGGT[CG]GTCGGTCGCCGTAATGCGGTCCGGATCGTGCACGTGCTGTTTCGCCACTGCCCTCCGGCT |
| 156 | cg20449685 | 61/62 | 19 | 58545668 | 58545789 | NM_182572 | CCGGGCCCGGGAGGGGGCGGGCCGGGCCGGGGTTCCAGAGCCCATCGGGAGTCCTGAGGT[CG]CCGTAGGGGAGGGAAGGGGAAGGCTAGAGGCCACGCGGGGCGGGGACTGGGGGGGCTGGG |

Fig. 26

| SEQ ID: | cgID | Cg Position | Chr No. | Chr. Start | Chr. End | Gene | Sequence |
|---|---|---|---|---|---|---|---|
| 157 | cg20596329 | 61/62 | 11 | 67895782 | 67895903 | | GGAAGCGAAGGCGGACCCTCCTCCCCC<br>AGCGCTACTGCCCGGGCCGAATTCCAA<br>GGCCAG[CG]GCTGCCCGCTGAGAAGGA<br>ATCCGGACCAACGGGATTCTGGCTTCCC<br>TCTGGCCCCTCCTG |
| 158 | cg20611680 | 61/62 | 1 | 16554111 | 16554232 | | CGTTTAAATTTCAAATGAGGAAAACGAAC<br>ACCTTCGTTTTTCAAACGCGGAGACAGA<br>CGC[CG]GGTGGGCCGCGTCGTATAGCG<br>ACTGATCTTGCTCCTTTCTTGGGCACCT<br>CCAATGTGCCA |
| 159 | cg20755170 | 61/62 | 5 | 145719964 | 145720085 | NM_002700 | AGAGACAGAAACAGAAACGAATGAAGTA<br>TTCGGCTGTCCACTGATTGCGGCAGGG<br>CGCAG[CG]TCGGGAGCCGGGAGAGCCT<br>AGTGCTCATCCCTCCCGGGTTCGGGGG<br>ATGGTTATCGGGAA |
| 160 | cg20897936 | 61/62 | 6 | 28554681 | 28554802 | NM_052923<br>NM_052923 | GGGCAATTCCATTGAAAGTGAAGCCATC<br>CTCTGAAAAGACCAGAAATAGTGAGCAC<br>AGAG[CG]ACTCACTAAAGCTTCAGGGTC<br>ACAAATTGAGCGGCAAGATACTTGCGAG<br>ACAGAAAACCG |
| 161 | cg20973210 | 61/62 | 19 | 2282669 | 2282790 | NM_198532 | CGGCTCTGCCGCGCCTCGCTGTGTGAC<br>TGCGGGCAAATCCCCACCCTCTCCGTGC<br>TCCGG[CG]AAGAAAGAAGAGTGGCTCCC<br>ACATCCCGCCTGCTAACAGCCACGCCTG<br>CTGGATTTTTCT |
| 162 | cg21179088 | 61/62 | 7 | 54612273 | 54612394 | NM_182546 | AGGGGTGTGGATATTCCAACAGGATGCC<br>TTTCTGTTTTGCAGCAAAATTTACCGAGT<br>TTC[CG]CGGAACGTGACGGCGACCGAG<br>GGGCAGAATGTGGAGATGTCCTGCGCC<br>TTCCAGAGCGGC |

**Fig. 27**

| SEQ ID: | cgID | Cg Position | Chr No. | Chr. Start | Chr. End | Gene | Sequence |
|---|---|---|---|---|---|---|---|
| 163 | cg21317965 | 61/62 | 6 | 50803790 | 50803911 | NM_003221 | ATCGGTGACTTCTCTAATGATGAATAAAG ACGGCTTCCTGGGAGGCATGTCTGTCAA CAC[CG]GCGAGGTGTTTTGCTCCGTCCC AGGCCGTTTGTCTCTGCTCAGTTCAACT TCGAAGTACA |
| 164 | cg21355828 | 61/62 | 7 | 149389754 | 149389875 | | GGAAGGGGCCCTCGGTCACTAAGTCCTT CCTCGCGAAGCCCGCAGGCATCTCCAG CCGCG[CG]CAGTCCTGGGCGCTCCCGC CGCCGCCCACCGAGGCACAGGCACCTT CTCCGTGAGCCGGG |
| 165 | cg21454031 | 61/62 | 17 | 73030296 | 73030417 | | AAGAATAAAGTTGCCCAAAGGGTTCCCA GGACACCCCAGCTCCACCTGGCGGGGC AGCCC[CG]GAGAACCAGAGGTAAATGAC CACCGCGACTGCAAACAGAGGGTCCTG CAAGAGAAGGCGA |
| 166 | cg21488279 | 61/62 | 5 | 122434118 | 122434239 | NM_001136239 | CCGCGCTGAGGCGCAGACACGGCGGCT CCTCCAGGCCCATTAATAACCCGGAGGG CGCTG[CG]ATCCGACAGCGCAGTCGCC ACAGGCTCTATTTCCCGTGGCCGCCTGC CTCCTTCCAGCCC |
| 167 | cg21493505 | 61/62 | 19 | 56879358 | 56879479 | NR_024056 NR_024057 NR_024055 NR_003127 | AAAGCCTCCCACGAAGTCTGAACCTGGT ACTGATATACGCTTCAGCCCTCAGAAGG CAAC[CG]CTTGATTCTCTGCGCAGGCGC AGCGTTCTCAACACCGCCAACGGGCACA AAGCCACGCCC |
| 168 | cg21595709 cg24745495 | 34/35 87/88 | 19 | 15344153 | 15344273 | NM_024794 NM_001142886 | TGCTACCCACCCAGGGGCGCGCGACCC TCCCTT[CG]GTCTGGCTCCAAAGACCTA GCAGCACTGACTTCACCCAGCTGTGGTT CCAA[CG]GCGGGTCCAGCGGCCTCGGC CCGGCGCCGTCC |

Fig. 28

| SEQ ID: | cgID | Cg Position | Chr No. | Chr. Start | Chr. End | Gene | Sequence |
|---|---|---|---|---|---|---|---|
| 169 | cg21996227 | 61/62 | 1 | 156130766 | 156130887 | NM_022367 | CGAGTTTGACTTCTTTGAGAGGCTCCAC ACATCGCGGGTGGCTAGAGTCTGCAAG GTCCG[CG]GCCTGGGCGGGGGGCGGG GCTAACTGGAGGAGAACCAATAGGGAG ATGGCAGGGGCAGGA |
| 170 | cg22195627 | 61/62 | 19 | 12305809 | 12305930 | | AGGTGGGCGTCGTACGTCGTTACGCAG CGCGGTTGCTGGGCACCTCGACTATCAC CTGAC[CG]TAGTAATATCTCCCGCTACG CGCGTTGTGACCAATGCTGCATACAGGA GATGAGGGAGGA |
| 171 | cg22517995 | 61/62 | 19 | 37096528 | 37096649 | NM_032825 NR_027239 NM_001145649 NM_001145650 | CATCGCTGGCGGCGGGTCCCCGCGCGG AGCGGGCGCTGTGTGTTCGCGCGCGCG GGCGCG[CG]CTGAATAGGGGTGGCCTC CTGCGCCCAACCTGCTGCATTTTAGGTG CTTGCCTCTCTTTG |
| 172 | cg22531904 | 61/62 | 7 | 99595216 | 99595337 | | GTACACGCCCTGGGAATCGGGGGTCTT CCAGTATAGGGAACCACCGAGATGCGG GCCCTC[CG]AAGTCCTAGAGAGGGCGC CTCGGCCGCCGGCACCCGCCACACTTC CGGCCTTTGTGGGCC |
| 173 | cg22633280 | 61/62 | 10 | 110671847 | 110671968 | | ACCTCCCTCCCGCTGTGCAAAGGAACGC AGGAGCCCGGCGCAGGTGGTGGGCTTA CCGCG[CG]CACGCCTGGCTGGAGAGGT GACGCCGCTGTCTGCCAACCTTTCCCAG CTTTTCCCACGAT |
| 174 | cg22694818 | 61/62 | 1 | 75595910 | 75596031 | NM_001001933 | GACTGGAAACGCTTCCCCTATTTCTTCC GTAGCGGACCGGGAGAGCTTACTGGCG CTCTG[CG]AACCGGCTGGAAAGAAACAC CGAGTCACTCGTACAGACTCTTGGTCGC AGAACTTGGCTT |

Fig. 29

| SEQ ID: | cgID | Cg Position | Chr No. | Chr. Start | Chr. End | Gene | Sequence |
|---|---|---|---|---|---|---|---|
| 175 | cg23077820 | 61/62 | 2 | 223154116 | 223154237 | NM_181458<br>NM_181461<br>NM_181459<br>NM_181460<br>NM_181457<br>NM_001127366 | CGGCCACACTCCTATTCACGTGATCGAT<br>TTCTGCATATTCCACTCGCCTGAACCGC<br>CGCG[CG]CTGACTGGTTCCGCCTCACC<br>GCCCGGGTGGGTTTTATTGCTCAGCCCT<br>GGGGACTTTTAA |
| 176 | cg23290344 | 61/62 | 8 | 24771406 | 24771527 | NM_001105541<br>NM_005382 | GGCTTCCGCTCGCAGTCGTGGTCCCGC<br>GGCTCGCCCAGCACCGTGTCCTCCTCCT<br>ATAAG[CG]CAGCATGCTCGCCCCGCGC<br>CTCGCTTACAGCTCGGCCATGCTCAGCT<br>CCGCCGAGAGCAG |
| 177 | cg23350904 | 61/62 | 17 | 4648520 | 4648641 | NM_001136046<br>NM_032265 | TGCCAGCCTTCTTCACCGAGAGCAGCGA<br>GTACAGCTGTGTGATGGACGGCCAGAC<br>CATGG[CG]GTGGCCACTGGAGGGGGCA<br>CCAGCCCTCCCCAGCCCAACCCCTTCC<br>GCTCCCCCTTTCGC |
| 178 | cg23353952 | 61/62 | 22 | 29876885 | 29877006 | NM_021076 | GCGCGCGTGGACCTGCAGAAGAAGGCG<br>CAGGCGCTGCAGGAGGAGTGCGGCTAC<br>CTGCGG[CG]CCACCACCAGGAAGAGGT<br>GGGCGAGCTGCTCGGCCAGATCCAGGG<br>CTCCGGCGCCGCGCA |
| 179 | cg23368787 | 61/62 | 19 | 36049282 | 36049403 | NM_000704 | GTTGAAGGGTATCTCGCAGACTTTTGGG<br>AAGCGGTCCCGGTAGCCCATGGCGTTG<br>CCCAG[CG]TCAGCTCCGAGAACTTGAGC<br>AGCGCCGTCTCCGATGCGTCTCCAATCA<br>CGATGCGCTGGG |
| 180 | cg23449696 | 61/62 | 3 | 147125882 | 147126003 | NM_003412 | AAATTTTCTCTGGCGATCAAAAGCCTGG<br>TCGGCAACAAAGACCGCGCCCGCTTTTT<br>CCAC[CG]TCCTGGTGTGGCGAGTTGCG<br>GAATTTCAATAACTGTGACAAGGGTGAC<br>TGATTTGTGAAC |

Fig. 30

| SEQ ID: | cgID | Cg Position | Chr No. | Chr. Start | Chr. End | Gene | Sequence |
|---|---|---|---|---|---|---|---|
| 181 | cg23676577 | 61/62 | 14 | 37049505 | 37049626 | NM_014360 | GACGCCTCCCGCTGACAAGAATCCCAAAGTTGCCTCAGCTTCCTCCTCTCCTTTTCTTCC[CG]CGCCCAACCCTGACATCACCACCCCTCCATCCCCCAGGTATCTTCCTAAAGCCGTGTCGG |
| 182 | cg24199834 | 61/62 | 4 | 147560066 | 147560187 | NM_004575<br>NM_004575 | CAGCTAGCGGCAAGCGGAGTCAGGCATCCGTTCAGACTGACAGCAGAGGCGGCGAAGGAG[CG]CGTAGCCGAGATCAGGCGTACAGAGTCCGGAGGCGGCGGCGGGTGAGCTCAACTTCGCAC |
| 183 | cg24411961 | 61/62 | 1 | 34642336 | 34642457 | NM_032884<br>NM_001134734 | CGAGGTTGCCGGGGGACCCCCAGGCAGGAGCTGAGATTTGTGTGGAGCGGCGAAGTGGGG[CG]GCGGCGCGTTTGGCTTTGACTTCTGATCCCCCGAAGCGCTCTGGGGTCCTTTGGGCCCCA |
| 184 | cg24673101 | 61/62 | 14 | 100069780 | 100069901 | NM_001144995 | CGCCCCCGCCGCCGCCGCCACCGCTTGCGGCCCCCGTCGCCGCCAGTGCCGCGCGCTCCT[CG]TCCAGCAGCAGCACCAGCTCCTTGAGCTCCAGGTTCTCGCGCAGCAGGGCCTCCTGGCGC |
| 185 | cg24715245 | 61/62 | 4 | 41258734 | 41258855 | NM_004181 | TCTCCACAACCACCAGATTATCTCACCGGCGAGTGAGACTGCAAGGTTTGGGGGCCCGGC[CG]TACCACTCCGCGCTGCGCACGGGGGGTTCGTACCCATCTGGCCGCGACCGTCCGTTTCCC |
| 186 | cg24766821 | 61/62 | 2 | 186603579 | 186603700 | | GAGCGGGGCGGGTGAGGAAGGGGCTGAGGGGGCTGTGCCGGCCATGGAGCTGTACCTCGG[CG]CCTGCTCCAAGCCTGCCAAAGTCGCCGTCACCAAGACGGTCGCCAGCGTCCTGGCCGCGG |

**Fig. 31**

| SEQ ID: | cgID | Cg Position | Chr No. | Chr. Start | Chr. End | Gene | Sequence |
|---|---|---|---|---|---|---|---|
| 187 | cg24809973 | 61/62 | 8 | 72468760 | 72468881 | | TCGGTCTTCTCCCGCCCCTCCCTCCCTTCCCCGCCTCTCCCCCAAGCTCCTCAGTGGCCG[CG]GCCCGTCAACACTGTCGCGCAGTCACTGGCGCAGGTTCCCAGCTCTCAGCTGGGGGTTTC |
| 188 | cg25032595 | 61/62 | 13 | 96204918 | 96205039 | NM_006984 NM_006984 NM_182848 NM_001160100 | GGAGGCGGGAGGCGGAGCCCCGGGGTTGGGAGTGCGGGGGTCGCGGCGCAGAGTGGGAGC[CG]GAGAGCGAGCGCGGCTGCAGCCGGCGGCATGGCTAGCACGGCTTCGGAGATCATCGCCTT |
| 189 | cg25285090 | 61/62 | 6 | 28510253 | 28510374 | | CAGATATTGCGGACAGTCTAATTCGCCCCGCGACGTGAGGGAGAACCCAGGAGCGGGCTC[CG]GTAGAGAAAGAAGCTTCCGGTCAACGACCACATCCACCTGAATCATGAGCAGGTTTTAAG |
| 190 | cg25334393 | 61/62 | 8 | 145955611 | 145955732 | NM_138367 | GAAAGAGCAGCAAGGGTGGGCTCTGCTGCCGCCCAGAGGCCTTCCGTGGTAAGACAGCCA[CG]GAAAGCACAGCGCGTGCCGCTGCGACGGGGCGTCCCGGGGGTGACACTGGCATGTGACGC |
| 191 | cg25397945 | 61/62 | 19 | 37096088 | 37096209 | NR_027239 NM_001145649 NM_001145650 NM_032825 NM_001145650 NM_001145649 | GTGCCAGCCGCAATAGGGCGGCCGTGGGTGCAAACGGAGGGGAGCGCCGGCAGCTAGCAC[CG]CGCGGCGACTAACGGGCCGCCCCGGAGACTCCTGGGAGCTCAGGCCCACGCGCGAGTGCG |
| 192 | cg25537993 | 61/62 | 19 | 58545122 | 58545243 | NM_182572 | CGTGTCTGGAATGCCGTCGTTTTGCCCCGCGGCCCCATCGTACATGCGCATCCCCGCAGC[CG]CCACCGTCTCCACTTTCCGCAGCAGCGTCCCTGACGCTGCGCGACCCGTTGCCAAGGCGA |

Fig. 32

| SEQ ID: | cgID | Cg Position | Chr No. | Chr. Start | Chr. End | Gene | Sequence |
|---|---|---|---|---|---|---|---|
| 193 | cg25641145 | 61/62 | 8 | 101572306 | 101572427 | NM_198401 | ATTAGGTGCTATAAGCATCAATTAATAGTTGCTGGCCTCTAGGTAGGTCTTCTATAGACC[CG]AAATTGACATATTAGGAATAGCCACTAATGATTGGCTGCACTATTTCTAAAATATAACCC |
| 194 | cg25729445 | 61/62 | 6 | 29595287 | 29595408 | NM_021904 NM_021903 NM_001470 | GAGCTCATAGTCCGGCAGGATGTCCCTGCGGCTATTCACGTCCTCCAGCGCCATCTCCAC[CG]CGGGCTGGCAGGCCTGGCCCCCTGGCCAGCCCCCGCTCATGGGAAACAGTGCCCCGATGT |
| 195 | cg26156279 | 61/62 | 10 | 103374583 | 103374704 | NM_022039 | CAGCAGTGCCACAGGAGCCCTGCTGGGGCTTTGATTTGGGAAAACAAACAGAAATGGCAC[CG]TGGGCCCTTCTCGGCCCCCACCCCAAGACCTATTTAACTAGAAAACTCTAGCTCAACTGC |
| 196 | cg26886462 | 61/62 | 5 | 155108389 | 155108510 | | CCAACTCGCCACCCATGGGATCGACTGCCCTCAGCTGCCCGGCTTGGAGTGCGTAGCCTT[CG]CCGCGTGGCCCAGGAGAGGAGCGAGACACCAAGGCGTTTCGCGGACTCCGCGCGCACCAG |
| 197 | cg26952618 | 61/62 | 16 | 10912485 | 10912606 | NM_001079512 NM_001079512 | TGGGGCAGCCTCAGCGCAGCTTCTCGGGTGGGGCGGGGCGCTCGGGGCCTAAGGCGCAGT[CG]CAGGCTGGGGAGGGGGCTCGGCTCGCCGGGGACGCGCCCAGGAGAGAAAGCGGCGGCAGG |
| 198 | cg26993102 | 61/62 | 6 | 30228185 | 30228306 | NR_027822 | TCGCCTACGATGGCAAGGATTACATCGCCCTGAACGAGGACCTGCACTCCTGGACCGCCG[CG]AACACAGCGGCTCAGATCTCCCAGCACAAGTGGGAAGCGGACAAATACTCAGAGCAGGTC |

Fig. 33

| SEQ ID: | cgID | Cg Position | Chr No. | Chr. Start | Chr. End | Gene | Sequence |
|---|---|---|---|---|---|---|---|
| 199 | cg27088072 | 61/62 | 2 | 242481393 | 242481514 | | CCCCTCTGCAGAGCAGGCTTGGCCGAGGAGTAAGCGCGGCCGCTGCGGAGGGTTTGGCAG[CG]GGGACAGGGCTGCGCCCGGTGTGTGCGCGGACACCTGCCGCCCGTCCTCAGCGCCCGGTA |
| 200 | cg27187667 | 61/62 | 18 | 44774949 | 44775070 | | GTCTGGCTGAGTGTACTTGGCGTCGGGCGTGCGGTGGGAGTGGAAAATGAACTTGTTGGG[CG]AGAAGTACATGTTGCAGTAGCTGCATTTGATGCACTTGGCGCGCGAGCTGTTGTAGCGCG |
| 201 | cg27235148 | 61/62 | 11 | 45376872 | 45376993 | | GGCGGAGACGGTTAGTCTAGCCTCTCTGTGGGGCCTCTCTGCTGGTACCCAGATGCTGAG[CG]CTGAAATTCTCATCCGCCACACACAACATGGCGGCTTCCTGGACCCGCGCCCAATTTTCG |

Fig. 34

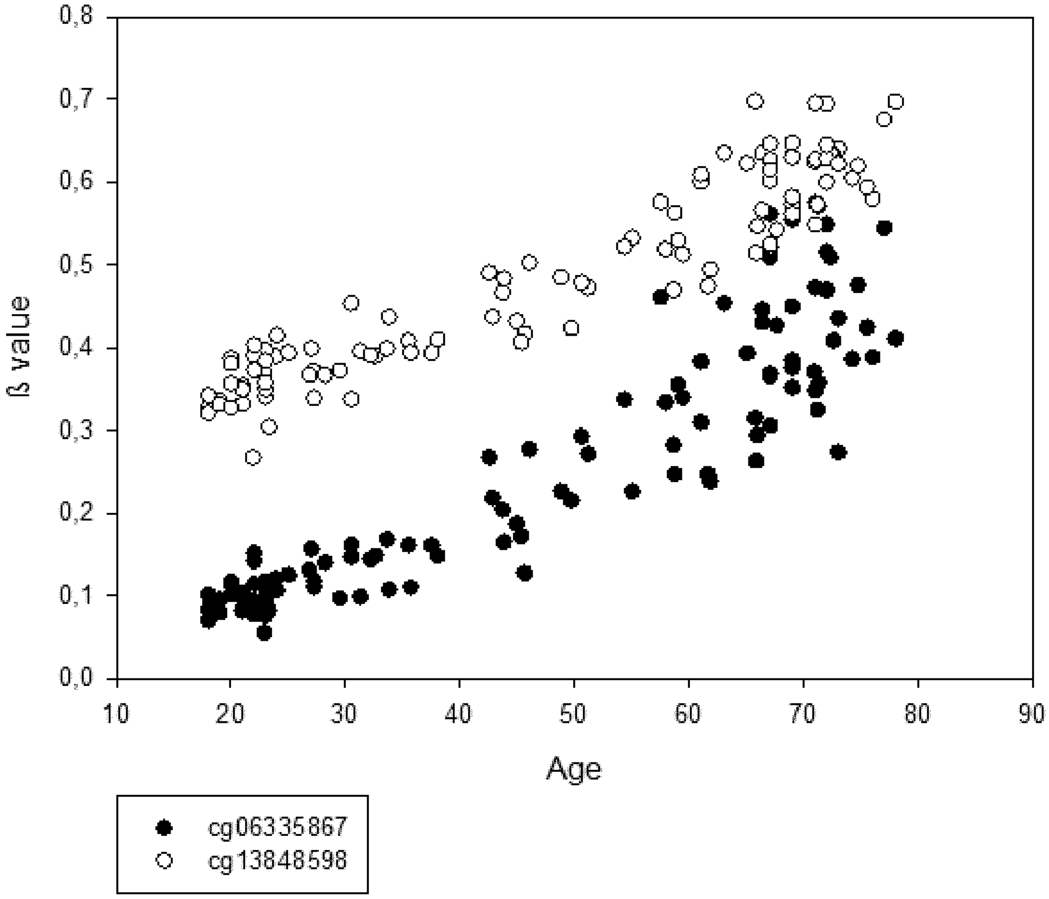
0,8
0,7
0,6
0,5
0,4
0,3
0,2
0,1
0,0
ß value
10
20
30
40
50
60
70
80
90
Age
cg06335867
cg13848598

Fig. 35

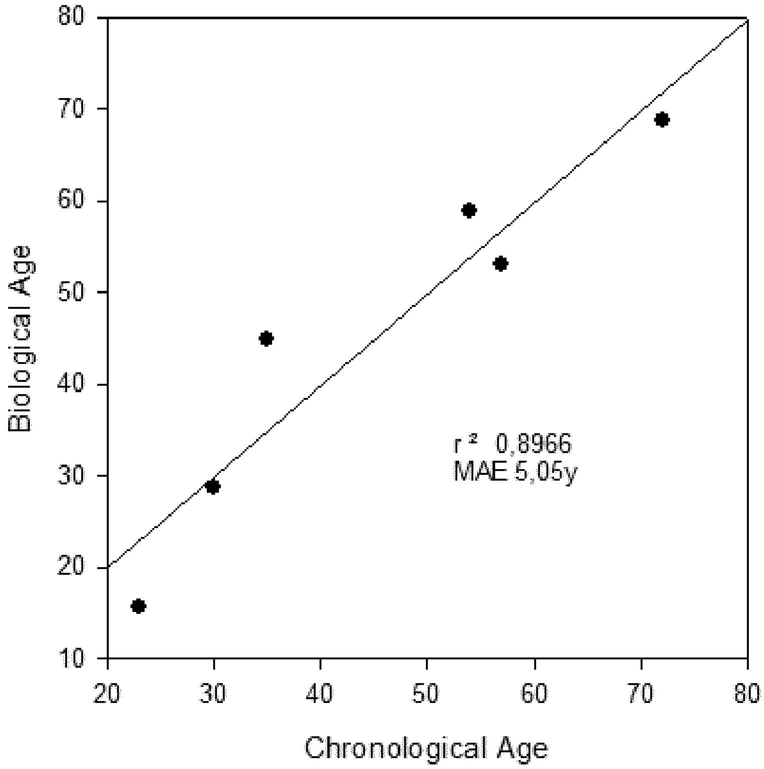
80
70
60
50
40
30
20
10
Biological Age
r² 0,8966
MAE 5,05y
20
30
40
50
60
70
80
Chronological Age

Fig. 36

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2711431 A


### Non-patent literature cited in the description


- **WEIDNER et al.** *Genome Biology,* 2014, 15R24
- **HORVATH.** *Genome Biology,* 2013, vol. 14, R115
- **BORMANN et al.** *Aging Cell,* 2016, vol. 15, 563-571

SEQUENCE LISTING

<110> Beiersdorf AG

<120> AGE DETERTMINATION OF A HUMAN INDIVIDUAL

<130> P131237EP00

<160> 201

<170> PatentIn version 3.5

<210> 1
<211> 122
<212> DNA
<213> Homo sapiens

<400> 1

gcggagctgc gacctcgggc agctggaagc ctgtgggctg ctgcttctcc aggcaggcgg 60

cggagggatg caggcaggat gagcggcggt cccttcgccg ccacccggag aaccgcggct 120

gg 122

<210> 2
<211> 122
<212> DNA
<213> Homo sapiens

<400> 2

ccctgggtct ccggtttcct ctgccctttc tccaaaaaca attctatggg gctgcaaagg 60

cgtagcgggt caggctggcg gggcggccgc tgtccgcggt gctgattccc tggtcctcgc 120

ag 122

<210> 3
<211> 122
<212> DNA
<213> Homo sapiens

<400> 3

caaccggggc ccaggcccag gtgggcgggc ggctgaggag cgtggctgcg cccacaaagc 60

cgccgggggc tgcggactac agcgaagccg gcgcggggct cggccctcac tacacgaggc 120

ct 122

<210> 4
<211> 122
<212> DNA
<213> Homo sapiens

<400> 4

actttcgagca gctggcggag cgcgagggcg agatcgagca gggtgagcgc cacggaactg 60

cgcccctccc gcggacggcc tccggaggac agccccgctc cacagccgct ccccccttccc 120

ca 122

<210> 5
<211> 122
<212> DNA
<213> Homo sapiens

<400> 5

aacgacccct tctccaggtg cgcgagccgc tccggcggcc gtgcacactg cgccccccttc 60

cgcccacctg cctggcctgc gtttctaacc acgcgggcgg tcccgagact tcgcgcaaaa 120

gg 122

<210> 6
<211> 122
<212> DNA
<213> Homo sapiens

<400> 6

cagcagcacc gcctggctca gccaggtcca ggaaggcctg gcgcagcagg gccgggcttt 60

cgcctagcgc gcagcctagc gccgagggcg gctgaggcgg gggctgcagg taggtgggca 120

cc 122

<210> 7
<211> 122
<212> DNA
<213> Homo sapiens

<400> 7

gtgaattacc gtctacaaca gctactctcc aggctgatct ggggtcttgc acacaaaggg 60

cgaagagagg ggcgtgggga ggctggaaag catggttgcc ccgcctggcc cggcgacgcc 120

cg 122

<210> 8
<211> 122
<212> DNA
<213> Homo sapiens

<400> 8

acagaggagc agcagaagcc gccgccgcag acgcccggat gcggaggacc cagagcgctg 60

cgcagctgag gagcacctgg ggagccaccg ccggcgccaa cttttgccat cccgggagcc 120

at 122

<210> 9
<211> 120
<212> DNA
<213> Homo sapiens

<400> 9

cagagccgca ggtagtagtc ggggctcccg gaggccctgg gcacttgcac cagaatcgcc 60

ttgcgcctcc ggtccaccca gtagctgagc ttccccgctg caggaggcgg caagtcgggg 120

<210> 10
<211> 122
<212> DNA
<213> Homo sapiens

<400> 10

tcgccttctt cattggcgga agctgggagc cgagagagtg agccgtgcgc agatgtgcgg 60

cggtggaggt ggcttttgtt tctcctccgc gccccctccc cccccgcccc gcacattgcc 120

tg 122

<210> 11
<211> 122
<212> DNA
<213> Homo sapiens

<400> 11

cacgctcagg ggaggggccg ggcttgccag gagcagcggg tcctcgctga gcagatcctc 60

cgggggcccc tgggaggcgg cctcaggggg cccaaggagg cagctgagca ggtcggtgtt 120

gc 122

<210> 12
<211> 122
<212> DNA
<213> Homo sapiens

<400> 12

gccatgcaat gccttctcaa ggcccagcac ttcgcccgat ccctgggagg gatggacagt 60

cgtgcgcccc atagaggttt gggtcaacag cctgcatata ggaccgtggt cccataagat 120

tt 122

<210> 13

<211> 122
<212> DNA
<213> Homo sapiens

<400> 13

gcgctgcggg ctgccgggaa ctgttctccg ctcggggtgc tgaaagcgga cgcgggagag 60

cgcgcagaga aggcgaggag ccgggtcggc caggctctcc tgcaggcgcg ggtcctgctc 120

gc 122

<210> 14
<211> 122
<212> DNA
<213> Homo sapiens

<400> 14

caggagcaaa gcagcgcgcc aggctggagc tgttccgtca ttaccgccac gtctgcgctt 60

cggttgtgag aaccgattcg ttaccctaac ctcttaggac taagagccag tcttctggtg 120

gc 122

<210> 15
<211> 122
<212> DNA
<213> Homo sapiens

<400> 15

gcggggtggg gggctgttgg tgcgggagcg cctgcgccgc tgtcgctgta gaaggtcgag 60

cggtcacttg ttccttcgtt cattcattca ctcatccatc catttgtttg ttcactcatt 120

cc 122

<210> 16
<211> 120
<212> DNA
<213> Homo sapiens

<400> 16

gtgctgagga ggccaaggag ggcactggga gctccgttcc gggagagctg tctaaggacg 60

cggaaccggg tggggaggcc agggaggtaa ccaagagtcc gagccgggcc tggaaattct 120

<210> 17
<211> 122
<212> DNA
<213> Homo sapiens

<400> 17

aagaaagctg ccatcacagg cagcagacct ttgttctctg accacttgat aatgtcagga 60

cgcggcaaag gaggtaaggg cctggggaaa gggggtgcca agcgccaccg caaggtgctg 120

cg 122

<210> 18
<211> 122
<212> DNA
<213> Homo sapiens

<400> 18

gcccgagagg atccagggaa agcagaaggg ggttaaggac catggacaga gcccgtcgcg 60

cgctcgttgc tgccgccttc cccagcactc tggcggctcc tgaggacagc ggtcccatct 120

tg 122

<210> 19
<211> 120
<212> DNA
<213> Homo sapiens

<400> 19

tggagcaggg cagcccccag aacggcgccc cgcggcccat gcgcttcccg cgcaccgtcg 60

ccgtgtactc gcccctggcc ttgcgccgtc tcaccgcctt cctggagggg cccgggcccg 120

<210> 20
<211> 122
<212> DNA
<213> Homo sapiens

<400> 20

caatcgtctg agcctgtcct cttattcccg gttgtaacta aatactgttg cgagcgcagc 60

cgaagccctt tgttggagat gtgtgagcgc agtctctaca gagcgggcta tgtgggctcg 120

ct 122

<210> 21
<211> 120
<212> DNA
<213> Homo sapiens

<400> 21

gagctcccct ctgctgctga ggcggctctc ggtggaagag tccggtctgg gcctcggcct 60

gggcccgggc cgctccccgc acctgaggcg cctgtcgcgc gccggcccgc gtctgctgag 120

<210> 22
<211> 122

<212> DNA
<213> Homo sapiens

<400> 22

ggccaatggc gacgctccca ccgcctccgt cccccaggtt gaaagcggcc cgggagccgg 60

cggcccaccc cgaaggcccc cgttcccggc tggctagccc ggcagatgct ggaggtctgg 120

ga 122

<210> 23
<211> 122
<212> DNA
<213> Homo sapiens

<400> 23

ggcgtaacta caatgaatcc ccaaggttgc ttctttctcc tgcacagggg actgggagga 60

cgctgcgccg gcagaggagc gcggtacccg ggactccctt ttcagcaccc caaggggcgg 120

ac 122

<210> 24
<211> 122
<212> DNA
<213> Homo sapiens

<400> 24

ggccgcgaac ccacaagtca agattcgcag gtgcgcgcgc taaggactga gctattgggg 60

cgatggagag agtctcgctc tgcgtcaggg acggaaggac gtgtccccca ccagaaaaag 120

ga 122

<210> 25
<211> 120
<212> DNA
<213> Homo sapiens

<400> 25

gcggggtgcg gcccctcgtg cccactcctg cgggccgggg tgagcgcgtg aggctcaggc 60

ccctgggcct gcccgcagtg gcctgtccca ccctggcctt ggggacagcc ggccttcgcg 120

<210> 26
<211> 122
<212> DNA
<213> Homo sapiens

<400> 26

gaccctgggt ggcaccttct aagggtgatt ctgggattct atgaattaat gcatggaaag 60

cgcttgcgag agcgctagcc ggagtccgcg ctatttaggt gtttgccaat attattgttg 120

tt 122

<210> 27
<211> 122
<212> DNA
<213> Homo sapiens

<400> 27

acccaggtcc ttcgaagaag gttgggaccg agctgctgcc gcgtgtgaag gtgtgtgtcg 60

cggctggggg tgccacaacg ggccatggag cccacctccc agagggagga agtctgtgca 120

ca 122

<210> 28
<211> 122
<212> DNA
<213> Homo sapiens

<400> 28

ctgcaccccg gcgggcgcac agacggtccc cagcggcggc ctgggccagc ggcgaagcag 60

cggcagacgg ttctccggcc cccgccgccc cctcaccgct cccggggcaa tctggcgctc 120

ag 122

<210> 29
<211> 122
<212> DNA
<213> Homo sapiens

<400> 29

gtcggacgcc gggcgggcgg atgtaggcag cagacggtgg acgctgaggg tcggggcctg 60

cgccctgtgg ggcagcctca gcgcagcttc tcgggtgggg cggggcgctc ggggcctaag 120

gc 122

<210> 30
<211> 122
<212> DNA
<213> Homo sapiens

<400> 30

tcctgtgctc ccaggtctgg gcgttaggat tctctcagtc ccggagccac gccggctgac 60

cgcagggctc ggggagcgcg gctgggcccc ttttcccggg tccgggaagc gccgggccac 120

gc 122

<210> 31
<211> 122
<212> DNA
<213> Homo sapiens

<400> 31

ctcggcgcga gcgggactat cagggtgacc tcacctgtga ccttcaccac cgtgaggtac 60

cgccgcgcct gggagtcacc cgcggccgct cgccggtagg tgcctgcctg agtctaatcg 120

ta 122

<210> 32
<211> 122
<212> DNA
<213> Homo sapiens

<400> 32

ctcggaaggg gcaggggaat gagcccaggg accccagcgg ggcgcaggta ggaggctgtg 60

cgctcgccgg gtgcgctccg gccccgattc ccagcgcagc cagtaagtgg cgctgggcct 120

cg 122

<210> 33
<211> 122
<212> DNA
<213> Homo sapiens

<400> 33

gtcgccaggc cagaggttag agggacatgt tcatggctta aatttattgc cctgactctt 60

cggagcggct tcctctcctc ctccccaccc cccggctttt gccattcatg ggccgctgga 120

tc 122

<210> 34
<211> 122
<212> DNA
<213> Homo sapiens

<400> 34

cggctggccg gcgccgcctc ctgggaagat ggcgctgcac ttccaggtca gtgtgctctg 60

cgccgcgggc ccgcgctccg ccacgctggg aacccggcgg gacgcgtctg gagaccgagg 120

gc 122

<210> 35
<211> 120
<212> DNA
<213> Homo sapiens

<400> 35

gattccgtgc ccggtggccg cgcgccgccc ggctgttccg cgtccccttg ccggctcaca 60

atgtgctgac cctcccggcc ccgccgcgcg cctcacaggt ctctcgacct ccgcgctccc 120

<210> 36
<211> 120
<212> DNA
<213> Homo sapiens

<400> 36

gcgcggccct gctgccaccc ccttgggggc tcggagcgcg acagcagctt ggggacgcct 60

cccgcgccca gcacggtgca cctgggccct gaggtcctgg ccgaaacgcg ccaagttggg 120

<210> 37
<211> 122
<212> DNA
<213> Homo sapiens

<400> 37

tgccccactc tgcctaagga aacgtagaaa ctttcccgat tcggatgggg aggggtaggg 60

cggagagtcc cagagaccag ctctggcttc gtttgcattg gaaattagca caaggtagtg 120

tt 122

<210> 38
<211> 122
<212> DNA
<213> Homo sapiens

<400> 38

cgagccgggc ctcgggcgtg gtccatgggc gtgaccttga gggtgacccg gagcgagctc 60

cgcgaagggg gattttgccc gttctgatcc cgcggaagcg ccatcgctgg cggcgggtcc 120

cc 122

<210> 39
<211> 122
<212> DNA
<213> Homo sapiens

<400> 39

ccctaatgta aaagtccccc tgcgagtttc cagcacggat ctgaaaggca ttgtaggcac 60

cggggtagac ggaggtcgcc tggatctgga acacgtcagc gggcacgctc cgctccgagg 120

tg 122

<210> 40
<211> 122
<212> DNA
<213> Homo sapiens

<400> 40

gctggacacg ctcaggctgg cgtccagcta catcgcccac ttgaggcaga tcctggctaa 60

cgacaaatac gagaacgggt acattcaccc ggtcaacctg gtgagtgctc ccggggctgc 120

ag 122

<210> 41
<211> 122
<212> DNA
<213> Homo sapiens

<400> 41

ctcacgtctc ttatgctggt taagcgcgac taccgctatg atcgggttct cttctggggc 60

cgcatccttg gcctcgtcgc cgattactac atcgcgcagg gcctgagtga ggaccagctc 120

gc 122

<210> 42
<211> 122
<212> DNA
<213> Homo sapiens

<400> 42

gagtgaggcg gagacccagg cgggcgggca gagcaggcgg caccgcacct cggccagagg 60

cggctgcagc agctgctgcc cttgtccctg ccgccgcctc tccagtccct tctgtgatta 120

cc 122

<210> 43
<211> 120
<212> DNA
<213> Homo sapiens

<400> 43

ccggggcagg gctggggtcc cggcgctgag cgcccccagc cgcgccgggc agcctggcgc 60

ctcgctgagt cggagcaacc cccggcagga gctttcaggc tctggtcaag cctcgggacc 120

<210> 44
<211> 122
<212> DNA
<213> Homo sapiens

<400> 44

tcgtagtcca cgggcccggc cagggtgagg cggcctgatc gcgggtcaag ccgaaagagg 60

cggcgcgcct ccggcggggt gcgggcgcca aatgcgaaca ccacgtcgcc gttaggtccc 120

tc 122

<210> 45
<211> 120
<212> DNA
<213> Homo sapiens

<400> 45

ggcccggggg gcagcagcgg gggtgggacg tggtggtggt ggtcggggct gccgccgctg 60

cccgcgctgg acgtactgct gccgtcgccc acgtcgcggg cccccgcgcc gggcgcgcca 120

<210> 46
<211> 122
<212> DNA
<213> Homo sapiens

<400> 46

agttggcgtc ctcagagtgg ccgctgccat cagactctgc gggtagagct gggccgggag 60

cgacgggcga cattggtagg gacccgggga cagcggtccc tatcccaggc ctgacgtggg 120

tc 122

<210> 47
<211> 122
<212> DNA
<213> Homo sapiens

<400> 47

ttggctaaaa aaaaagttgc tactcctggc agccctggtt tgtcaaaagg ggatgtcaag 60

cgctttacaa tacctgggat tgatgaggcg ggcgggccaa tgagctgcgc gcggcgcctc 120

gg 122

<210> 48
<211> 122
<212> DNA
<213> Homo sapiens

<400> 48

ggagaggcgg acaggagggc ggcggagagc gctgggccgg ttgtctccag cgcgcactat 60

cgcgggcgcg tagtagatgt cgctgttgtc cgtgcttacc cggccggccg gccaggctct 120

gg 122

<210> 49

<211> 122
<212> DNA
<213> Homo sapiens

<400> 49

tgaccacccg agggttcccc ttttccactc tccttcccac tctgtttttg tcccagcgcg 60

cgccagcgcc tctcaggcct gccgcctgct ctcgcacctg ctcgccttcc ccaggcgccc 120

ag 122

<210> 50
<211> 122
<212> DNA
<213> Homo sapiens

<400> 50

gaggtgcggc gcttccctaa gcgtgggcta cttccgtatt tccgagacag ccaatgaccg 60

cgataggtgt cttccttgac agcacagtct catgtccccg acatccagac ttactcgtgg 120

cg 122

<210> 51
<211> 122
<212> DNA
<213> Homo sapiens

<400> 51

cagtccgctg accaaagccg cagtgttcag gccccggggt ttcccagccg tagtggccgc 60

cgccacagct gcgcgcttta ttgtctgctt tcagtcgcag gtgacctcga gcgatctcga 120

ca 122

<210> 52
<211> 122
<212> DNA
<213> Homo sapiens

<400> 52

cggcggcggc ggcgggcgga ctggggagtc cgcagcacct gcgagctggt gttgggtccc 60

cgcggcgagg ctgcctggag gaggagcgag cggatgctgc cctcgcccgc cgccgccggt 120

gc 122

<210> 53
<211> 122
<212> DNA
<213> Homo sapiens

<400> 53

aaaaatgctt tgagataaac ctggagccac agctctctgc acagtcagat cgcaggaacg 60

cggcctagct gcttcagcca cgttctcgcg gcgccacctg gtggccatgg accgccatcg 120

cc 122

<210> 54
<211> 122
<212> DNA
<213> Homo sapiens

<400> 54

gcctccgcca gcagggcctg gacgcaccgc ctcctttgac ctcgggcttc ccccgcgctc 60

cgctgcttgg ggcagactgg ccccgagagg gagccaccat ctcccctgct ccagggtctc 120

ca 122

<210> 55
<211> 122
<212> DNA
<213> Homo sapiens

<400> 55

gaagcgagag caagtgaatg tctcagtccc caggtggcgg tggtggccgc gggggcgcga 60

cggcggggcg ggggcgctgc tgctgcgggg gcaggaagcg gcaagggcgg tggcggcccg 120

ct 122

<210> 56
<211> 122
<212> DNA
<213> Homo sapiens

<400> 56

acacgccgca tagtaaagcg gcagagaact tgcatcaggc gtctctgacc accgagcctg 60

cgaagaccta gctgattacg accaggaaac aggaggggac atggcccaag gtgggcgggg 120

cc 122

<210> 57
<211> 122
<212> DNA
<213> Homo sapiens

<400> 57

gggaggggca cgaggctcct ttgctcccgc aggagcgtcg aatacgtatg gaatacgcgt 60

cggtgtggat ctggcctcag cttcctgagc ctgaatcaat gcttctaatc acgtgggcgc 120

tc 122

<210> 58
<211> 122
<212> DNA
<213> Homo sapiens

<400> 58

```
caaccttggg cgtacggatg tgctccatgg ctggcccacg tctgcagggt cccgggcggg        60
cgcggcctca cgcacctgcg cgcctctgcg gcgcgatggg aggggcgcgg ggagggggct       120
tc                                                                      122
```

<210> 59
<211> 122
<212> DNA
<213> Homo sapiens

<400> 59

```
cgggcaaaaa acagatttgg cgtttccgct ttctcgcttg tgttgggatc caggaaccgg        60
cgacgcgccg gccaagtctc ctttcctggg tctctctcgc gcgctctctc actgatgcac       120
tc                                                                      122
```

<210> 60
<211> 122
<212> DNA
<213> Homo sapiens

<400> 60

```
agcgtacctg tcccgttctg gccttggaga acgcagtggc tcacaagggc accgctagca        60
cgcgaggcgc ggcgaaggga gcggagggaa gcggcgggct gtcgcgcagg tgcggcccca       120
cc                                                                      122
```

<210> 61
<211> 122
<212> DNA
<213> Homo sapiens

<400> 61

```
atcctccaga acccttcagt aatcaaaata aataatagtt caatgcaata tatcttatct        60
cgattataag ataatcaagc catttatctg gattgctgag tcttttggtg cccccttatt       120
tt                                                                      122
```

<210> 62
<211> 122
<212> DNA
<213> Homo sapiens

<400> 62

```
ctttcgcccc aaccggcagc tggcgggcct ggtggagagc gtgcggcggc tggggttggg    60
cgcggggccc ggggcgcggc gatgcgcgcg gcacggcgag gacctgagcc gcttctgcga   120
gg                                                                  122
```

<210> 63
<211> 122
<212> DNA
<213> Homo sapiens

<400> 63

```
tcggcgagcg cgtgcacttg aaccccacgc gcggcttctg gtgcctcaac cgcgagcaac    60
cgcgtggccg ccgctgctcc aactaccacg tgcgcttccg ctgcccacta ggtgagggcg   120
gg                                                                  122
```

<210> 64
<211> 122
<212> DNA
<213> Homo sapiens

<400> 64

```
gctgggtggc ccctcacctg cttacagaag cgcaggaacc cgatggagta ggtcatgccc    60
cggaggcagc aggtgccgta caagcagctg gacctgcggg ggacgtccct gagccggtgc   120
gg                                                                  122
```

<210> 65
<211> 120
<212> DNA
<213> Homo sapiens

<400> 65

```
atggcaccgg tggtctgggg gagaggctgg gcctggcgcg ggacgaggcg aagcgccggt    60
ggccgacggc ttctgaggaa ttatctttta cttggcgcca cacggggcgg ggcctcctgc   120
```

<210> 66
<211> 122
<212> DNA
<213> Homo sapiens

<400> 66

```
caccccatcc ctctctgcag ccgcaggagg tggcccccgc ctgtccccac cttctctccc    60
cgctcctctc gccactctgt cgcagagggg tccggctagc cacgaggccc cggcccccgg   120
ca                                                                  122
```

<210> 67
<211> 122
<212> DNA
<213> Homo sapiens

<400> 67

gcgcatttct ggcctcatct atgaggagac ccgcggagtg ttgaaggtgt tcctggagaa 60

cgtgatccgg gacgccgtga cctacacgga gcacgccaag cgcaagacgg tcaccgccat 120

gg 122

<210> 68
<211> 122
<212> DNA
<213> Homo sapiens

<400> 68

cggggggcct tggatccagg gcgattcaga gggccccggt cggagctgtc ggagattgag 60

cgcgcgcggt cccgggatct ccgacgaggc cctggacccc cgggcggcga agctgcggcg 120

cg 122

<210> 69
<211> 122
<212> DNA
<213> Homo sapiens

<400> 69

cacagatttc ttctgccctg gagaccacag aacttaccct atcgaatcta ggattggcgc 60

cgaagctact cccgcccttt gacgtccccg ggcaccccgc ccccttcacc tcccagcttt 120

gc 122

<210> 70
<211> 122
<212> DNA
<213> Homo sapiens

<400> 70

tcgtcgtcct cggaccatca ctttggcatt tctcgatttt gtctgcttct gaagggaccg 60

cgttgtcagg cgagggacgg aatcttggag gctccctggg cccatggaaa caggagcgag 120

ga 122

<210> 71
<211> 120
<212> DNA
<213> Homo sapiens

<400> 71

gaggagagcg aggctcctcc gggaaagctc cttctgctcc aggtgacagc ggagagagat 60

gccaccgcgc ggcgaccggc agggccgcgt cccctctgcg tcctagcaca gcgacgcccc 120

<210> 72
<211> 122
<212> DNA
<213> Homo sapiens

<400> 72

acgtcctccc aggcgaagac cagctcgtcc tggggactct tgtcggtgag cttgagatga 60

cggcgccacg agttgaagtt ggctgcgtct ggctgagtgt acttggcgtc gggcgtgcgg 120

tg 122

<210> 73
<211> 122
<212> DNA
<213> Homo sapiens

<400> 73

ctcccggtat caccttcgaa cgcgctggcc ggccccctcc tagcctggtg cagtcggctc 60

cgcttctact cagtctcggg tcctcctggg ccaacgcaga ccctggtgcc tccttcctta 120

ga 122

<210> 74
<211> 122
<212> DNA
<213> Homo sapiens

<400> 74

aaattaaccc agccaaagcg ttggggttca agcgagcgac tggaccataa atcagcgtcg 60

cggtggcgtc gggaagctga gcgctcggag gtgcactcag ctgcctctgg gttcgcccag 120

tc 122

<210> 75
<211> 122
<212> DNA
<213> Homo sapiens

<400> 75

tttgtgcaga gctggggtgg gtaatcctgg ggccaggtct gccccctgca gtgccttgac 60

cgtctcctgc cgctgcctca gctttaccct tcaagctcga gtcggttccc gagctctccg 120

tc 122

<210> 76

<211> 122
<212> DNA
<213> Homo sapiens

<400> 76

accccagctc cttcctccca aggcgagtat gtttaccctg ctgggcaccc ttccgctccg 60

cggggaccac tgcgggatcg cgctctctaa aactcctctc aactgccccg cagtgcgcgg 120

cg 122

<210> 77
<211> 120
<212> DNA
<213> Homo sapiens

<400> 77

ctgggtctct ccttcaagac cacatccccc agagaccggc ggagtttcgc acagccttct 60

gggacttata gttctttaag gacgcggagt gggctcacgc cgcgggtagg caaacagagc 120

<210> 78
<211> 122
<212> DNA
<213> Homo sapiens

<400> 78

tgcagaagga agaaccttag ctcactgcag cgccacgccg gggacggcca cccctgcctt 60

cggtcccatc agggctcctc caccagccgt tcccctgcct tgagtccggg ggcacatccc 120

ca 122

<210> 79
<211> 122
<212> DNA
<213> Homo sapiens

<400> 79

ttcttgctct gattgtcagg tcatcatgga atgcaaactt caccgtggac gtgatttcaa 60

cgttggtgcg gatttggaga tgtccaactc tgtcctcaat tagccagaac tcaagttgag 120

at 122

<210> 80
<211> 122
<212> DNA
<213> Homo sapiens

<400> 80

gaggcctgca aacctacttg gtgcacacag atgcagctca tcacagcgcg gtcacaatgt 60

cgggagggaa atgaataaaa tggaaatact accttgttgc ctactgcaga agcagcggct 120

tt 122

<210> 81
<211> 122
<212> DNA
<213> Homo sapiens

<400> 81

ttctggccca cgtcgctgag gtccgggtgc ggattgagct tggtgggcag ggtctgctcg 60

cgacagcccc cgctagacag gagttcgcgc tccttggagt tccgccattt catgcgtcgg 120

tt 122

<210> 82
<211> 122
<212> DNA
<213> Homo sapiens

<400> 82

gcgcccagac tgcgcgccgc gccgctgcgc ccaacattcc cgaggacggc ttcgcgggcg 60

cgtatcgtcc agaccggagc accgccccac cgctagcgca ggagacctgc cggggaagtc 120

gc 122

<210> 83
<211> 122
<212> DNA
<213> Homo sapiens

<400> 83

ggggtcgcca tgaccgagtg gcccaggccc gagcgaagcc cgcgcgcggt gagtccgccg 60

cggcccatcc gtccctccgc ccgccagagc gtccatcggg acgcccaccc gggagggtct 120

cg 122

<210> 84
<211> 122
<212> DNA
<213> Homo sapiens

<400> 84

acgggagttg aggctgaaca aggcgtgggg agcggtggga gctgcagccc gaccgctctc 60

cgtccccgcg cagggagccg ctgccccttg ggagtgggct tagccgttgt ctacgccacc 120

cg 122

<210> 85
<211> 122
<212> DNA
<213> Homo sapiens

<400> 85

actccagggc agcagccaga ctggcatatg tagggctctc cggttacttt ctctgtatgt 60

cgcgggtgag aggaacagcg aggacaattt agcgcaaaca cacgaagggt cggatctcaa 120

gg 122

<210> 86
<211> 122
<212> DNA
<213> Homo sapiens

<400> 86

tcatcccacc cctgtggacc tgtggcagtt ggcaagttcc cggggaggcc aggtgaacca 60

cgatgaacca cggcgacggc aggggccagc gtccccgtct ccctcccgtt ctagaagaca 120

ta 122

<210> 87
<211> 122
<212> DNA
<213> Homo sapiens

<400> 87

cctctggcct gtggctcact gcatgcagcc cctggcgtgc aatactagtg ctccacggcg 60

cgatgtgctt ctagcccttg cactgcacct aggctcaggg ttcaaacggc cagcccgaaa 120

ag 122

<210> 88
<211> 122
<212> DNA
<213> Homo sapiens

<400> 88

gcctgcaagc gcaaggaaca ggagcagcag aaggagcgcg ccctgcagcc caagaagcag 60

cgcctggtgt tcaccgacct gcagcgacgc acgctgatcg ccatcttcaa ggagaacaag 120

cg 122

<210> 89
<211> 122
<212> DNA
<213> Homo sapiens

<400> 89

accgggaggc cattttgcgc gtgcgctgct cgcctcgcgc cgccctcggc tctgcggact 60

cggatcccgc caaatttgaa cgcgagattg tcaggccctg aggggcttga ggggcggggg 120

aa 122

<210> 90
<211> 122
<212> DNA
<213> Homo sapiens

<400> 90

tcttgcgcgg cgtccggtgc agagccagca gtacgctgct gttgcccagc acggccaccg 60

cgaaagtcac cgccagcacg gcgatctcca gtttggccag ctcctcgttg cgcacgtccc 120

tc 122

<210> 91
<211> 122
<212> DNA
<213> Homo sapiens

<400> 91

gggtgctcac gctgtcgccc acccagtagg gctggatgaa gaccaccacg ttgatgatgg 60

cgaagcagat ggtgaagatg gcccacagca cgccgatggc ccgcgagttc cgcatgtagt 120

gc 122

<210> 92
<211> 122
<212> DNA
<213> Homo sapiens

<400> 92

ccgcggatgc gcgcccctcc cctgcagcca gcttactccg agggtccccg cgcgggcggc 60

cgcactcacc catgttaacg aagctcatga ccaggtcggc gcggcccagg cgccgctccg 120

cg 122

<210> 93
<211> 122
<212> DNA
<213> Homo sapiens

<400> 93

gcaggactct ggtccttcct gtggttcgtg ggcttctgct tcctcaccaa tcagtggcag 60

cgcacggcgc cagggccggc cacgacgcag gcgggggacg cggcgcgggc cgccatcgcc 120

tt 122

<210> 94
<211> 120
<212> DNA
<213> Homo sapiens

<400> 94

tgtactgtga tggggctttg tggtgagagg aacctctgag aagcctcgtg cggcttgagt 60

ttagagtcac gccctgccca gcgacattct cccgcgcacg ggagaacctg cacttccggt 120

<210> 95
<211> 122
<212> DNA
<213> Homo sapiens

<400> 95

aggacagggt agggtctgca ggagcctagg accaggcgga ggacgcggtc tgcggcgctg 60

cgctccaggg ccgacgacgg agaggctggg aggcgctcag gacgggaatc cacctcgcag 120

gt 122

<210> 96
<211> 122
<212> DNA
<213> Homo sapiens

<400> 96

gccgctagtc cctgccacgc cattgaacct tctcaagcac gcgtggccca gccagcaacg 60

cgctctttaa cccgcctccc agccccgcct tcggccaatc cgcatccgag gcgctgcgcg 120

cc 122

<210> 97
<211> 120
<212> DNA
<213> Homo sapiens

<400> 97

cccgagactc agtgacagtc gcagcttaac cccgttgggg gcgccgcccc gctgaggtgg 60

ttgcgtctcc aagtcgtgag cctccaatag ctgctcccgc tttcgcgtcg caaccccagg 120

<210> 98
<211> 122
<212> DNA
<213> Homo sapiens

<400> 98

agcaccgcgg acagcgccca gatggccagc acagcgtagg cgctgaggcg cagcgagatg 60

cgccgcctca gcgggtgcac cagcacgacg tagcggtcca ctgcgatggt ggtgagcgtg 120

aa 122

<210> 99
<211> 122
<212> DNA
<213> Homo sapiens

<400> 99

cgcggagcag cgcctgggcc gcgccgacct ggtcatgagc ttcgtcaaca tgggtgagtg 60

cggccgcccg cgcggggacc ctcggagtaa gctggctgca ggggaggggc gcgcatccgc 120

gg 122

<210> 100
<211> 122
<212> DNA
<213> Homo sapiens

<400> 100

cccgggggc tgcagcccgg ccagcgggtc ctctacaagc aatcgatcgc gcagcgcgcg 60

cggaccatgg cgctgtacaa ccccatcccg gtcaagcaga actgcttcac cgtcaaccgc 120

tc 122

<210> 101
<211> 122
<212> DNA
<213> Homo sapiens

<400> 101

catccgaggc acacaatcaa ttagctgaag caaagccccg gactggccct cggagcggcc 60

cgcagcggga ctctgcgggc accggctagc gggcgaggtg aggagtgcgg gcgcggggcc 120

gc 122

<210> 102
<211> 120
<212> DNA
<213> Homo sapiens

<400> 102

gcaatctggg gtcgctttgt aaggagctcc gacctccggg cagtgcaggg atactcgacg 60

gcgcgcgatg tcccggcggc tccgcgagcc tggcgcgctg agagccccag cagtccgagt 120

<210> 103

<211> 122
<212> DNA
<213> Homo sapiens

<400> 103

cattgacttt aaaaggccat tttccttcgt cttctacaag aagcaagaaa ctttttttcga 60

cgtaggcttc ataccctccc ttcggaaact cagtccgctg accaaagccg cagtgttcag 120

gc 122

<210> 104
<211> 122
<212> DNA
<213> Homo sapiens

<400> 104

gccctggtct gacagggaag gcgagggggc gctggtgccc ctgcaggtgg gcagggaaac 60

cgccctcttc aggacgcgct tgtatttctg catcctgtgt cctgagcctg gcgcgggaac 120

cg 122

<210> 105
<211> 122
<212> DNA
<213> Homo sapiens

<400> 105

catcctcgga ggatgatata gaccggcggc ccatccggag agtgcgctcc aagagcgaca 60

cgccgtacct cgcagaggcc aggatctcct ttaacctggg ggcaggtaag aacgcccctg 120

gc 122

<210> 106
<211> 122
<212> DNA
<213> Homo sapiens

<400> 106

tgtcaggccc tgaggggctt gaggggcggg ggaacgacgc cgctctccaa agttggaccc 60

cgtggcgagc ggcggcgaca gccgggtgct cgctgcctcc cgaggtgctc ccttttcccg 120

cc 122

<210> 107
<211> 122
<212> DNA
<213> Homo sapiens

<400> 107

cgagatagcg cggcgacatg gccacacaat ggagcccgca ggcgggagtg cggggcgggg 60

cgcggcgccc tggccttgcg cgcttacggg gtcctctcca gggccctctg gggcctctga 120

ct 122

<210> 108
<211> 122
<212> DNA
<213> Homo sapiens

<400> 108

cgtaagacag gaggatgacc agcagaggga gcaggtaggt gaccagcagc agcccccagg 60

cgtagagctg gcgctggcgc tcctgggagc cccagaactc ctcgcagagg cgcacgtcgt 120

gc 122

<210> 109
<211> 122
<212> DNA
<213> Homo sapiens

<400> 109

gcgcggcgcg gcggagcacc cggcgggcac gcctcgcacg ccagagcaaa ctttcacgac 60

cgcgggagag gcaccgcgca caccgttacc tttgcagccg catccttccc tggcccttgc 120

cc 122

<210> 110
<211> 122
<212> DNA
<213> Homo sapiens

<400> 110

ctacaacgac cccaagtgct gcgacttcgt caccaaccgg gcctacgcca tcgcctcgtc 60

cgtagtctcc ttctacgtgc ccctgtgcat catggccttc gtgtacctgc gggtgttccg 120

cg 122

<210> 111
<211> 120
<212> DNA
<213> Homo sapiens

<400> 111

ttcgctgcgc aaacacatga aggtaatcgc cgcactctcg tcgccccctt tgaggcagga 60

gctctcttgg ctctcggctt ggggtcgggc ggcgagtggc agacaggcgg tggcgggagc 120

<210> 112

<211> 122
<212> DNA
<213> Homo sapiens

<400> 112

ggtggtcgag ggtcgcagcc ccagcgtgct caggatgttg tagatctgct tgcggtcgcc 60

cgtgtcgctg cggggcggga ggaggtaggg cggtcgctga gtgtcgctcc gacagtccat 120

cc 122

<210> 113
<211> 122
<212> DNA
<213> Homo sapiens

<400> 113

gagcacacgt gtggagcatg gttgtggcat gggcgtctgg gcccctctcc tagacctcca 60

cgaggtgcgg acgcgcctct gccccgccct tttgtgcgcc ctcaccacta gaaaaagagt 120

tt 122

<210> 114
<211> 122
<212> DNA
<213> Homo sapiens

<400> 114

gccgcagtgg tggcggtggc ggagagcgcg gcggagtgag tgcggctgtg gacccgggac 60

cgggcgccgc tgggcgggcg cggcagggtc ccggatccgt cctccgtgac gcgccggctt 120

cc 122

<210> 115
<211> 122
<212> DNA
<213> Homo sapiens

<400> 115

tactcctcgg ccacgcggcg gctggcaact gacgcgcgcg atgttgcgcc ggccaatcag 60

cgtatggctc agtcccgcgg gccccgcttc caggtggagc tgctcctgct ctcggggtag 120

cc 122

<210> 116
<211> 122
<212> DNA
<213> Homo sapiens

<400> 116

cctgcgtgca gctgggtctg cagctgctgc ccgaggggcc tgcggccgac gaggtgttcg 60

cgctgctgcg gcgcgaggtg ctgcgcaccg tgtgcgagcg cccgggcccc gcggcctgcg 120

cg 122

<210> 117
<211> 122
<212> DNA
<213> Homo sapiens

<400> 117

aagggggcgg caccgctgac gtcatttccg gggtcggggt atataagcgg ggcgcgaggg 60

cgctgctgct gccaccgctc ctgccactgc agtgctcgag ccccgtgcag ggagcttgc 120

gg 122

<210> 118
<211> 122
<212> DNA
<213> Homo sapiens

<400> 118

tggctttctt tctgttatta gaattcttta tgaggaattt ggcctcaggc caaaatctta 60

cgtgatgttt gagcccacta tagacaatta caaggcttta tctgacaaac tcaatggcgg 120

aa 122

<210> 119
<211> 122
<212> DNA
<213> Homo sapiens

<400> 119

cggggacgag ggaccaggat ggggattcgg gggctggtag ccgggctgtc gcggatccag 60

cgcgtccttg gaaagtagga tggcgaggcc gggcacgttc ttaagtacgc tgagactagc 120

gg 122

<210> 120
<211> 122
<212> DNA
<213> Homo sapiens

<400> 120

aaaaaagatg tgtttgtgta gcggggacgc agcttgacac ccatttcctt tggctcgcat 60

cgcggtcgca gagcccaatc gcggggaaac gggcccccca gaaaaacagg caacgacccc 120

ca 122

<210> 121
<211> 122
<212> DNA
<213> Homo sapiens

<400> 121

gccggggtta gccttcgagg cggggagagg gagcggggtc cttcaaggac cgcatgttcg 60

cgatagcaca tttgagccct ggcaggccac ctgggccgcc ttctttcgga aaccttgcgg 120

gg 122

<210> 122
<211> 122
<212> DNA
<213> Homo sapiens

<400> 122

ctcgggaggc gctttgcctt tgaggaagat ggagaggagt cgggagaagc gcctagaaac 60

cgcattgatt tagacatcaa tcctggccgg ctccctccgc ctgccgagct gcggggccgc 120

gc 122

<210> 123
<211> 122
<212> DNA
<213> Homo sapiens

<400> 123

gaatgaaagg ggcccaagta gggaacagga gtgaggagag acagggttag cgggggcagt 60

cgaaggagac aacggaaagg cagaaaacag aaaaataacg caagagagag aaaaagtaaa 120

gg 122

<210> 124
<211> 122
<212> DNA
<213> Homo sapiens

<400> 124

cgagaacaag cagggctggc agaactccat ccgccacaat ctgtccctca acaagtgctt 60

cgtgaaggtg ccgcgccact acgacgaccc gggcaagggc aactactgga tgctggaccc 120

gt 122

<210> 125
<211> 122
<212> DNA
<213> Homo sapiens

<400> 125

```
gccgacgggg ggcgggaggc tgggcgggag caggccggcg agggagcgcg cgctggggtg        60
cggacctcca ggcgtccggg gctcccgctc caacgtgggt ctcaagcgaa cccgctgtga       120
tc                                                                     122
```

<210> 126
<211> 122
<212> DNA
<213> Homo sapiens

<400> 126

```
cgcgagggca gctccgcgcg gaggtgggac cggcgggttg ggaacggagt tggggcccgt        60
cgggggccgg cgcgtggagg gtgggaggat ccggccgctg ccgggcggat gggagctgcg       120
cg                                                                     122
```

<210> 127
<211> 122
<212> DNA
<213> Homo sapiens

<400> 127

```
ttcctctccg tctccctccc accccggccg tctatgctcc aggccctctc ctcgcggtgc        60
cggtgaaccc gccagccgcc ccgatgtaca gcatgatgat ggagaccgac ctgcactcgc       120
cc                                                                     122
```

<210> 128
<211> 122
<212> DNA
<213> Homo sapiens

<400> 128

```
tcttcctgtt tttactcctc cttttcattc ataacaaaag ctacagctcc aggagcccag        60
cgccgggctg tgacccaagc cgagcgtgga agaatggggt tcctcgggac cggcacttgg       120
at                                                                     122
```

<210> 129
<211> 122
<212> DNA
<213> Homo sapiens

<400> 129

cctgccggcc gggcggcgat ttgcaggtcc agccggcgcc ggtttcgcgc ggcggctcaa 60

cgtccacgga gccccaggaa tacccacccg ctgcccagat cggcagccgc tgctgcgggg 120

ag 122

<210> 130
<211> 122
<212> DNA
<213> Homo sapiens

<400> 130

tagcccaagg gagaccaaag actccacctt gagcatcgcc ctttggaggc gggcagagtc 60

cggccgcagg ccacaaagcg atccccaccc gaaggactcc acaaggacag tcctttcctt 120

gc 122

<210> 131
<211> 122
<212> DNA
<213> Homo sapiens

<400> 131

atccacgtcc aggaatccca gactgtgcag aggttagggg ccctggcgag ggggcccagt 60

cgtatgcgat aagcgccgct tgtcccgcag gcggaagagg cgctgagttg gaggtcgcgc 120

aa 122

<210> 132
<211> 122
<212> DNA
<213> Homo sapiens

<400> 132

ctgagagcac gcgccagaca caggttggct cggcccgaag cgaatttggg actctcgtcg 60

cggtgtccta ttaattagct tttcccggcg cctcagggac tccggggtga agatggagag 120

gt 122

<210> 133
<211> 122
<212> DNA
<213> Homo sapiens

<400> 133

agcgagtttc cgtagtgtag tggttatcac gtttgcctaa cacgcgaaag gtccccggtt 60

cgaaaccggg cagaaacaga gcgtagtttc gtttttttgg ttgttttttt tttttttttt 120

tt 122

<210> 134
<211> 122
<212> DNA
<213> Homo sapiens

<400> 134

cgaaccagcg aagaaagcta tcgatcgtaa aacaaaataa acaccaaaca atgttgccgc 60

cgcttaaaaa aaaaaaagtg gggcggggag agaaggaagg gtaattggga agtggcaatc 120

tg 122

<210> 135
<211> 122
<212> DNA
<213> Homo sapiens

<400> 135

cattctggaa gtgcctttgc tgtgtttact agccccatcc ccgcctgctg cacccggaga 60

cgtctgaagt ctctaatcgc tcagcgaaaa gttggtttcg ggaagggcag cgccggggtg 120

cg 122

<210> 136
<211> 122
<212> DNA
<213> Homo sapiens

<400> 136

gatcccgctg cgctccaacg tgtacgaggt ctacgccacg gacaaggatg agggcctcaa 60

cggggcggtg cgctacagct tcctgaagac tgcgggcaac cgggactggg agttcttcat 120

ca 122

<210> 137
<211> 122
<212> DNA
<213> Homo sapiens

<400> 137

tggtactagc acgtcaccta gaaggaagaa tcctggaatg gcacgggtcc aaactagagg 60

cggcctctca gcatggaccc gcttcaacct catctgcatg gcaggcgttt tgcaaggcgt 120

ca 122

<210> 138
<211> 122
<212> DNA
<213> Homo sapiens

<400> 138

agcctcctgc tgtctctgga gctggcgtcc ggcagtgggc agggcctcag cccggaccgt 60

cgggcctcgc tgctcacgtc tcttatgctg gttaagcgcg actaccgcta tgatcgggtt 120

ct 122

<210> 139
<211> 122
<212> DNA
<213> Homo sapiens

<400> 139

tttgggagag aggtggccca agagaagtga acttgcacag aagaggttga gccaccgcag 60

cgcaggcggc tcagggttat tgcgacccta gccccctcac ttctccctcc gaggctaagt 120

gt 122

<210> 140
<211> 122
<212> DNA
<213> Homo sapiens

<400> 140

acaaacaggg cgggggcgcg gaggtttggg gtgggggccg caggggctg gaaggaagtg 60

cgcagtgtgg cgagagcgcg aacaaagccc tctccggagc cccgtcaccc ctcggtgacc 120

cc 122

<210> 141
<211> 122
<212> DNA
<213> Homo sapiens

<400> 141

agtcaagcac ccctcttagg gctgcaggct ataggaacag tttctgccat gagcgtgcat 60

cgatgaggtt atccacccgc cccctccact ttgtgtgtct gggtctccag atttcattat 120

aa 122

<210> 142
<211> 122
<212> DNA
<213> Homo sapiens

<400> 142

tccctcccgg cccctcgact acacctgggg tctctccttc cttcctcctc gggaggagcc 60

cgactgggag acgccgcggg gactacccat ttccagcccg ctcacctttc ccatgcagca 120

gc 122

<210> 143
<211> 122
<212> DNA
<213> Homo sapiens

<400> 143

gccacgcccc catcctgtgc ctataaaaac ccccgagacc ctagcgggca cagacacaag 60

cggttggaca tgaagaggaa cacaccggcg ggagaacaca gaacacccac cctctcttta 120

gt 122

<210> 144
<211> 122
<212> DNA
<213> Homo sapiens

<400> 144

aaatcgctat ttaaaaatat acaaataagg cccaagcata aaatctaact ctggggctgg 60

cggtggaggg aaggtgaggg agggggctct ggcacacgtc cctcgtgtgt gcttgcgcga 120

cg 122

<210> 145
<211> 122
<212> DNA
<213> Homo sapiens

<400> 145

tgggaaattc cctcttccta atgcgtctgt cttgcaactc ctattagcat ccttcgacag 60

cggcctttga aaatctcctt taaatttcaa tacctctact cgtttcccaa atggggggaa 120

gg 122

<210> 146
<211> 122
<212> DNA
<213> Homo sapiens

<400> 146

cctcccactc gccgacctgg gcgctgtcct tggtgctgcc ggcgccatag cgagcacaag 60

cgccttacct tctgcccctg cggaagctgc ggacgtcccc caaacgttaa aaacgccata 120

tc 122

<210> 147
<211> 122
<212> DNA
<213> Homo sapiens

<400> 147

gctgcagagg ccagtgtggc ggaggaggaa gaggggtggg ggagggagct ttttctctga 60

cggaccttgg agaagggcag tggacgagca tgtgtccctc cgcttccgca gaggacggaa 120

ag 122

<210> 148
<211> 122
<212> DNA
<213> Homo sapiens

<400> 148

cggcgcaggt gggagtctgg gaagaagtgc ggtgtcggca ggaagtccca gcgcgacgtc 60

cgggaaagcc acaggaggag gcgacctgca ggaggctacc ctttgacccc aaccctactc 120

tc 122

<210> 149
<211> 122
<212> DNA
<213> Homo sapiens

<400> 149

tcccgagggc accttgggtc acatcaaacg ccggtacccg caggtgcggc ctgttgtggg 60

cggggcagag aggcggaggc gggactccgg gggggtgggg gccgggccta gtgtgtgggc 120

gg 122

<210> 150
<211> 122
<212> DNA
<213> Homo sapiens

<400> 150

ttcaccaagc gctacgcgga ggaggagatg taccgtccac acccggcctt ctaccgcccg 60

cgtctcagcg actgctccga ctactcgggc cagttcctgc agcccgaggc gtggcgccgc 120

gg 122

<210> 151
<211> 120
<212> DNA
<213> Homo sapiens

<400> 151

atttcctgca ggagccggtc agcgtggact gcggccacag cttctgcctc aggtgcatct 60

ccgagttctg cgagaagtcg gacggcgcgc agggcggcgt ctacgcctgt ccgcagtgcc 120

<210> 152

<211> 122
<212> DNA
<213> Homo sapiens

<400> 152

```
tgagcgggtg gcaggcgggg tgcgcgcccg gctggggcag tgcgcgcttg cggctgccgc      60
cgttgaacat ggccttgacg tcctcccagg cgaagaccag ctcgtcctgg ggactcttgt     120
cg                                                                    122
```

<210> 153
<211> 122
<212> DNA
<213> Homo sapiens

<400> 153

```
gggataccgc taggagggca ggaggtaaag tccagaggct ggcgcgtggt gcggccccga      60
cgccgcgggc tgatcctctg tccctgtggg gccgccacct acccgcggtg agcctcttct     120
cg                                                                    122
```

<210> 154
<211> 122
<212> DNA
<213> Homo sapiens

<400> 154

```
cactccccca cagaaagcgg ctgtattgca aaggccgctc cggcagcgca cggccaagcg      60
cggtgtcgcg acccgcaggg ctgcggctcg ccatgaaatc cctcaagtct cccctcaggg     120
aa                                                                    122
```

<210> 155
<211> 122
<212> DNA
<213> Homo sapiens

<400> 155

```
aaactgtaag ccagaaaacc tggccaggcc tccgcgacgg cgagaaacgg tcttgaaggt      60
cggtcggtcg ccgtaatgcg gtccggatcg tgcacgtgct gtttcgccac tgccctccgg     120
ct                                                                    122
```

<210> 156
<211> 122
<212> DNA
<213> Homo sapiens

<400> 156

ccgggcccgg gagggggcgg gccgggccgg ggttccagag cccatcggga gtcctgaggt 60

cgccgtaggg gagggaaggg gaaggctaga ggccacgcgg ggcggggact gggggggctg 120

gg 122

<210> 157
<211> 122
<212> DNA
<213> Homo sapiens

<400> 157

ggaagcgaag gcggaccctc ctcccccagc gctactgccc gggccgaatt ccaaggccag 60

cggctgcccg ctgagaagga atccggacca acgggattct ggcttccctc tggcccctcc 120

tg 122

<210> 158
<211> 122
<212> DNA
<213> Homo sapiens

<400> 158

cgtttaaatt tcaaatgagg aaaacgaaca ccttcgtttt tcaaacgcgg agacagacgc 60

cgggtgggcc gcgtcgtata gcgactgatc ttgctccttt cttgggcacc tccaatgtgc 120

ca 122

<210> 159
<211> 122
<212> DNA
<213> Homo sapiens

<400> 159

agagacagaa acagaaacga atgaagtatt cggctgtcca ctgattgcgg cagggcgcag 60

cgtcgggagc cgggagagcc tagtgctcat ccctcccggg ttcgggggat ggttatcggg 120

aa 122

<210> 160
<211> 122
<212> DNA
<213> Homo sapiens

<400> 160

gggcaattcc attgaaagtg aagccatcct ctgaaaagac cagaaatagt gagcacagag 60

cgactcacta aagcttcagg gtcacaaatt gagcggcaag atacttgcga gacagaaaac 120

cg 122

<210> 161
<211> 122
<212> DNA
<213> Homo sapiens

<400> 161

cggctctgcc gcgcctcgct gtgtgactgc gggcaaatcc ccaccctctc cgtgctccgg 60

cgaagaaaga agagtggctc ccacatcccg cctgctaaca gccacgcctg ctggattttt 120

ct 122

<210> 162
<211> 122
<212> DNA
<213> Homo sapiens

<400> 162

aggggtgtgg atattccaac aggatgcctt tctgttttgc agcaaaattt accgagtttc 60

cgcggaacgt gacggcgacc gaggggcaga atgtggagat gtcctgcgcc ttccagagcg 120

gc 122

<210> 163
<211> 122
<212> DNA
<213> Homo sapiens

<400> 163

atcggtgact tctctaatga tgaataaaga cggcttcctg ggaggcatgt ctgtcaacac 60

cggcgaggtg ttttgctccg tcccaggccg tttgtctctg ctcagttcaa cttcgaagta 120

ca 122

<210> 164
<211> 122
<212> DNA
<213> Homo sapiens

<400> 164

ggaaggggcc ctcggtcact aagtccttcc tcgcgaagcc cgcaggcatc tccagccgcg 60

cgcagtcctg ggcgctcccg ccgccgccca ccgaggcaca ggcaccttct ccgtgagccg 120

gg 122

<210> 165
<211> 122
<212> DNA
<213> Homo sapiens

<400> 165

aagaataaag ttgcccaaag ggttcccagg acaccccagc tccacctggc ggggcagccc 60

cggagaacca gaggtaaatg accaccgcga ctgcaaacag agggtcctgc aagagaaggc 120

ga 122

<210> 166
<211> 122
<212> DNA
<213> Homo sapiens

<400> 166

ccgcgctgag gcgcagacac ggcggctcct ccaggcccat taataacccg gagggcgctg 60

cgatccgaca gcgcagtcgc cacaggctct atttcccgtg gccgcctgcc tccttccagc 120

cc 122

<210> 167
<211> 122
<212> DNA
<213> Homo sapiens

<400> 167

aaagcctccc acgaagtctg aacctggtac tgatatacgc ttcagccctc agaaggcaac 60

cgcttgattc tctgcgcagg cgcagcgttc tcaacaccgc caacgggcac aaagccacgc 120

cc 122

<210> 168
<211> 120
<212> DNA
<213> Homo sapiens

<400> 168

tgctacccac ccagggggcgc gcgaccctcc cttcggtctg gctccaaaga cctagcagca 60

ctgacttcac ccagctgtgg ttccaacggc gggtccagcg gcctcggccc ggcgccgtcc 120

<210> 169

<211> 122
<212> DNA
<213> Homo sapiens

<400> 169

cgagtttgac ttctttgaga ggctccacac atcgcgggtg gctagagtct gcaaggtccg 60

cggcctgggc gggggcggg gctaactgga ggagaaccaa tagggagatg gcaggggcag 120

ga 122

<210> 170
<211> 122
<212> DNA
<213> Homo sapiens

<400> 170

aggtgggcgt cgtacgtcgt tacgcagcgc ggttgctggg cacctcgact atcacctgac 60

cgtagtaata tctcccgcta cgcgcgttgt gaccaatgct gcatacagga gatgagggag 120

ga 122

<210> 171
<211> 122
<212> DNA
<213> Homo sapiens

<400> 171

catcgctggc ggcgggtccc cgcgcggagc gggcgctgtg tgttcgcgcg cgcgggcgcg 60

cgctgaatag gggtggcctc ctgcgcccaa cctgctgcat tttaggtgct tgcctctctt 120

tg 122

<210> 172
<211> 122
<212> DNA
<213> Homo sapiens

<400> 172

gtacacgccc tgggaatcgg gggtcttcca gtatagggaa ccaccgagat gcgggccctc 60

cgaagtccta gagagggcgc ctcggccgcc ggcacccgcc acacttccgg cctttgtggg 120

cc 122

<210> 173
<211> 122
<212> DNA
<213> Homo sapiens

<400> 173

acctccctcc cgctgtgcaa aggaacgcag gagcccggcg caggtggtgg gcttaccgcg 60

cgcacgcctg gctggagagg tgacgccgct gtctgccaac ctttcccagc ttttcccacg 120

at 122

<210> 174
<211> 122
<212> DNA
<213> Homo sapiens

<400> 174

gactggaaac gcttccccta tttcttccgt agcggaccgg gagagcttac tggcgctctg 60

cgaaccggct ggaaagaaac accgagtcac tcgtacagac tcttggtcgc agaacttggc 120

tt 122

<210> 175
<211> 122
<212> DNA
<213> Homo sapiens

<400> 175

cggccacact cctattcacg tgatcgattt ctgcatattc cactcgcctg aaccgccgcg 60

cgctgactgg ttccgcctca ccgcccgggt gggttttatt gctcagccct ggggactttt 120

aa 122

<210> 176
<211> 122
<212> DNA
<213> Homo sapiens

<400> 176

ggcttccgct cgcagtcgtg gtcccgcggc tcgcccagca ccgtgtcctc ctcctataag 60

cgcagcatgc tcgccccgcg cctcgcttac agctcggcca tgctcagctc cgccgagagc 120

ag 122

<210> 177
<211> 122
<212> DNA
<213> Homo sapiens

<400> 177

tgccagcctt cttcaccgag agcagcgagt acagctgtgt gatggacggc cagaccatgg 60

cggtggccac tggagggggc accagccctc cccagcccaa ccccttccgc tccccctttc 120

gc 122

<210> 178
<211> 122
<212> DNA
<213> Homo sapiens

<400> 178

gcgcgcgtgg acctgcagaa gaaggcgcag gcgctgcagg aggagtgcgg ctacctgcgg 60

cgccaccacc aggaagaggt gggcgagctg ctcggccaga tccagggctc cggcgccgcg 120

ca 122

<210> 179
<211> 122
<212> DNA
<213> Homo sapiens

<400> 179

gttgaagggt atctcgcaga cttttgggaa gcggtcccgg tagcccatgg cgttgcccag 60

cgtcagctcc gagaacttga gcagcgccgt ctccgatgcg tctccaatca cgatgcgctg 120

gg 122

<210> 180
<211> 122
<212> DNA
<213> Homo sapiens

<400> 180

aaattttctc tggcgatcaa aagcctggtc ggcaacaaag accgcgcccg ctttttccac 60

cgtcctggtg tggcgagttg cggaatttca ataactgtga caagggtgac tgatttgtga 120

ac 122

<210> 181
<211> 122
<212> DNA
<213> Homo sapiens

<400> 181

gacgcctccc gctgacaaga atcccaaagt tgcctcagct tcctcctctc cttttcttcc 60

cgcgcccaac cctgacatca ccacccctcc atcccccagg tatcttccta aagccgtgtc 120

gg 122

<210> 182
<211> 122
<212> DNA
<213> Homo sapiens

<400> 182

cagctagcgg caagcggagt caggcatccg ttcagactga cagcagaggc ggcgaaggag 60

cgcgtagccg agatcaggcg tacagagtcc ggaggcggcg gcgggtgagc tcaacttcgc 120

ac 122

<210> 183
<211> 122
<212> DNA
<213> Homo sapiens

<400> 183

cgaggttgcc gggggacccc caggcaggag ctgagatttg tgtggagcgg cgaagtgggg 60

cggcggcgcg tttggctttg acttctgatc ccccgaagcg ctctggggtc ctttgggccc 120

ca 122

<210> 184
<211> 122
<212> DNA
<213> Homo sapiens

<400> 184

cgcccccgcc gccgccgcca ccgcttgcgg cccccgtcgc cgccagtgcc gcgcgctcct 60

cgtccagcag cagcaccagc tccttgagct ccaggttctc gcgcagcagg gcctcctggc 120

gc 122

<210> 185
<211> 122
<212> DNA
<213> Homo sapiens

<400> 185

tctccacaac caccagatta tctcaccggc gagtgagact gcaaggtttg ggggcccggc 60

cgtaccactc cgcgctgcgc acggggggtt cgtacccatc tggccgcgac cgtccgtttc 120

cc 122

<210> 186
<211> 122
<212> DNA
<213> Homo sapiens

<400> 186

gagcggggcg ggtgaggaag gggctgaggg ggctgtgccg gccatggagc tgtacctcgg 60

cgcctgctcc aagcctgcca aagtcgccgt caccaagacg gtcgccagcg tcctggccgc 120

gg 122

<210> 187
<211> 122
<212> DNA
<213> Homo sapiens

<400> 187

tcggtcttct cccgcccctc cctcccttcc ccgcctctcc cccaagctcc tcagtggccg 60

cggcccgtca acactgtcgc gcagtcactg gcgcaggttc ccagctctca gctgggggtt 120

tc 122

<210> 188
<211> 122
<212> DNA
<213> Homo sapiens

<400> 188

ggaggcggga ggcggagccc cggggttggg agtgcggggg tcgcggcgca gagtgggagc 60

cggagagcga gcgcggctgc agccggcggc atggctagca cggcttcgga gatcatcgcc 120

tt 122

<210> 189
<211> 122
<212> DNA
<213> Homo sapiens

<400> 189

cagatattgc ggacagtcta attcgccccg cgacgtgagg gagaacccag gagcgggctc 60

cggtagagaa agaagcttcc ggtcaacgac cacatccacc tgaatcatga gcaggtttta 120

ag 122

<210> 190
<211> 122
<212> DNA
<213> Homo sapiens

<400> 190

gaaagagcag caagggtggg ctctgctgcc gcccagaggc cttccgtggt aagacagcca 60

cggaaagcac agcgcgtgcc gctgcgacgg ggcgtcccgg gggtgacact ggcatgtgac 120

gc 122

<210> 191
<211> 122
<212> DNA
<213> Homo sapiens

<400> 191

gtgccagccg caatagggcg gccgtgggtg caaacggagg ggagcgccgg cagctagcac 60

cgcgcggcga ctaacgggcc gccccggaga ctcctgggag ctcaggccca cgcgcgagtg 120

cg 122

<210> 192
<211> 122
<212> DNA
<213> Homo sapiens

<400> 192

cgtgtctgga atgccgtcgt tttgccccgc ggccccatcg tacatgcgca tccccgcagc 60

cgccaccgtc tccactttcc gcagcagcgt ccctgacgct gcgcgacccg ttgccaaggc 120

ga 122

<210> 193
<211> 122
<212> DNA
<213> Homo sapiens

<400> 193

attaggtgct ataagcatca attaatagtt gctggcctct aggtaggtct tctatagacc 60

cgaaattgac atattaggaa tagccactaa tgattggctg cactatttct aaaatataac 120

cc 122

<210> 194
<211> 122
<212> DNA
<213> Homo sapiens

<400> 194

gagctcatag tccggcagga tgtccctgcg gctattcacg tcctccagcg ccatctccac 60

cgcgggctgg caggcctggc cccctggcca gcccccgctc atgggaaaca gtgccccgat 120

gt 122

<210> 195
<211> 122
<212> DNA
<213> Homo sapiens

<400> 195

cagcagtgcc acaggagccc tgctggggct ttgatttggg aaaacaaaca gaaatggcac 60

cgtgggccct tctcggcccc caccccaaga cctatttaac tagaaaactc tagctcaact 120

gc 122

<210> 196
<211> 122
<212> DNA
<213> Homo sapiens

<400> 196

ccaactcgcc acccatggga tcgactgccc tcagctgccc ggcttggagt gcgtagcctt 60

cgccgcgtgg cccaggagag gagcgagaca ccaaggcgtt tcgcggactc cgcgcgcacc 120

ag 122

<210> 197
<211> 122
<212> DNA
<213> Homo sapiens

<400> 197

tggggcagcc tcagcgcagc ttctcgggtg ggcggggcg ctcggggcct aaggcgcagt 60

cgcaggctgg ggaggggct cggctcgccg gggacgcgcc caggagagaa agcggcggca 120

gg 122

<210> 198
<211> 122
<212> DNA
<213> Homo sapiens

<400> 198

tcgcctacga tggcaaggat tacatcgccc tgaacgagga cctgcactcc tggaccgccg 60

cgaacacagc ggctcagatc tcccagcaca agtgggaagc ggacaaatac tcagagcagg 120

tc 122

<210> 199
<211> 122
<212> DNA
<213> Homo sapiens

<400> 199

cccctctgca gagcaggctt ggccgaggag taagcgcggc cgctgcggag ggtttggcag 60

cggggacagg gctgcgcccg gtgtgtgcgc ggacacctgc cgcccgtcct cagcgcccgg 120

ta 122

<210> 200
<211> 122
<212> DNA
<213> Homo sapiens

<400> 200

```
gtctggctga gtgtacttgg cgtcgggcgt gcggtgggag tggaaaatga acttgttggg      60
cgagaagtac atgttgcagt agctgcattt gatgcacttg gcgcgcgagc tgttgtagcg     120
cg                                                                    122
```

<210> 201
<211> 122
<212> DNA
<213> Homo sapiens

<400> 201

```
ggcggagacg gttagtctag cctctctgtg gggcctctct gctggtaccc agatgctgag      60
cgctgaaatt ctcatccgcc acacacaaca tggcggcttc ctggacccgc gcccaatttt     120
cg                                                                    122
```